(19) Europäisches Patentamt
European Patent Office
Office européen des brevets

(11) EP 1 263 738 B1

(12) **EUROPÄISCHE PATENTSCHRIFT**

(45) Veröffentlichungstag und Bekanntmachung des Hinweises auf die Patenterteilung:
**27.06.2007 Patentblatt 2007/26**

(21) Anmeldenummer: **01931488.9**

(22) Anmeldetag: **01.03.2001**

(51) Int Cl.:
***C07D 239/10*** (2006.01) ***C07D 249/12*** (2006.01)
***C07D 257/04*** (2006.01) ***C07D 233/32*** (2006.01)
***A01N 43/54*** (2006.01)

(86) Internationale Anmeldenummer:
**PCT/EP2001/002279**

(87) Internationale Veröffentlichungsnummer:
**WO 2001/066527 (13.09.2001 Gazette 2001/37)**

(54) **SUBSTITUIERTE BENZOYLCYCLOHEXENONE**

SUBSTITUTED BENZOYLCYCLOHEXENONES

BENZOYLCYCLOHEXENONES SUBSTITUEES

(84) Benannte Vertragsstaaten:
**AT BE CH CY DE DK ES FI FR GB GR IE IT LI LU MC NL PT SE TR**

(30) Priorität: **06.03.2000 DE 10010937**
**09.06.2000 DE 10028687**

(43) Veröffentlichungstag der Anmeldung:
**11.12.2002 Patentblatt 2002/50**

(73) Patentinhaber:
- **Bayer CropScience AG**
  **40789 Monheim (DE)**
- **Bayer CropScience K.K.**
  **Tokyo (JP)**

(72) Erfinder:
- **MÜLLER, Klaus-Helmut**
  **40593 Düsseldorf (DE)**
- **SCHWARZ, Hans-Georg**
  **40764 Langenfeld (DE)**
- **HERRMANN, Stefan**
  **40764 Langenfeld (DE)**
- **HOISCHEN, Dorothee**
  **40474 Düsseldorf (DE)**
- **LEHR, Stefan**
  **40764 Langenfeld (DE)**
- **SCHALLNER, Otto**
  **40789 Monheim (DE)**
- **DREWES, Mark, Wilhelm**
  **40764 Langenfeld (DE)**
- **DAHMEN, Peter**
  **41470 Neuss (DE)**
- **FEUCHT, Dieter**
  **40789 Monheim (DE)**
- **PONTZEN, Rolf**
  **42799 Leichlingen (DE)**
- **YANAGI, Akihiko**
  **Oyama-shi,**
  **Tochigi 323-0829 (JP)**
- **NARABU, Shinichi**
  **Yuki-shi,**
  **Ibaraki 307-0001 (JP)**
- **GOTO, Toshio**
  **Shimotsuga-gun,**
  **Tochigi 329-0414 (JP)**
- **ITO, Seishi**
  **Oyama-shi,**
  **Tochigi 323-0022 (JP)**
- **UENO, Chieko**
  **Oyama-shi,**
  **Tochigi 323-0807 (JP)**

(74) Vertreter: **Schwenk, Norbert**
**Bayer CropScience GmbH**
**Patent- und Lizenzabteilung**
**Industriepark Höchst,**
**Gebäude K 607**
**65926 Frankfurt am Main (DE)**

(56) Entgegenhaltungen:
**DE-A- 19 921 732**

- **CHEMICAL ABSTRACTS, vol. 130, no. 14, 1999 Columbus, Ohio, US; abstract no. 178767w, Seite 223; Spalte 2; XP002174489 & JP 01 121280 A (NIPPON SODA) 26. Januar 1999 (1999-01-26)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 263 738 B1

## Beschreibung

Die Erfindung betrifft neue substituierte Benzoylcyclohexenone, Verfahren zu ihrer Herstellung und ihre Verwendung als Herbizide.

Es ist bereits bekannt, dass bestimmte substituierte Benzoylcyclohexandione herbizide Eigenschaften aufweisen (vgl. EP-A-090 262, EP-A-135 191, EP-A-186 118, EP-A-186 119, EP-A-186 120, EP-A-319 075, WO-A-96/26200, WO-A-97/46530, WO-A-99/07688, WO-A-00/05221). Die Wirkung dieser Verbindungen ist jedoch nicht in allen Belangen zufriedenstellend.

Es wurden nun die neuen substituierten Benzoylcyclohexenone der Formel (I),

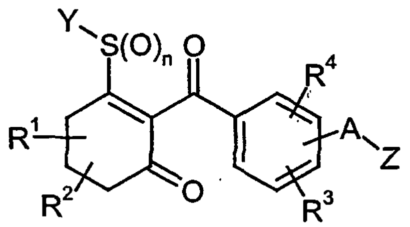

(I)

in welcher

n für die Zahlen 0, 1 oder 2 steht,

A für eine Einfachbindung oder für Alkandiyl (Alkylen) mit 1 bis 6 Kohlenstoffatomen steht,

$R^1$ für Wasserstoff, Phenyl oder gegebenenfalls durch Cyano, Halogen, $C_1$-$C_4$-Alkoxy oder $C_1$-$C_4$-Alkylthio substituiertes Alkyl mit 1 bis 6 Kohlenstoffatomen steht,

$R^2$ für Wasserstoff oder gegebenenfalls durch Cyano, Halogen, $C_1$-$C_4$-Alkoxy oder $C_1$-$C_4$-Alkylthio substituiertes Alkyl mit 1 bis 6 Kohlenstoffatomen steht, oder zusammen mit $R^1$ für Alkandiyl (Alkylen) mit 1 bis 6 Kohlenstoffatomen steht, oder zusammen mit $R^1$ - in diesem Fall an das gleiche Kohlenstoffatom gebunden - und dem Kohlenstoffatom, an welches $R^1$ und $R^2$ dann gebunden sind, für eine (C=O)-Gruppierung steht,

$R^3$ für Wasserstoff, Nitro, Cyano, Carboxy, Carbamoyl, Thiocarbamoyl, Halogen, oder für jeweils gegebenenfalls durch Cyano, Halogen oder $C_1$-$C_4$-Alkoxy substituiertes Alkyl, Alkoxy, Alkylthio, Alkylsulfinyl, Alkylsulfonyl, Alkylamino, Dialkylamino oder Dialkylaminosulfonyl mit jeweils 1 bis 6 Kohlenstoffatomen in den Alkylgruppen steht,

$R^4$ für Wasserstoff, Nitro, Cyano, Carboxy, Carbamoyl, Thiocarbamoyl, Halogen, oder für jeweils gegebenenfalls durch Cyano, Halogen oder $C_1$-$C_4$-Alkoxy substituiertes Alkyl, Alkoxy, Alkylthio, Alkylsulfinyl, Alkylsulfonyl, Alkylamino, Dialkylamino oder Dialkylaminosulfonyl mit jeweils 1 bis 6 Kohlenstoffatomen in den Alkylgruppen steht, und

Y für Wasserstoff, für gegebenenfalls durch Cyano, Halogen oder $C_1$-$C_4$-Alkoxy substituiertes Alkyl mit 1 bis 10 Kohlenstoffatomen, für jeweils gegebenenfalls durch Cyano, Halogen, $C_1$-$C_4$-Alkyl-carbonyl oder $C_1$-$C_4$-Alkoxycarbonyl substituiertes Alkenyl oder Alkinyl mit jeweils 2 bis 10 Kohlenstoffatomen, oder für jeweils gegebenenfalls durch Nitro, Amino, Cyano, Carboxy, Carbamoyl, Thiocarbamoyl, Halogen, $C_1$-$C_4$-Alkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alky1tllio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl, $C_1$-$C_4$-Halogenalkylsulfonyl, $C_1$-$C_4$-Alkylcarbonyl, $C_1$-$C_4$-Alkoxy-carbonyl, $C_1$-$C_4$-Alkyl-carbonyl-amino, $C_1$-$C_4$-Alköxy-carbonyl-amino, $C_1$-$C_4$-Akyl-sulfonyl-wnino substituiertes Aryl oder Arylalkyl mit jeweils 6 oder 10 Kohlenstoffatomen in den Arylgruppen und gegebenenfalls 1 bis 4 Kohlenstoffatomen im Alkylteil steht,

Z für eine gegebenenfalls substituierte 4- bis 12-gliedrige, gesättigte oder ungesättigte, monocyclische oder bicyclische, heterocyclische Gruppierung steht, welche 1 bis 4 Heteroatome (bis zu 4 Stickstoffatome und gegebenenfalls-alternativ oder additiv - ein Sauerstoffatom oder ein Schwefelatom, oder eine SO-Gruppierung oder eine $SO_2$-Gruppierung) enthält, und welche zusätzlich ein bis drei Oxo-Gruppen (C=O) und/oder Thioxo-Gruppen (C=S) als Bestandteile des Heterocyclus enthält,

- einschließlich aller möglichen tautomeren und gegebenenfalls möglichen stereoisomeren Formen der Verbindungen der allgemeinen Formel (I), und der möglichen Salze oder Metall-koordinierten Derivate der Verbindungen der allgemeinen Formel (I) - gefunden.

In den Definitionen sind die Kohlenwasserstoffketten, wie Alkyl oder Alkandiyl - auch in Verbindung mit Heteroatomen, wie in Alkoxy - jeweils geradkettig oder verzweigt.

Bevorzugte Bedeutungen der in den oben und nachstehend aufgeführten Formeln vorhandenen Reste bzw. Gruppierungen werden im Folgenden definiert.

n steht bevorzugt für die Zahlen 0 oder 2.

A steht bevorzugt für eine Einfachbindung oder für Alkandiyl (Alkylen) mit 1 bis 4 Kohlenstoffatomen.

$R^1$ steht bevorzugt für Wasserstoff, Phenyl oder gegebenenfalls durch Cyano, Halogen, $C_1$-$C_4$-Alkoxy oder $C_1$-$C_4$-Alkylthio substituiertes Alkyl mit 1 bis 5 Kohlenstoffatomen.

$R^2$ steht bevorzugt für Wasserstoff oder gegebenenfalls durch Cyano, Halogen, $C_1$-$C_4$-Alkoxy oder $C_1$-$C_4$-Akylthio substituiertes Alkyl mit 1 bis 5 Kohlenstoffatomen, oder zusammen mit $R^1$ für Alkandiyl (Alkylen) mit 1 bis 5 Kohlenstoffatomen.

$R^3$ steht bevorzugt für Wasserstoff, Nitro, Cyano, Carboxy, Carbamoyl, Thiocarbamoyl, Halogen, oder für jeweils gegebenenfalls durch Cyano, Halogen oder $C_1$-$C_4$-Alkoxy substituiertes Alkyl, Alkoxy, Alkylthio, Alkylsulfinyl, Alkylsulfonyl, Alkylamino, Dialkylamino oder Dialkylaminosulfonyl mit jeweils 1 bis 5 Kohlenstoffatomen in den Alkylgruppen.

$R^4$ steht bevorzugt für Wasserstoff, Nitro, Cyano, Carboxy, Carbamoyl, Thiocarbamoyl, Halogen, oder für jeweils gegebenenfalls durch Cyano, Halogen oder $C_1$-$C_4$-Alkoxy substituiertes Alkyl, Alkoxy, Alkylthio, Alkylsulfinyl, Alkylsulfonyl, Alkylamino, Dialkylamino oder Dialkylaminosulfonyl mit jeweils 1 bis 5 Kohlenstoffatomen in den Alkylgruppen.

Y steht bevorzugt für Wasserstoff, für gegebenenfalls durch Cyano, Halogen oder $C_1$-$C_4$-Alkoxy substituiertes Alkyl mit 1 bis 6 Kohlenstoffatomen, für jeweils gegebenenfalls durch Cyano, Halogen, $C_1$-$C_4$-Alkyl-carbonyl oder $C_1$-$C_4$-Alkoxy-carbonyl substituiertes Alkenyl oder Alkinyl mit jeweils 2 bis 6 Kohlenstoffatomen, oder für jeweils gegebenenfalls durch Nitro, Amino, Cyano, Carboxy, Carbamoyl, Thiocarbamoyl, Halogen, $C_1$-$C_4$-Alkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl, $C_1$-$C_4$-Halogenalkylsulfonyl, $C_1$-$C_4$-Alkyl-carbonyl, $C_1$-$C_4$-Alkoxy-carbonyl, $C_1$-$C_4$-Alkylcarbonyl-amino, $C_1$-$C_4$-Alkoxy-carbonyl-amino, $C_1$-$C_4$-Alkyl-sulfonyl-amino substituiertes Aryl oder Arylalkyl mit jeweils 6 oder 10 Kohlenstoffatomen in den Arylgruppen und gegebenenfalls 1 bis 4 Kohlenstoffatomen im Alkylteil.

Z steht bevorzugt für eine der nachstehenden heterocyclischen Gruppierungen

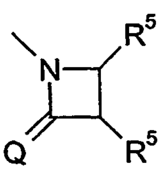 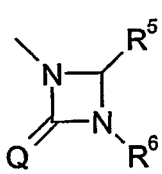 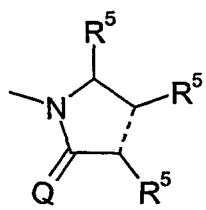 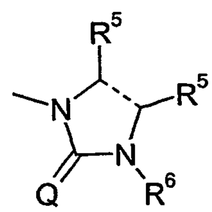

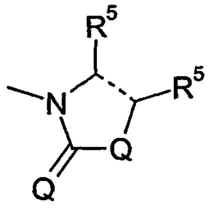 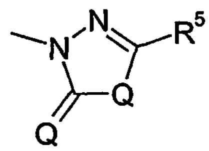 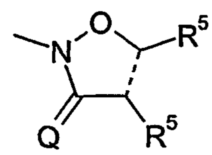 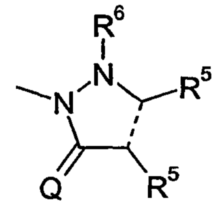

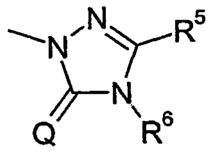 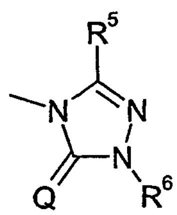 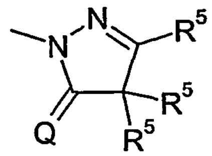 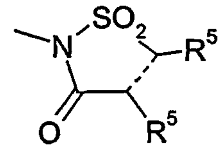

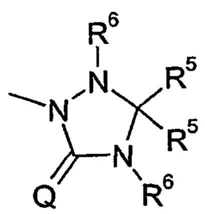 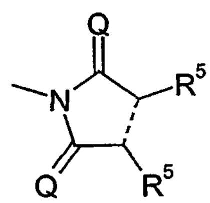 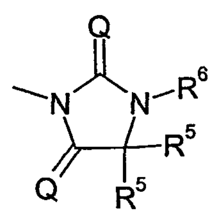 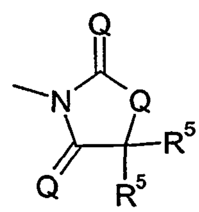

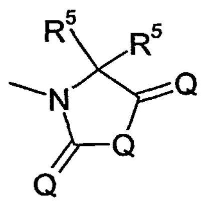 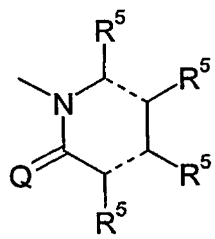 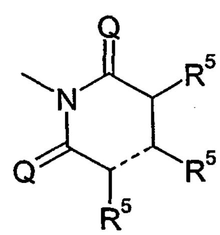 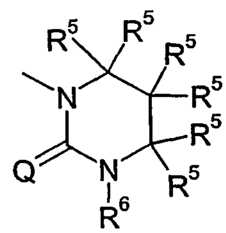

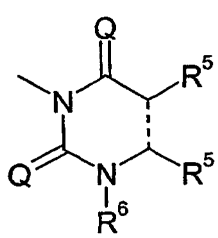 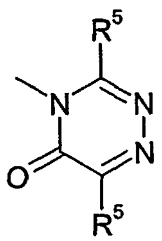 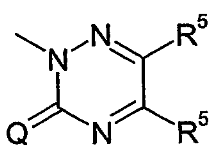 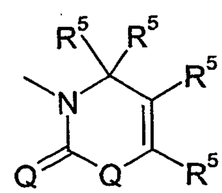

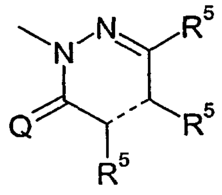 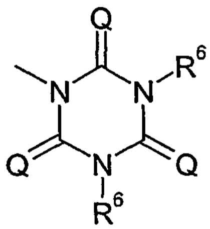 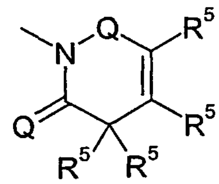 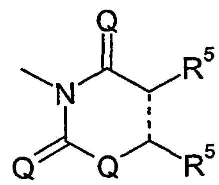

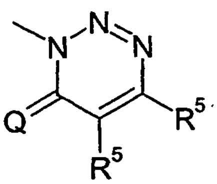 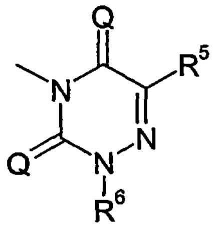 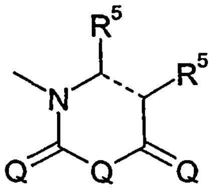 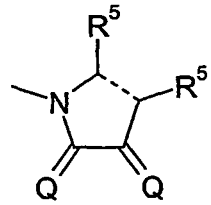

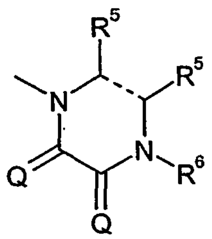 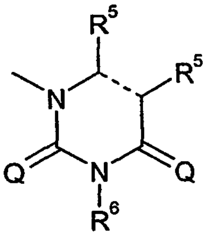 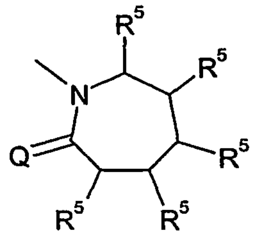 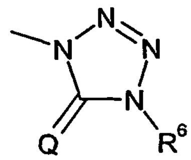

worin jeweils die gestrichelt gezeichnete Bindung eine Einfachbindung oder eine Doppelbindung ist, und worin jeder Heterocyclus bevorzugt nur zwei Substituenten der Definition $R^5$ und/oder $R^6$ in beliebiger Kombination trägt.
Q für Sauerstoff oder Schwefel steht,
$R^5$ für Wasserstoff, Hydroxy, Mercapto, Cyano, Halogen, für jeweils gegebenenfalls durch Cyano, Halogen, $C_1$-$C_4$-Alkoxy, $C_1$-$C_4$-Akylthio, $C_1$-$C_4$-Alkylsulfinyl oder $C_1$-$C_4$-Alkylsulfonyl substituiertes Alkyl, Alkylcarbonyl, Alkoxy, Alkoxycarbonyl, Alkylthio, Alkylsulfinyl oder Alkylsulfonyl mit jeweils bis zu 6 Kohlenstoffatomen in den Alkylgruppen, für jeweils gegebenenfalls durch Halogen substituiertes Alkylamino oder Dialkylamino mit jeweils bis zu 6 Kohlenstoffatomen in den Alkylgruppen, für jeweils gegebenenfalls durch Halogen substituiertes Alkenyl, Alkinyl, Alkenyloxy, Alkenylthio, Alkinylthio oder Alkenylamino mit jeweils bis zu 6 Kohlenstoffatomen in den Alkenyl- bzw. Alkinylgruppen, für jeweils gegebenenfalls durch Halogen substituiertes Cycloalkyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in den Cycloalkylgruppen und gegebenenfalls bis zu 4 Kohlenstoffatomen im Alkylteil, oder für jeweils gegebenenfalls durch Halogen, $C_1$-$C_4$-Alkyl oder $C_1$-$C_4$-Alkoxy substituiertes Phenyl, Phenyloxy, Phenylthio, Phenylamino, Benzyl, Benzyloxy, Benzylthio oder Benzylamino steht, für Pyrrolidino, Piperidino oder Morpholino steht, oder - für den Fall, dass zwei benachbarte Reste $R^5$ und $R^5$ sich an einer Doppelbindung befinden - auch zusammen mit dem benachbarten Rest $R^5$ für eine Benzogruppierung steht, und
$R^6$ für Wasserstoff, Hydroxy, Amino, Alkylidenamino mit bis zu 4 Kohlenstoffatomen, für jeweils gegebenenfalls durch Halogen oder $C_1$-$C_4$-Akoxy substituiertes Alkyl, Alkoxy, Alkylamino, Dialkylamino oder Alkanoylamino mit jeweils bis zu 6 Kohlenstoffatomen in den Alkylgruppen, für jeweils gegebenenfalls durch Halogen substituiertes Alkenyl, Alkinyl oder Alkenyloxy mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl oder Cycloalkylamino mit jeweils 3 bis 6 Kohlenstoffatomen in den Cycloalkylgruppen und gegebenenfalls bis zu 3 Kohlenstoffatomen im Alkylteil, oder für jeweils gegebenenfalls durch Halogen, $C_1$-$C_4$-Alkyl oder $C_1$-$C_4$-Alkoxy substituiertes Phenyl oder Benzyl steht, oder zusammen mit einem benachbarten Rest $R^5$ oder $R^6$ für gegebenenfalls durch Halogen oder $C_1$-$C_4$-Alkyl substituiertes Alkandiyl mit 3 bis 5 Kohlenstoffatomen steht,wobei die einzelnen Reste $R^5$ und $R^6$ - soweit mehrere davon an gleiche heterocyclische Gruppierungen gebunden sind, gleiche oder verschiedene Bedeutungen im Rahmen der obigen Definition haben können.

n steht besonders bevorzugt für die Zahl 0.

A steht besonders bevorzugt für eine Einfachbindung oder für Methylen, Ethyliden (Ethan-1,1-diyl) oder Dimethylen (Ethan-1,2-diyl).

$R^1$ steht besonders bevorzugt für Wasserstoff, Phenyl oder jeweils gegebenenfalls durch Cyano, Fluor, Chlor, Brom, Methoxy, Ethoxy, n- oder i-Propoxy, Methylthio, Ethylthio, n- oder i-Propylthio substituiertes Methyl, Ethyl, n- oder i-Propyl, n-, i- oder s-Butyl.

$R^2$ steht besonders bevorzugt für Wasserstoff oder jeweils gegebenenfalls durch Cyano, Fluor, Chlor, Brom, Methoxy, Ethoxy, n- oder i-Propoxy, Methylthio, Ethylthio, n- oder i-Propylthio substituiertes Methyl, Ethyl, n- oder i-Propyl, n-, i- oder s-Butyl, oder zusammen mit $R^1$ für Methylen, Ethyliden (Ethan-1,1-diyl), Dimethylen (Ethan-1,2-diyl), Propyliden (Propan-1,1-diyl) oder Trimethylen (Propan-1,3-diyl).

$R^3$ steht besonders bevorzugt für Wasserstoff, Nitro, Cyano, Carboxy, Carbamoyl, Thiocarbamoyl, Fluor, Chlor, Brom, Iod, oder für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Dimethylaminosulfonyl oder Diethylaminosulfonyl.

$R^4$ steht besonders bevorzugt für Wasserstoff, Nitro, Cyano, Carboxy, Carbamoyl, Thiocarbamoyl, Fluor, Chlor, Brom, Iod, oder für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Dimethylaminosulfonyl oder Diethylaminosulfonyl.

Y steht besonders bevorzugt für Wasserstoff, für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, für jeweils gegebenenfalls durch Cyano, Fluor, Chlor, Brom, Acetyl, Propionyl, n- oder i-Butyroyl, Methoxycarbonyl, Ethoxycarbonyl, n- oder i-Propoxycarbonyl substituiertes Ethenyl, Propenyl, Butenyl, Pentenyl, Ethinyl, Propinyl oder Butinyl, oder für jeweils gegebenenfalls durch Nitro, Amino, Cyano, Carboxy, Carbamoyl, Thiocarbamoyl, Fluor, Chlor, Brom, Iod, Methyl, Ethyl, n- oder i-Propyl, n-, i-, s- oder t-Butyl, Trifluormethyl, Methoxymethyl, Ethoxymethyl, Methoxyethyl, Ethoxyethyl, Methoxy, Ethoxy, n- oder i-Propoxy, n-, i-, s- oder t-Butoxy, Di-fluormethoxy, Trifluormethoxy, Methylthio, Ethylthio, n- oder i-Propylthio, n-, i-, s- oder t-Butylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl, Acetyl, Propionyl, n- oder i-Butyroyl, Methoxycarbonyl, Ethoxycarbonyl, n- oder i-Propoxycarbonyl, Acetylamino, Propionylamino, Methoxycarbonylamino, Ethoxycarbonylamino, Methylsulfonylamino, Ethylsulfonylamino, n- oder i-Propylsulfonylamino substituiertes Phenyl, Naphthyl, Benzyl oder Phenylethyl.

Z steht besonders bevorzugt für eine der nachstehenden heterocyclischen Gruppierungen

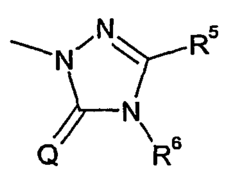

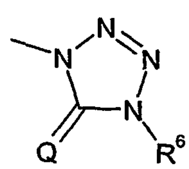

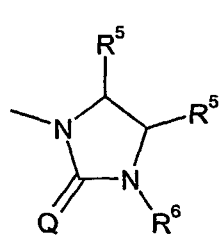

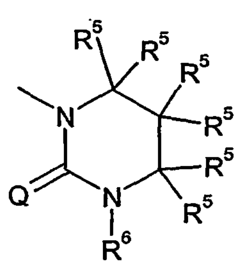

Q steht bevorzugt für Sauerstoff,

$R^5$ steht bevorzugt für Wasserstoff, Hydroxy, Mercapto, Cyano, Fluor, Chlor, Brom, Iod, für jeweils gegebenenfalls durch Fluor, Chlor, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propyl-sulfonyl, für Methylamino, Ethylamino, n- oder i-Propylamino, n-, i-, s- oder t-Butylamino, Dimethylamino, Diethylamino, Di-n-propyl-amino oder Di-i-propylamino, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenyl-thio, Propenylamino oder Butenylamino, für jeweils gegebenenfalls durch Fluor und/oder Chlor substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl, Phenyloxy, Phenylthio, Phenylamino, Benzyl, Benzyloxy, Benzylthio oder Benzylamino, oder - für den Fall, dass zwei benachbarte Reste $R^5$ und $R^5$ sich an einer Doppelbindung befinden - zusammen mit dem benachbarten Rest $R^5$ auch für eine Benzogruppierung,

$R^6$ steht bevorzugt für Wasserstoff, Hydroxy, Amino, für jeweils gegebenenfalls durch Fluor und/oder Chlor, Methoxy oder Ethoxy substituiertes Methyl, Ethyl, n- oder i-Propyl, n-, i- oder s-Butyl, Methoxy, Ethoxy, n- oder i-Propoxy, Methylamino, Ethylamino oder Dimethylamino, für jeweils gegebenenfalls durch Fluor und/oder Chlor substituiertes Ethenyl, Propenyl, Ethinyl, Propinyl oder Propenyloxy, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentyhnethyl oder Cyclohexylmethyl, oder für jeweils gegebenenfalls durch Fluor, Chlor, Methyl, Ethyl, n-oder i-Propyl, n-, i-, s- oder t-Butyl, Methoxy, Ethoxy, n- oder i-Propoxy substituiertes Phenyl oder Benzyl, oder zusammen mit einem benachbarten Rest $R^5$ oder $R^6$ für jeweils gegebenenfalls durch Methyl und/oder Ethyl substituiertes Propan-1,3-diyl (Trimethylen), Butan-1,4-diyl (Tetramethylen) oder Pentan-1,5-diyl (Pentamethylen).

A steht ganz besonders bevorzugt für eine Einfachbindung oder für Methylen.

$R^1$ steht ganz besonders bevorzugt für Wasserstoff, Phenyl oder jeweils gegebenenfalls durch Fluor oder Chlor substituiertes Methyl, Ethyl, n- oder i-Propyl.

$R^2$ steht ganz besonders bevorzugt für Wasserstoff oder jeweils gegebenenfalls durch Fluor oder Chlor substituiertes Methyl, Ethyl, n- oder i-Propyl, oder zusammen mit $R^1$ für Methylen oder Dimethylen.

$R^3$ steht ganz besonders bevorzugt für Wasserstoff, Nitro, Cyano, Fluor, Chlor, Brom, Iod, oder für jeweils gegebenenfalls durch Fluor, Chlor, Methoxy oder Ethoxy substituiertes Methyl, Ethyl, n- oder i-Propyl, Methoxy, Ethoxy, Methylthio, Ethylthio, Methylsulfinyl, Ethylsulfinyl, Methylsulfonyl, Ethylsulfonyl, Methylamino, Ethylamino, Dimethylamino oder Dimethylaminosulfonyl.

$R^4$ steht ganz besonders bevorzugt für Wasserstoff, Nitro, Cyano, Fluor, Chlor, Brom, Iod, oder für jeweils gegebenenfalls durch Fluor, Chlor, Methoxy oder Ethoxy substituiertes Methyl, Ethyl, n- oder i-Propyl, Methoxy, Ethoxy, Methylthio, Ethylthio, Methylsulfinyl, Ethylsulfinyl, Methylsulfonyl, Ethylsulfonyl, Methylamino, Ethylamino, Dimethylamino oder Dimethylaminosulfonyl.

Y steht ganz besonders bevorzugt für Wasserstoff, für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Pentenyl, Propinyl oder Butinyl, oder für jeweils gegebenenfalls durch Nitro, Amino, Cyano, Fluor, Chlor, Brom, Methyl, Ethyl, n- oder i-Propyl, n-, i-, s- oder t-Butyl, Trifluormethyl, Methoxymethyl, Ethoxymethyl, Methoxyethyl, Ethoxyethyl, Methoxy, Ethoxy, n-oder i-Propoxy, Di-fluormethoxy, Trifluormethoxy, Methylthio, Ethylthio, n- oder i-Propylthio, Difluormethylthio, Trifluonnethylthio, Methylsulfinyl, Ethylsulfinyl, Trifluormethylsulfinyl, Methylsulfonyl, Ethylsulfonyl, Trifluormethylsulfonyl, Acetyl, Propionyl, Methoxycarbonyl, Ethoxycarbonyl, Acetylamino, Propionylamino, Methoxycarbonylamino, Ethoxycarbonylamino, Methylsulfonylamino oder Ethylsulfonylamino substituiertes Phenyl oder Benzyl.

Z steht ganz besonders bevorzugt für die nachstehenden heterocyclischen Gruppierungen

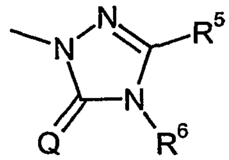 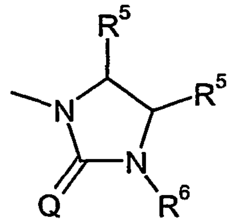 .

$R^5$ steht besonders bevorzugt für Wasserstoff, Fluor, Chlor, Brom, für jeweils gegebenenfalls durch Fluor, Chlor, Methoxy, Ethoxy, n- oder i-Propoxy, Methylthio, Ethylthio, Methylsulfinyl, Ethylsulfinyl, Methylsulfonyl, Ethylsulfonyl substituiertes Methyl, Ethyl, n- oder i-Propyl, n-, i- oder s-Butyl, Methoxy, Ethoxy, n- oder i-Propoxy, n-, i-oder s-Butoxy, Methylthio, Ethylthio, n- oder i-Propylthio, n-, i- oder s-Butylthio, Methylsulfinyl, Ethylsulfinyl, n- oder i-Propylsulfinyl, Methylsulfonyl, Ethylsulfonyl, n- oder i-Propylsulfonyl, für Methyl-amino, Ethylamino, n- oder i-Propylamino, n-, i- oder s-Butylamino, Dimethylamino oder Diethylamino, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, für jeweils gegebenenfalls durch Fluor und/oder Chlor substituiertes Cyclopropyl, Cyclopropyloxy, Cyclopropylthio, Cyclopropylamino, Cyclopropylmethyl, Cyclopropylmethoxy, Cyclopropylmethylthio oder Cyclopropylmethylamino.

$R^6$ steht besonders bevorzugt für Wasserstoff, für Amino, für jeweils gegebenenfalls durch Fluor und/oder Chlor, Methoxy oder Ethoxy substituiertes Methyl, Ethyl, Methoxy, Ethoxy, Methylamino oder Ethylamino, für Dimethylamino, oder für jeweils gegebenenfalls durch Fluor und/oder Chlor substituiertes Cyclopropyl, Cyclopropylmethyl, Phenyl oder Benzyl, oder zusammen mit einem benachbarten Rest $R^5$ oder $R^6$ für jeweils gegebenenfalls durch Methyl und/oder Ethyl substituiertes Propan-1,3-diyl (Trimethylen), Butan-1,4-diyl (Tetramethylen) oder Pentan-1,5-diyl (pentamethylen).

A steht am meisten bevorzugt für Methylen.

$R^1$ steht am meisten bevorzugt für Wasserstoff.

$R^2$ steht am meisten bevorzugt für Wasserstoff.

$R^3$ steht am meisten bevorzugt für Chlor, Trifluormethyl oder Methoxy.

$R^4$ steht am meisten bevorzugt für Chlor, Trifluormethyl oder Methylsulfonyl.

Y steht am meisten bevorzugt für Wasserstoff, für jeweils gegebenenfalls durch Fluor, Chlor oder Methoxy substituiertes Methyl, Ethyl, n- oder i-Propyl, n-, i-, s- oder t-Butyl, für jeweils gegebenenfalls durch Fluor oder Chlor substituiertes Propenyl, Butenyl, Pentenyl oder für gegebenenfalls durch Amino, Cyano, Fluor, Chlor, Methyl, Ethyl, Trifluormethyl, Methoxy oder Ethoxy substituiertes Phenyl.

$R^5$ steht ganz besonders bevorzugt für Wasserstoff, Methyl oder Ethyl.

$R^6$ steht ganz besonders bevorzugt für Wasserstoff, Methyl, Ethyl, n- oder i-Propyl, n-, i-, s- oder t-Butyl, Cyclopropyl, Cyclopropylmethyl, Cyclopentyl, Cyclopentylmethyl, Cyclohexyl, Cyclohexylmethyl, Phenyl oder Benzyl.

$R^5$ steht am meisten bevorzugt für Wasserstoff.

$R^6$ steht am meisten bevorzugt für Methyl, Ethyl, n- oder i-Propyl, n-, i-, s- oder t-Butyl, Cyclopropyl, Cyclopropylmethyl oder Phenyl.

Gegenstand der Erfindung sind vorzugsweise auch Natrium-, Kalium-, Magnesium-, Calcium-, Ammonium-, $C_1$-$C_4$-Alkyl-ammonium-, Di-($C_1$-$C_4$-alkyl)-ammonium-, Tri-($C_1$-$C_4$-alkyl)-ammonium-, Tetra-($C_1$-$C_4$-alkyl)-ammonium, Tri-($C_1$-$C_4$-alkyl)-sulfonium-, $C_5$- oder $C_6$-Cycloalkyl-ammonium- und Di-($C_1$-$C_2$-alkyl)-benzylammonium-Salze von Verbindungen der Formel (I), in welcher A, n, $R^1$, $R^2$, $R^3$, $R^4$, Yund Z die oben angegebenen Bedeutungen haben, oder auch Komplexverbindungen (Koordinationsverbindungen) dieser Verbindungen mit Metallen wie Kupfer, Eisen, Kobalt, Nickel.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Erfindungsgemäß ganz besonders bevorzugt sind diejenigen Verbindungen der Formel (I), bei welchen eine Kombination der vorstehend als ganz besonders bevorzugt aufgeführten Bedeutungen vorliegt.

Erfindungsgemäß am meisten bevorzugt sind diejenigen Verbindungen der Formel (I), bei welchen eine Kombination der vorstehend als am meisten bevorzugt aufgeführten Bedeutungen vorliegt.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Die Position der Reste $R^1$, $R^2$, $R^3$, $R^4$ und A-Z kann gemäß Formel (I) variieren.

Der Rest $R^3$ findet sich bevorzugt in Position (4) am Phenylring, besonders bevorzugt in Position (2).

Der Rest $R^4$ findet sich bevorzugt in Position (4) am Phenylring, wenn der Rest $R^3$ sich an Position (2) befindet.

Der Rest A-Z findet sich bevorzugt in Position (2) am Phenylring, besonders bevorzugt in Position (3), wobei das Substitutionsmuster (2)-$R^3$, (4)-$R^4$ und (3)-A-Z besonders bevorzugt ist.

Die Verbindungen der nachstehenden allgemeinen Formeln (IA), (IB) und (IC) sind ganz besonders Gegenstand der vorliegenden Erfindung.

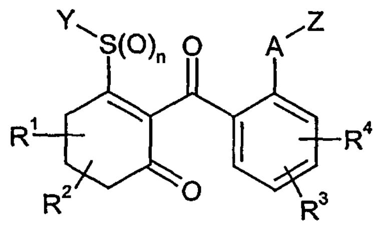

(IA)

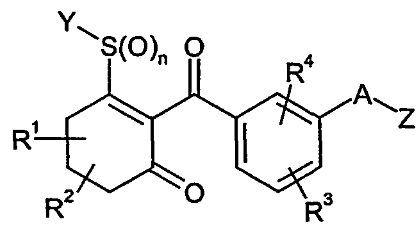

(IB)

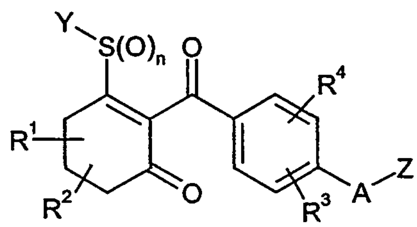

(IC)

n, A, $R^1$, $R^2$, $R^3$, $R^4$, Y und Z haben in den allgemeinen Formeln (IA), (IB) und (IC) jeweils die in den vorausgehenden Definitionen angegebenen Bedeutungen.

Die Verbindungen der allgemeinen Formeln (IA), (IB) und (IC), bei welchen A für Methylen steht, sind ganz besonders hervorzuheben.

Beispiele für die erfindungsgemäßen Verbindungen der allgemeinen Formel (I) sind in den nachstehenden Gruppen aufgeführt.

Gruppe 1

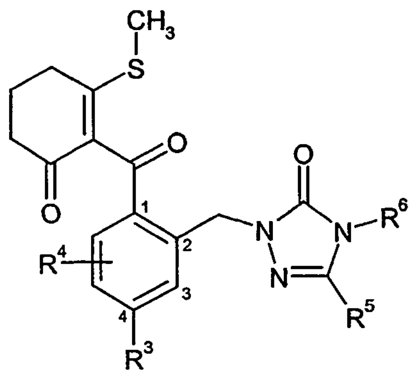

(IA-1)

$R^3$, $R^4$, $R^5$ und $R^6$ haben hierbei beispielhaft die in der nachstehenden Tabelle angegebenen Bedeutungen:

| $R^3$ | (Position-) $R^4$ | $R^5$ | $R^6$ |
|---|---|---|---|
| H | - | $CF_3$ | $CH_3$ |
| F | - | $CF_3$ | $CH_3$ |
| Cl | - | $CF_3$ | $CH_3$ |
| Br | - | $CF_3$ | $CH_3$ |
| I | - | $CF_3$ | $CH_3$ |
| $NO_2$ | - | $CF_3$ | $CH_3$ |
| CN | - | $CF_3$ | $CH_3$ |
| $CH_3$ | - | $CF_3$ | $CH_3$ |
| $OCH_3$ | - | $CF_3$ | $CH_3$ |
| $CF_3$ | - | $CF_3$ | $CH_3$ |
| $OCHF_2$ | - | $CF_3$ | $CH_3$ |
| $OCF_3$ | - | $CF_3$ | $CH_3$ |
| $SO_2CH_3$ | - | $CF_3$ | $CH_3$ |
| H | - | $OCH_3$ | $CH_3$ |
| F | - | $OCH_3$ | $CH_3$ |
| Cl | - | $OCH_3$ | $CH_3$ |
| Br | - | $OCH_3$ | $CH_3$ |
| I | - | $OCH_3$ | $CH_3$ |
| $NO_2$ | - | $OCH_3$ | $CH_3$ |
| CN | - | $OCH_3$ | $CH_3$ |
| $CH_3$ | - | $OCH_3$ | $CH_3$ |
| $OCH_3$ | - | $OCH_3$ | $CH_3$ |
| $CF_3$ | - | $OCH_3$ | $CH_3$ |
| $OCHF_2$ | - | $OCH_3$ | $CH_3$ |
| $OCF_3$ | - | $OCH_3$ | $CH_3$ |
| $SO_2CH_3$ | - | $OCH_3$ | $CH_3$ |
| H | - | $SCH_3$ | $CH_3$ |

(fortgesetzt)

| $R^3$ | (Position-) $R^4$ | $R^5$ | $R^6$ |
|---|---|---|---|
| F | - | $SCH_3$ | $CH_3$ |
| Cl | - | $SCH_3$ | $CH_3$ |
| Br | - | $SCH_3$ | $CH_3$ |
| I | - | $SCH_3$ | $CH_3$ |
| $NO_2$ | - | $SCH_3$ | $CH_3$ |
| CN | - | $SCH_3$ | $CH_3$ |
| $CH_3$ | - | $SCH_3$ | $CH_3$ |
| $OCH_3$ | - | $SCH_3$ | $CH_3$ |
| $CF_3$ | - | $SCH_3$ | $CH_3$ |
| $OCHF_2$ | - | $SCH_3$ | $CH_3$ |
| $OCF_3$ | - | $SCH_3$ | $CH_3$ |
| $SO_2CH_3$ | - | $SCH_3$ | $CH_3$ |
| H | - | $OC_2H_5$ | $CH_3$ |
| F | - | $OC_2H_5$ | $CH_3$ |
| Cl | - | $OC_2H_5$ | $CH_3$ |
| Br | - | $OC_2H_5$ | $CH_3$ |
| I | - | $OC_2H_5$ | $CH_3$ |
| $NO_2$ | - | $OC_2H_5$ | $CH_3$ |
| CN | - | $OC_2H_5$ | $CH_3$ |
| $CH_3$ | - | $OC_2H_5$ | $CH_3$ |
| $OCH_3$ | - | $OC_2H_5$ | $CH_3$ |
| $CF_3$ | - | $OC_2H_5$ | $CH_3$ |
| $OCHF_2$ | - | $OC_2H_5$ | $CH_3$ |
| $OCF_3$ | - | $OC_2H_5$ | $CH_3$ |
| $SO_2CH_3$ | - | $OC_2H_5$ | $CH_3$ |
| H | - | $N(CH_3)_2$ | $CH_3$ |
| F | - | $N(CH_3)_2$ | $CH_3$ |
| Cl | - | $N(CH_3)_2$ | $CH_3$ |
| Br | - | $N(CH_3)_2$ | $CH_3$ |
| I | - | $N(CH_3)_2$ | $CH_3$ |
| $NO_2$ | - | $N(CH_3)_2$ | $CH_3$ |
| CN | - | $N(CH_3)_2$ | $CH_3$ |
| $CH_3$ | - | $N(CH_3)_2$ | $CH_3$ |
| $OCH_3$ | - | $N(CH_3)_2$ | $CH_3$ |
| $CF_3$ | - | $N(CH_3)_2$ | $CH_3$ |
| $OCHF_2$ | - | $N(CH_3)_2$ | $CH_3$ |
| $OCF_3$ | - | $N(CH_3)_2$ | $CH_3$ |
| $SO_2CH_3$ | - | $N(CH_3)_2$ | $CH_3$ |

(fortgesetzt)

| $R^3$ | (Position-) $R^4$ | $R^5$ | $R^6$ |
|---|---|---|---|
| H | - | $OCH_3$ | 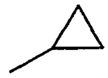 |
| F | - | $OCH_3$ | 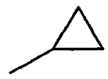 |
| Cl | - | $OCH_3$ | 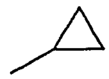 |
| Br | - | $OCH_3$ | 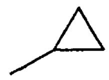 |
| I | - | $OCH_3$ | 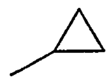 |
| $NO_2$ | - | $OCH_3$ | 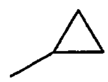 |
| CN | - | $OCH_3$ | 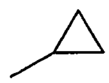 |
| $CH_3$ | - | $OCH_3$ | 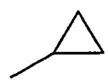 |
| $OCH_3$ | - | $OCH_3$ | 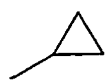 |
| $CF_3$ | - | $OCH_3$ | 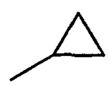 |
| $OCHF_2$ | - | $OCH_3$ | 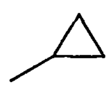 |
| $OCF_3$ | - | $OCH_3$ | 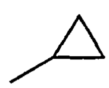 |
| $SO_2CH_3$ | - | $OCH_3$ | 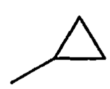 |
| H | (3-) Cl | $CF_3$ | $CH_3$ |
| F | (3-) Cl | $CH_3$ | $CH_3$ |
| Cl | (3-) Cl | $OCH_3$ | $CH_3$ |
| Br | (3-) Cl | Br | 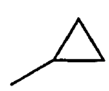 |

(fortgesetzt)

| $R^3$ | (Position-) $R^4$ | $R^5$ | $R^6$ |
|---|---|---|---|
| Cl | (3-) Cl | $CF_3$ | $CH_3$ |
| $NO_2$ | (3-) Cl | $CH_3$ | $CH_3$ |
| Cl | (3-) Cl | $SCH_3$ | $CH_3$ |
| $CH_3$ | (3-) Cl | Cl | $CH_3$ |
| $OCH_3$ | (3-) Cl | $OCH_3$ | $CH_3$ |
| $CF_3$ | (3-) Cl | $CF_3$ | $CH_3$ |
| $OCHF_2$ | (3-) Cl | $CH_3$ | $CH_3$ |
| $OCF_3$ | (3-) Cl | $CH_3$ | $CH_3$ |
| $SO_2CH_3$ | (3-) Cl | $OCH_3$ | $CH_3$ |
| F | - | $C_2H_5$ | $OCH_3$ |
| Cl | - | $C_2H_5$ | $OCH_3$ |
| Br | - | $C_2H_5$ | $OCH_3$ |
| $CF_3$ | - | $C_2H_5$ | $OCH_3$ |
| $SO_2CH_3$ | - | $C_2H_5$ | $OCH_3$ |
| F | - | $C_2H_5$ | $C_2H_5$ |
| Cl | - | $C_2H_5$ | $C_2H_5$ |
| Br | - | $C_2H_5$ | $C_2H_5$ |
| $CF_3$ | - | $C_2H_5$ | $C_2H_5$ |
| $SO_2CH_3$ | - | $C_2H_5$ | $C_2H_5$ |

Gruppe 2

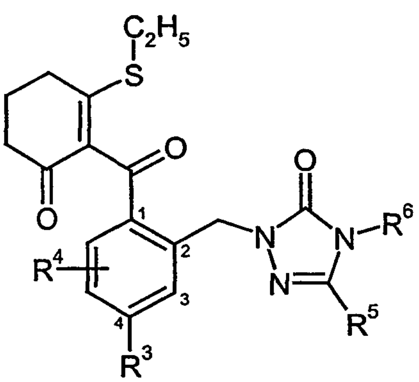

(IA-2)

$R^3$, $R^4$, $R^5$ und $R^6$ haben hierbei beispielhaft die vorausgehend in Gruppe 1 angegebenen Bedeutungen.

Gruppe 3

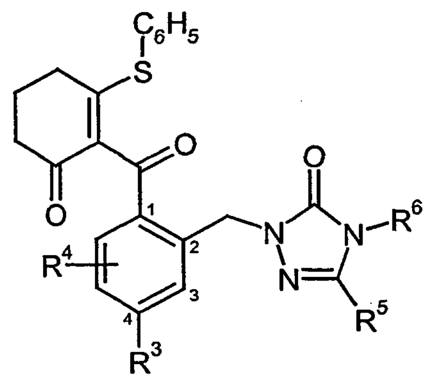

(IA-3)

$R^3$, $R^4$, $R^5$ und $R^6$ haben hierbei beispielhaft die vorausgehend in Gruppe 1 angegebenen Bedeutungen.

<u>Gruppe 4</u>

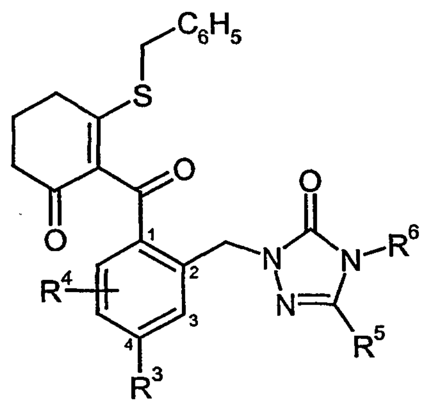

(IA-4)

$R^3$, $R^4$, $R^5$ und $R^6$ haben hierbei beispielhaft die vorausgehend in Gruppe 1 angegebenen Bedeutungen.

<u>Gruppe 5</u>

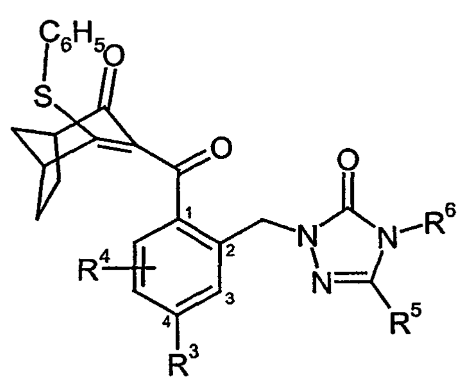

(IA-5)

$R^3$, $R^4$, $R^5$ und $R^6$ haben hierbei beispielhaft die vorausgehend in Gruppe 1 angegebenen Bedeutungen.

Gruppe 6

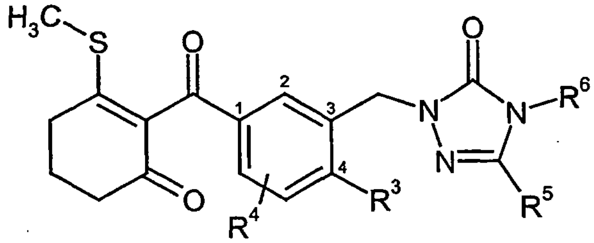

(IB-1)

$R^3$, $R^4$, $R^5$ und $R^6$ haben hierbei beispielhaft die in der nachstehenden Tabelle angegebenen Bedeutungen:

| $R^3$ | (Position-) $R^4$ | $R^5$ | $R^6$ |
|---|---|---|---|
| Cl | (2-) Cl | $CF_3$ | $CH_3$ |
| Cl | (2-) Cl | $SCH_3$ | $CH_3$ |
| Cl | (2-) Cl | $SC_2H_5$ | $CH_3$ |
| Cl | (2-) Cl | $SC_3H_7$ | $CH_3$ |
| Cl | (2-) Cl | $SC_3H_7$-i | $CH_3$ |
| Cl | (2-) Cl | 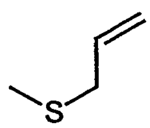 | $CH_3$ |
| Cl | (2-) Cl | 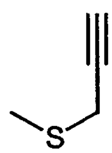 | $CH_3$ |
| Cl | (2-) Cl | 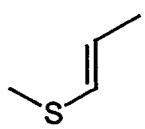 | $CH_3$ |
| Cl | (2-) Cl | 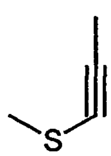 | $CH_3$ |
| Cl | (2-) Cl | 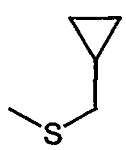 | $CH_3$ |
| Cl | (2-) Cl | $SCH=C=CH_2$ | $CH_3$ |
| Cl | (2-) Cl | $SCH_2CN$ | $CH_3$ |
| Cl | (2-) Cl | $SCH_2CH_2CN$ | $CH_3$ |
| Cl | (2-) Cl | $OCH_3$ | $CH_3$ |

(fortgesetzt)

| $R^3$ | (Position-) $R^4$ | $R^5$ | $R^6$ |
|---|---|---|---|
| Cl | (2-) Cl | $OC_2H_5$ | $CH_3$ |
| Cl | (2-) Cl | $OC_3H_7$ | $CH_3$ |
| Cl | (2-) Cl | $OC_3H_7$-i | $CH_3$ |
| Cl | (2-) Cl | $OC_4H_9$ | $CH_3$ |
| Cl | (2-) Cl | $OCH_2CF_3$ | $CH_3$ |
| Cl | (2-) Cl | 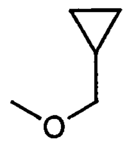 | $CH_3$ |
| Cl | (2-) Cl | $OC_6H_5$ | $CH_3$ |
| Cl | (2-) Cl | H | $CH_3$ |
| Cl | (2-) Cl | $CH_3$ | $CH_3$ |
| Cl | (2-) Cl | $C_2H_5$ | $CH_3$ |
| Cl | (2-) Cl | $C_3H_7$ | $CH_3$ |
| Cl | (2-) Cl | $C_3H_7$-i | $CH_3$ |
| Cl | (2-) Cl | $C_4H_9$ | $CH_3$ |
| Cl | (2-) Cl | $C_4H_9$-i | $CH_3$ |
| Cl | (2-)Cl | $C_4H_9$-s | $CH_3$ |
| Cl | (2-) Cl | $C_4H_9$-t | $CH_3$ |
| Cl | (2-) Cl | 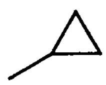 | $CH_3$ |
| Cl | (2-) Cl | 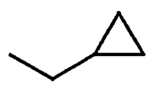 | $CH_3$ |
| Cl | (2-) Cl | CH=$CHCH_3$ | $CH_3$ |
| Cl | (2-) Cl | 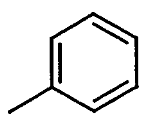 | $CH_3$ |
| Cl | (2-) Cl | 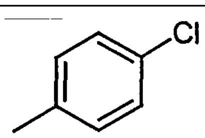 | $CH_3$ |
| Cl | (2-) Cl | 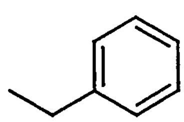 | $CH_3$ |
| Cl | (2-) Cl | $N(CH_3)_2$ | $CH_3$ |

(fortgesetzt)

| $R^3$ | (Position-) $R^4$ | $R^5$ | $R^6$ |
|---|---|---|---|
| Cl | (2-) Cl | 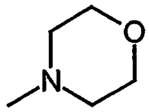 | $CH_3$ |
| Cl | (2-) Cl | Cl | $CH_3$ |
| Cl | (2-) Cl | Br | $CH_3$ |
| $SO_2CH_3$ | (2-) Cl | $CF_3$ | $CH_3$ |
| $SO_2CH_3$ | (2-) Cl | $SCH_3$ | $CH_3$ |
| $SO_2CH_3$ | (2-) Cl | $SC_2H_5$ | $CH_3$ |
| $SO_2CH_3$ | (2-) Cl | $SC_3H_7$ | $CH_3$ |
| $SO_2CH_3$ | (2-) Cl | $SC_3H_7$-i | $CH_3$ |
| $SO_2CH_3$ | (2-) Cl | 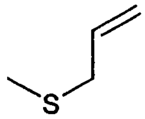 | $CH_3$ |
| $SO_2CH_3$ | (2-) Cl | 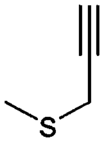 | $CH_3$ |
| $SO_2CH_3$ | (2-) Cl | 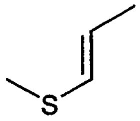 | $CH_3$ |
| $SO_2CH_3$ | (2-) Cl | 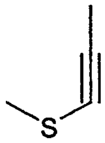 | $CH_3$ |
| $SO_2CH_3$ | (2-) Cl | 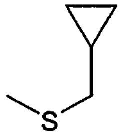 | $CH_3$ |
| $SO_2CH_3$ | (2-) Cl | $SCH=C=CH_2$ | $CH_3$ |
| $SO_2CH_3$ | (2-) Cl | $SCH_2CN$ | $CH_3$ |
| $SO_2CH_3$ | (2-) Cl | $SCH_2CH_2CN$ | $CH_3$ |
| $SO_2CH_3$ | (2-) Cl | $OCH_3$ | $CH_3$ |
| $SO_2CH_3$ | (2-) Cl | $OC_2H_5$ | $CH_3$ |
| $SO_2CH_3$ | (2-) Cl | $OC_3H_7$ | $CH_3$ |
| $SO_2CH_3$ | (2-) Cl | $OC_3H_7$-i | $CH_3$ |
| $SO_2CH_3$ | (2-) Cl | $OC_4H_9$ | $CH_3$ |
| $SO_2CH_3$ | (2-) Cl | $OCH_2CF_3$ | $CH_3$ |

(fortgesetzt)

| $R^3$ | (Position-) $R^4$ | $R^5$ | $R^6$ |
|---|---|---|---|
| $SO_2CH_3$ | (2-) Cl | 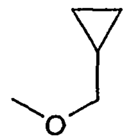 | $CH_3$ |
| $SO_2CH_3$ | (2-) Cl | $OC_6H_5$ | $CH_3$ |
| $SO_2CH_3$ | (2-) Cl | H | $CH_3$ |
| $SO_2CH_3$ | (2-) Cl | $CH_3$ | $CH_3$ |
| $SO_2CH_3$ | (2-) Cl | $C_2H_5$ | $CH_3$ |
| $SO_2CH_3$ | (2-) Cl | $C_3H_7$ | $CH_3$ |
| $SO_2CH_3$ | (2-) Cl | $C_3H_7$-i | $CH_3$ |
| $SO_2CH_3$ | (2-) Cl | $C_4H_9$ | $CH_3$ |
| $SO_2CH_3$ | (2-) Cl | $C_4H_9$-i | $CH_3$ |
| $SO_2CH_3$ | (2-) Cl | $C_4H_9$-s | $CH_3$ |
| $SO_2CH_3$ | (2-) Cl | $C_4H_9$-t | $CH_3$ |
| $SO_2CH_3$ | (2-) Cl | 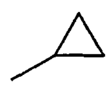 | $CH_3$ |
| $SO_2CH_3$ | (2-) Cl | 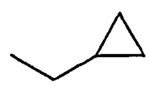 | $CH_3$ |
| $SO_2CH_3$ | (2-) Cl | $CH=CHCH_3$ | $CH_3$ |
| $SO_2CH_3$ | (2-) Cl | 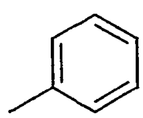 | $CH_3$ |
| $SO_2CH_3$ | (2-) Cl | 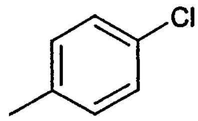 | $CH_3$ |
| $SO_2CH_3$ | (2-) Cl | 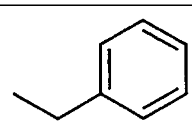 | $CH_3$ |
| $SO_2CH_3$ | (2-) Cl | $N(CH_3)_2$ | $CH_3$ |
| $SO_2CH_3$ | (2-) Cl | 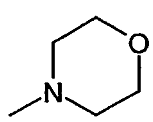 | $CH_3$ |
| $SO_2CH_3$ | (2-) Cl | Cl | $CH_3$ |
| $SO_2CH_3$ | (2-) Cl | Br | $CH_3$ |
| Cl | (2-) $SO_2CH_3$ | $CF_3$ | $CH_3$ |
| Cl | (2-) $SO_2CH_3$ | $SCH_3$ | $CH_3$ |

(fortgesetzt)

| $R^3$ | (Position-) $R^4$ | $R^5$ | $R^6$ |
|---|---|---|---|
| Cl | (2-) $SO_2CH_3$ | $SC_2H_5$ | $CH_3$ |
| Cl | (2-) $SO_2CH_3$ | $SC_3H_7$ | $CH_3$ |
| Cl | (2-) $SO_2CH_3$ | $SC_3H_7$-i | $CH_3$ |
| Cl | (2-) $SO_2CH_3$ | 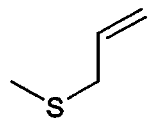 | $CH_3$ |
| Cl | (2-) $SO_2CH_3$ | 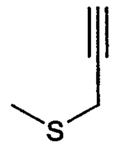 | $CH_3$ |
| Cl | (2-) $SO_2CH_3$ | 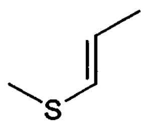 | $CH_3$ |
| Cl | (2-) $SO_2CH_3$ | 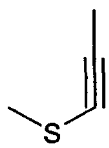 | $CH_3$ |
| Cl | (2-) $SO_2CH_3$ | 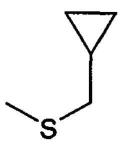 | $CH_3$ |
| Cl | (2-) $SO_2CH_3$ | $SCH=C=CH_2$ | $CH_3$ |
| Cl | (2-) $SO_2CH_3$ | $SCH_2CN$ | $CH_3$ |
| Cl | (2-) $SO_2CH_3$ | $SCH_2CH_2CN$ | $CH_3$ |
| Cl | (2-) $SO_2CH_3$ | $OCH_3$ | $CH_3$ |
| Cl | (2-) $SO_2CH_3$ | $OC_2H_5$ | $CH_3$ |
| Cl | (2-) $SO_2CH_3$ | $OC_3H_7$ | $CH_3$ |
| Cl | (2-) $SO_2CH_3$ | $OC_3H_7$-i | $CH_3$ |
| Cl | (2-) $SO_2CH_3$ | $OC_4H_9$ | $CH_3$ |
| Cl | (2-) $SO_2CH_3$ | $OCH_2CF_3$ | $CH_3$ |
| Cl | (2-) $SO_2CH_3$ | 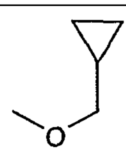 | $CH_3$ |
| Cl | (2-) $SO_2CH_3$ | $OC_6H_5$ | $CH_3$ |
| Cl | (2-) $SO_2CH_3$ | H | $CH_3$ |
| Cl | (2-) $SO_2CH_3$ | $CH_3$ | $CH_3$ |
| Cl | (2-) $SO_2CH_3$ | $C_2H_5$ | $CH_3$ |

(fortgesetzt)

| $R^3$ | (Position-) $R^4$ | $R^5$ | $R^6$ |
|---|---|---|---|
| Cl | (2-) $SO_2CH_3$ | $C_3H_7$ | $CH_3$ |
| Cl | (2-) $SO_2CH_3$ | $C_3H_7$-i | $CH_3$ |
| Cl | (2-) $SO_2CH_3$ | $C_4H_9$ | $CH_3$ |
| Cl | (2-) $SO_2CH_3$ | $C_4H_9$-i | $CH_3$ |
| Cl | (2-) $SO_2CH_3$ | $C_4H_9$-s | $CH_3$ |
| Cl | (2-) $SO_2CH_3$ | $C_4H_9$-t | $CH_3$ |
| Cl | (2-) $SO_2CH_3$ | 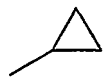 | $CH_3$ |
| Cl | (2-) $SO_2CH_3$ | 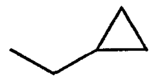 | $CH_3$ |
| Cl | (2-) $SO_2CH_3$ | CH=$CHCH_3$ | $CH_3$ |
| Cl | (2-) $SO_2CH_3$ | 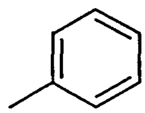 | $CH_3$ |
| Cl | (2-) $SO_2CH_3$ | 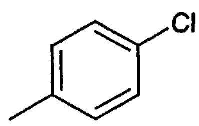 | $CH_3$ |
| Cl | (2-) $SO_2CH_3$ | 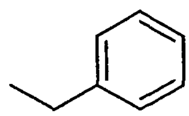 | $CH_3$ |
| Cl | (2-) $SO_2CH_3$ | $N(CH_3)_2$ | $CH_3$ |
| Cl | (2-) $SO_2CH_3$ | 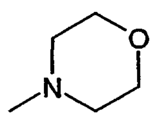 | $CH_3$ |
| Cl | (2-) $SO_2CH_3$ | Cl | $CH_3$ |
| Cl | (2-) $SO_2CH_3$ | Br | $CH_3$ |
| Cl | (2-) Cl | $CF_3$ | 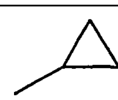 |
| Cl | (2-) Cl | $SCH_3$ | 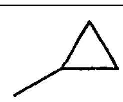 |
| Cl | (2-) Cl | $SC_2H_5$ | 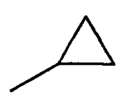 |

(fortgesetzt)

| $R^3$ | (Position-) $R^4$ | $R^5$ | $R^6$ |
|---|---|---|---|
| Cl | (2-) Cl | $SC_3H_7$ | 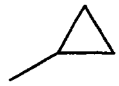 |
| Cl | (2-) Cl | $SC_3H_7$-i | 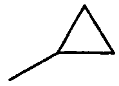 |
| Cl | (2-) Cl | 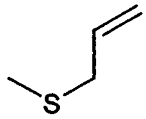 | 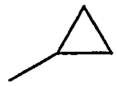 |
| Cl | (2-) Cl | 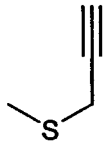 | 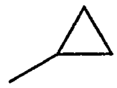 |
| Cl | (2-) Cl | 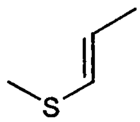 | 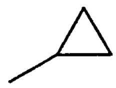 |
| Cl | (2-) Cl | 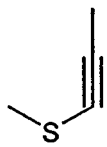 | 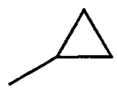 |
| Cl | (2-) Cl | 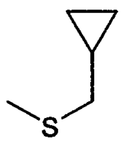 | 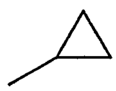 |
| Cl | (2-) Cl | $SCH{=}C{=}CH_2$ | 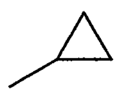 |
| Cl | (2-) Cl | $SCH_2CN$ | 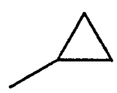 |
| Cl | (2-) Cl | $SCH_2CH_2CN$ | 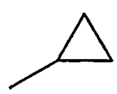 |
| Cl | (2-) Cl | $OCH_3$ | 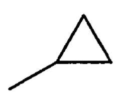 |
| Cl | (2-) Cl | $OC_2H_5$ | 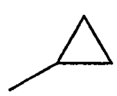 |

(fortgesetzt)

| $R^3$ | (Position-) $R^4$ | $R^5$ | $R^6$ |
|---|---|---|---|
| Cl | (2-) Cl | $OC_3H_7$ | 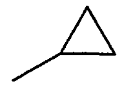 |
| Cl | (2-) Cl | $OC_3H_7$-i | 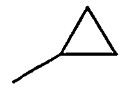 |
| Cl | (2-) Cl | $OC_4H_9$ | 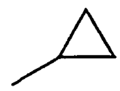 |
| Cl | (2-) Cl | $OCH_2CF_3$ | 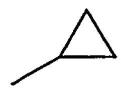 |
| Cl | (2-) Cl | 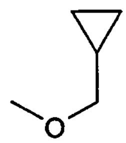 | 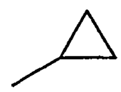 |
| Cl | (2-) Cl | $OC_6H_5$ | 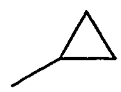 |
| Cl | (2-) Cl | H | 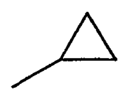 |
| Cl | (2-) Cl | $CH_3$ | 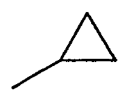 |
| Cl | (2-) Cl | $C_2H_5$ | 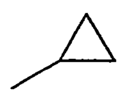 |
| Cl | (2-) Cl | $C_3H_7$ | 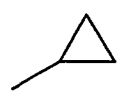 |
| Cl | (2-) Cl | $C_3H_7$-i | 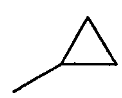 |
| Cl | (2-) Cl | $C_4H_9$ | 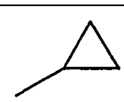 |
| Cl | (2-) Cl | $C_4H_9$-i | 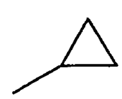 |

(fortgesetzt)

| $R^3$ | (Position-) $R^4$ | $R^5$ | $R^6$ |
|---|---|---|---|
| Cl | (2-) Cl | $C_4H_9$-s | 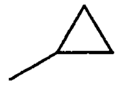 |
| Cl | (2-) Cl | $C_4H_9$-t | 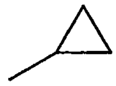 |
| Cl | (2-) Cl | 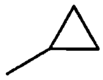 | 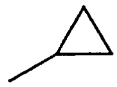 |
| Cl | (2-) Cl | 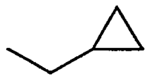 | 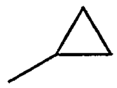 |
| Cl | (2-) Cl | $CH=CHCH_3$ | 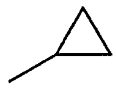 |
| Cl | (2-) Cl | 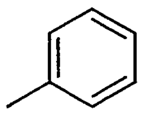 | 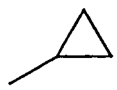 |
| Cl | (2-) Cl | 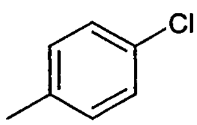 | 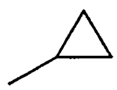 |
| Cl | (2-) Cl | 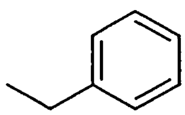 | 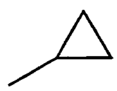 |
| Cl | (2-) Cl | $N(CH_3)_2$ | 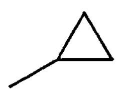 |
| Cl | (2-) Cl | 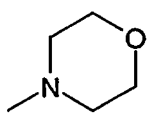 | 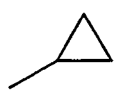 |
| Cl | (2-) Cl | Cl | 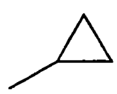 |
| Cl | (2-) Cl | Br | 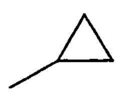 |
| $SO_2CH_3$ | (2-) Cl | $CF_3$ | 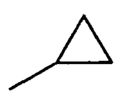 |

(fortgesetzt)

| $R^3$ | (Position-) $R^4$ | $R^5$ | $R^6$ |
|---|---|---|---|
| $SO_2CH_3$ | (2-) Cl | $SCH_3$ | 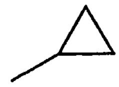 |
| $SO_2CH_3$ | (2-) Cl | $SC_2H_5$ | 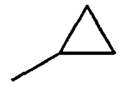 |
| $SO_2CH_3$ | (2-) Cl | $SC_3H_7$ | 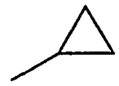 |
| $SO_2CH_3$ | (2-) Cl | $SC_3H_7$-i | 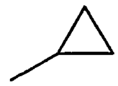 |
| $SO_2CH_3$ | (2-) Cl | 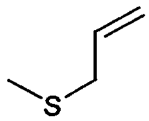 | 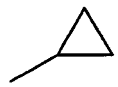 |
| $SO_2CH_3$ | (2-) Cl | 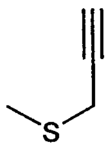 | 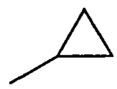 |
| $SO_2CH_3$ | (2-) Cl | 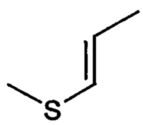 | 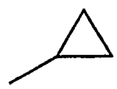 |
| $SO_2CH_3$ | (2-) Cl | 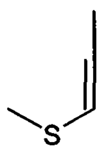 | 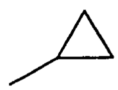 |
| $SO_2CH_3$ | (2-) Cl | 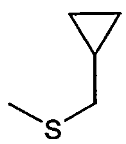 | 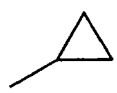 |
| $SO_2CH_3$ | (2-) Cl | $SCH=C=CH_2$ | 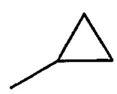 |
| $SO_2CH_3$ | (2-) Cl | $SCH_2CN$ | 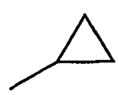 |
| $SO_2CH_3$ | (2-) Cl | $SCH_2CH_2CN$ | 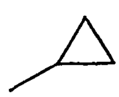 |

(fortgesetzt)

| $R^3$ | (Position-) $R^4$ | $R^5$ | $R^6$ |
|---|---|---|---|
| $SO_2CH_3$ | (2-) Cl | $OCH_3$ | 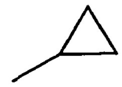 |
| $SO_2CH_3$ | (2-) Cl | $OC_2H_5$ | 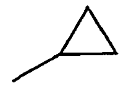 |
| $SO_2CH_3$ | (2-) Cl | $OC_3H_7$ | 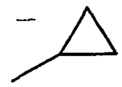 |
| $SO_2CH_3$ | (2-) Cl | $OC_3H_7$-i | 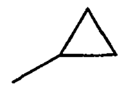 |
| $SO_2CH_3$ | (2-) Cl | $OC_4H_9$ | 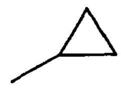 |
| $SO_2CH_3$ | (2-) Cl | $OCH_2CF_3$ | 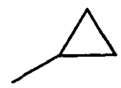 |
| $SO_2CH_3$ | (2-) Cl | 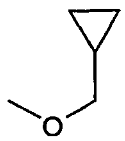 | 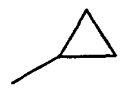 |
| $SO_2CH_3$ | (2-) Cl | $OC_6H_5$ | 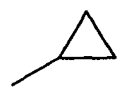 |
| $SO_2CH_3$ | (2-) Cl | H | 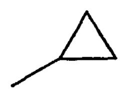 |
| $SO_2CH_3$ | (2-) Cl | $CH_3$ | 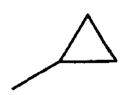 |
| $SO_2CH_3$ | (2-) Cl | $C_2H_5$ | 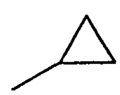 |
| $SO_2CH_3$ | (2-) Cl | $C_3H_7$ | 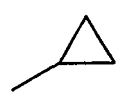 |
| $SO_2CH_3$ | (2-) Cl | $C_3H_7$-i | 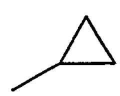 |
| $SO_2CH_3$ | (2-) Cl | $C_4H_9$ | 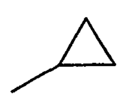 |

(fortgesetzt)

| $R^3$ | (Position-) $R^4$ | $R^5$ | $R^6$ |
|---|---|---|---|
| $SO_2CH_3$ | (2-) Cl | $C_4H_9$-1 | 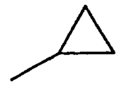 |
| $SO_2CH_3$ | (2-) Cl | $C_4H_9$-s | 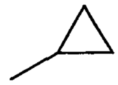 |
| $SO_2CH_3$ | (2-) Cl | $C_4H_9$-t | 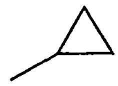 |
| $SO_2CH_3$ | (2-) Cl | 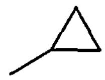 | 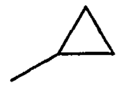 |
| $SO_2CH_3$ | (2-) Cl | 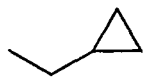 | 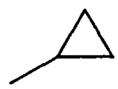 |
| $SO_2CH_3$ | (2-) Cl | CH=CHCH$_3$ | 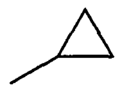 |
| $SO_2CH_3$ | (2-) Cl | 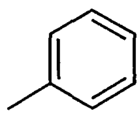 | 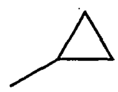 |
| $SO_2CH_3$ | (2-) Cl | 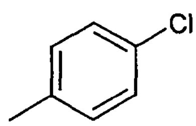 | 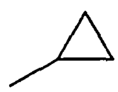 |
| $SO_2CH_3$ | (2-) Cl | 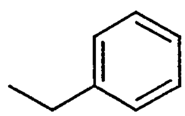 | 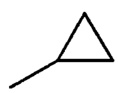 |
| $SO_2CH_3$ | (2-) Cl | $N(CH_3)_2$ | 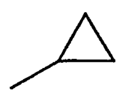 |
| $SO_2CH_3$ | (2-) Cl | 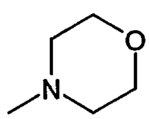 | 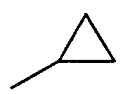 |
| $SO_2CH_3$ | (2-) Cl | Cl | 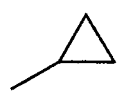 |
| $SO_2CH_3$ | (2-) Cl | Br | 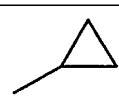 |

(fortgesetzt)

| $R^3$ | (Position-) $R^4$ | $R^5$ | $R^6$ |
|---|---|---|---|
| Cl | (2-) $SO_2CH_3$ | $CF_3$ | 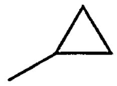 |
| Cl | (2-) $SO_2CH_3$ | $SCH_3$ | 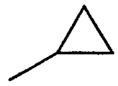 |
| Cl | (2-) $SO_2CH_3$ | $SC_2H_5$ | 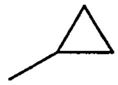 |
| Cl | (2-) $SO_2CH_3$ | $SC_3H_7$ | 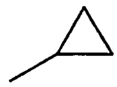 |
| Cl | (2-) $SO_2CH_3$ | $SC_3H_7$-i | 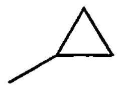 |
| Cl | (2-) $SO_2CH_3$ | 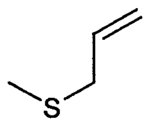 | 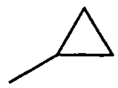 |
| Cl | (2-) $SO_2CH_3$ | 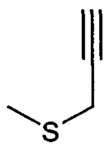 | 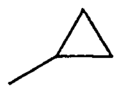 |
| Cl | (2-) $SO_2CH_3$ | 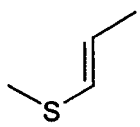 | 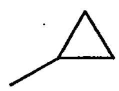 |
| Cl | (2-) $SO_2CH_3$ | 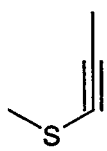 | 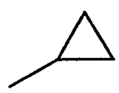 |
| Cl | (2-) $SO_2CH_3$ | 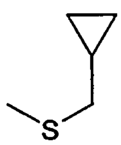 | 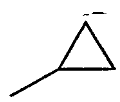 |
| Cl | (2-) $SO_2CH_3$ | $SCH{=}C{=}CH_2$ | 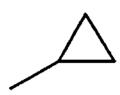 |
| Cl | (2-) $SO_2CH_3$ | $SCH_2CN$ | 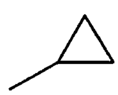 |

(fortgesetzt)

| $R^3$ | (Position-) $R^4$ | $R^5$ | $R^6$ |
|---|---|---|---|
| Cl | (2-) $SO_2CH_3$ | $SCH_2CH_2CN$ | 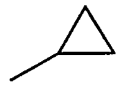 |
| Cl | (2-) $SO_2CH_3$ | $OCH_3$ | 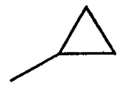 |
| Cl | (2-) $SO_2CH_3$ | $OC_2H_5$ | 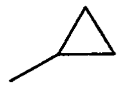 |
| Cl | (2-) $SO_2CH_3$ | $OC_3H_7$ | 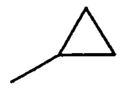 |
| Cl | (2-) $SO_2CH_3$ | $0C_3H_7$-i | 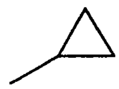 |
| Cl | (2-) $SO_2CH_3$ | $OC_4H_9$ | 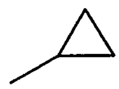 |
| Cl | (2-) $SO_2CH_3$ | $OCH_2CF_3$ | 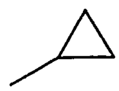 |
| Cl | (2-) $SO_2CH_3$ | 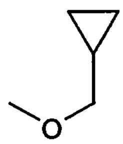 | 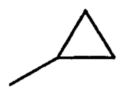 |
| Cl | (2-) $SO_2CH_3$ | $OC_6H_5$ | 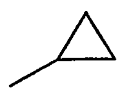 |
| Cl | (2-) $SO_2CH_3$ | H | 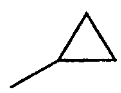 |
| Cl | (2-) $SO_2CH_3$ | $CH_3$ | 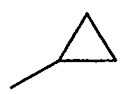 |
| Cl | (2-) $SO_2CH_3$ | $C_2H_5$ | 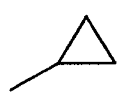 |
| Cl | (2-) $SO_2CH_3$ | $C_3H_7$ | 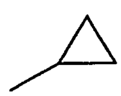 |

(fortgesetzt)

| $R^3$ | (Position-) $R^4$ | $R^5$ | $R^6$ |
|---|---|---|---|
| Cl | (2-) $SO_2CH_3$ | $C_3H_7$-i | 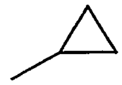 |
| Cl | (2-) $SO_2CH_3$ | $C_4H_9$ | 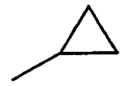 |
| Cl | (2-) $SO_2CH_3$ | $C_4H_9$-1 | 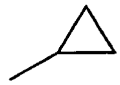 |
| Cl | (2-) $SO_2CH_3$ | $C_4H_9$-s | 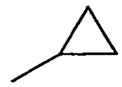 |
| Cl | (2-) $SO_2CH_3$ | $C_4H_9$-t | 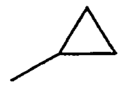 |
| Cl | (2-) $SO_2CH_3$ | 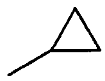 | 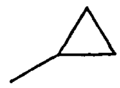 |
| Cl | (2-)$SO_2CH_3$ | 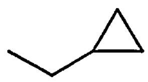 | 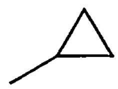 |
| Cl | (2-) $SO_2CH_3$ | $CH=CHCH_3$ | 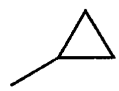 |
| Cl | (2-) $SO_2CH_3$ | 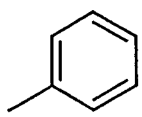 | 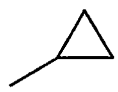 |
| Cl | (2-) $SO_2CH_3$ | 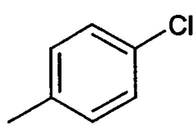 | 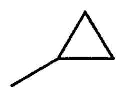 |
| Cl | (2-) $SO_2CH_3$ | 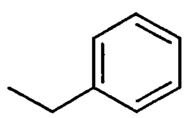 | 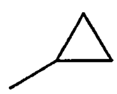 |
| Cl | (2-) $SO_2CH_3$ | $N(CH_3)_2$ | 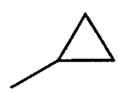 |
| Cl | (2-) $SO_2CH_3$ | 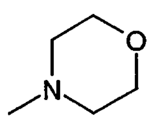 | 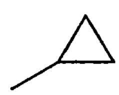 |

(fortgesetzt)

| $R^3$ | (Position-) $R^4$ | $R^5$ | $R^6$ |
|---|---|---|---|
| Cl | (2-) $SO_2CH_3$ | Cl | 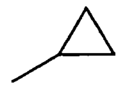 |
| Cl | (2-) $SO_2CH_3$ | Br | 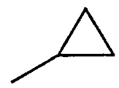 |
| Cl | (2-) Cl | $CF_3$ | $N(CH_3)_2$ |
| Cl | (2-) Cl | $SCH_3$ | $N(CH_3)_2$ |
| Cl | (2-) Cl | $SC_2H_5$ | $N(CH_3)_2$ |
| Cl | (2-) Cl | $SC_3H_7$ | $N(CH_3)_2$ |
| Cl | (2-) Cl | $SC_3H_7$-i | $N(CH_3)_2$ |
| Cl | (2-) Cl | 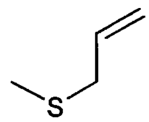 | $N(CH_3)_2$ |
| Cl | (2-) Cl | 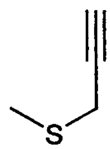 | $N(CH_3)_2$ |
| Cl | (2-) Cl | 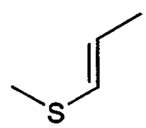 | $N(CH_3)_2$ |
| Cl | (2-) Cl | 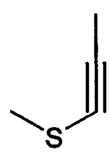 | $N(CH_3)_2$ |
| Cl | (2-) Cl | 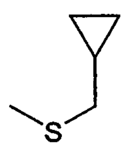 | $N(CH_3)_2$ |
| Cl | (2-) Cl | $SCH{=}C{=}CH_2$ | $N(CH_3)_2$ |
| Cl | (2-) Cl | $SCH_2CN$ | $N(CH_3)_2$ |
| Cl | (2-) Cl | $SCH_2CH_2CN$ | $N(CH_3)_2$ |
| Cl | (2-) Cl | $OCH_3$ | $N(CH_3)_2$ |
| Cl | (2-) Cl | $OC_2H_5$ | $N(CH_3)_2$ |
| Cl | (2-) Cl | $OC_3H_7$ | $N(CH_3)_2$ |
| Cl | (2-) Cl | $OC_3H_7$-i | $N(CH_3)_2$ |
| Cl | (2-) Cl | $OC_4H_9$ | $N(CH_3)_2$ |
| Cl | (2-) Cl | $OCH_2CF_3$ | $N(CH_3)_2$ |

(fortgesetzt)

| $R^3$ | (Position-) $R^4$ | $R^5$ | $R^6$ |
|---|---|---|---|
| Cl | (2-) Cl | 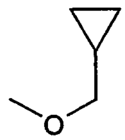 | $N(CH_3)_2$ |
| Cl | (2-) Cl | $OC_6H_5$ | $N(CH_3)_2$ |
| Cl | (2-) Cl | H | $N(CH_3)_2$ |
| Cl | (2-) Cl | $CH_3$ | $N(CH_3)_2$ |
| Cl | (2-) Cl | $C_2H_5$ | $N(CH_3)_2$ |
| Cl | (2-) Cl | $C_3H_7$ | $N(CH_3)_2$ |
| Cl | (2-) Cl | $C_3H_7$-i | $N(CH_3)_2$ |
| Cl | (2-) Cl | $C_4H_9$ | $N(CH_3)_2$ |
| Cl | (2-) Cl | $C_4H_9$-i | $N(CH_3)_2$ |
| Cl | (2-) Cl | $C_4H_9$-s | $N(CH_3)_2$ |
| Cl | (2-) Cl | $C_4H_9$-t | $N(CH_3)_2$ |
| Cl | (2-) Cl | 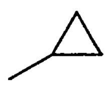 | $N(CH_3)_2$ |
| Cl | (2-) Cl | 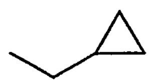 | $N(CH_3)_2$ |
| Cl | (2-) Cl | $CH=CHCH_3$ | $N(CH_3)_2$ |
| Cl | (2-) Cl | 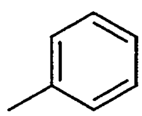 | $N(CH_3)_2$ |
| Cl | (2-) Cl | 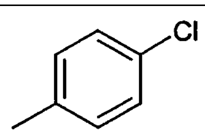 | $N(CH_3)_2$ |
| Cl | (2-) Cl | 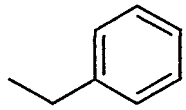 | $N(CH_3)_2$ |
| Cl | (2-) Cl | $N(CH_3)_2$ | $N(CH_3)_2$ |
| Cl | (2-) Cl | 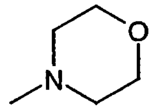 | $N(CH_3)_2$ |
| Cl | (2-) Cl | Cl | $N(CH_3)_2$ |
| Cl | (2-) Cl | Br | $N(CH_3)_2$ |
| $SO_2CH_3$ | (2-) Cl | $CF_3$ | $N(CH_3)_2$ |
| $SO_2CH_3$ | (2-) Cl | $SCH_3$ | $N(CH_3)_2$ |

(fortgesetzt)

| $R^3$ | (Position-) $R^4$ | $R^5$ | $R^6$ |
|---|---|---|---|
| $SO_2CH_3$ | (2-) Cl | $SC_2H_5$ | $N(CH_3)_2$ |
| $SO_2CH_3$ | (2-) Cl | $SC_3H_7$ | $N(CH_3)_2$ |
| $SO_2CH_3$ | (2-) Cl | $SC_3H_7$-i | $N(CH_3)_2$ |
| $SO_2CH_3$ | (2-) Cl | 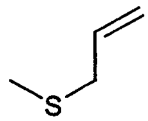 | $N(CH_3)_2$ |
| $SO_2CH_3$ | (2-) Cl | 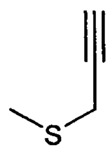 | $N(CH_3)_2$ |
| $SO_2CH_3$ | (2-) Cl | 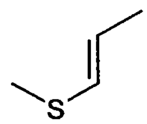 | $N(CH_3)_2$ |
| $SO_2CH_3$ | (2-) Cl | 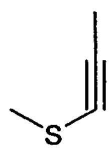 | $N(CH_3)_2$ |
| $SO_2CH_3$ | (2-) Cl | 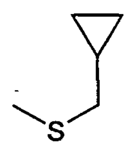 | $N(CH_3)_2$ |
| $SO_2CH_3$ | (2-) Cl | $SCH{=}C{=}CH_2$ | $N(CH_3)_2$ |
| $SO_2CH_3$ | (2-) Cl | $SCH_2CN$ | $N(CH_3)_2$ |
| $SO_2CH_3$ | (2-) Cl | $SCH_2CH_2CN$ | $N(CH_3)_2$ |
| $SO_2CH_3$ | (2-) Cl | $OCH_3$ | $N(CH_3)_2$ |
| $SO_2CH_3$ | (2-) Cl | $OC_2H_5$ | $N(CH_3)_2$ |
| $SO_2CH_3$ | (2-) Cl | $OC_3H_7$ | $N(CH_3)_2$ |
| $SO_2CH_3$ | (2-) Cl | $OC_3H_7$-i | $N(CH_3)_2$ |
| $SO_2CH_3$ | (2-) Cl | $OC_4H_9$ | $N(CH_3)_2$ |
| $SO_2CH_3$ | (2-) Cl | $OCH_2CF_3$ | $N(CH_3)_2$ |
| $SO_2CH_3$ | (2-) Cl | 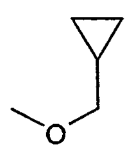 | $N(CH_3)_2$ |
| $SO_2CH_3$ | (2-) Cl | $OC_6H_5$ | $N(CH_3)_2$ |
| $SO_2CH_3$ | (2-) Cl | H | $N(CH_3)_2$ |
| $SO_2CH_3$ | (2-) Cl | $CH_3$ | $N(CH_3)_2$ |
| $SO_2CH_3$ | (2-) Cl | $C_2H_5$ | $N(CH_3)_2$ |

(fortgesetzt)

| $R^3$ | (Position-) $R^4$ | $R^5$ | $R^6$ |
|---|---|---|---|
| $SO_2CH_3$ | (2-) Cl | $C_3H_7$ | $N(CH_3)_2$ |
| $SO_2CH_3$ | (2-) Cl | $C_3H_7$-i | $N(CH_3)_2$ |
| $SO_2CH_3$ | (2-) Cl | $C_4H_9$ | $N(CH_3)_2$ |
| $SO_2CH_3$ | (2-) Cl | $C_4H_9$-i | $N(CH_3)_2$ |
| $SO_2CH_3$ | (2-) Cl | $C_4H_9$-s | $N(CH_3)_2$ |
| $SO_2CH_3$ | (2-) Cl | $C_4H_9$-t | $N(CH_3)_2$ |
| $SO_2CH_3$ | (2-) Cl | 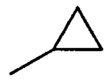 | $N(CH_3)_2$ |
| $SO_2CH_3$ | (2-) Cl | 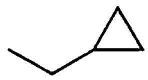 | $N(CH_3)_2$ |
| $SO_2CH_3$ | (2-) Cl | $CH{=}CHCH_3$ | $N(CH_3)_2$ |
| $SO_2CH_3$ | (2-) Cl | 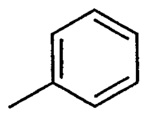 | $N(CH_3)_2$ |
| $SO_2CH_3$ | (2-) Cl | 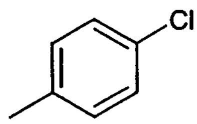 | $N(CH_3)_2$ |
| $SO_2CH_3$ | (2-) Cl | 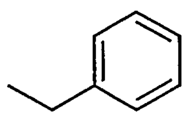 | $N(CH_3)_2$ |
| $SO_2CH_3$ | (2-) Cl | $N(CH_3)_2$ | $N(CH_3)_2$ |
| $SO_2CH_3$ | (2-) Cl | 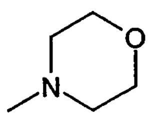 | $N(CH_3)_2$ |
| $SO_2CH_3$ | (2-) Cl | Cl | $N(CH_3)_2$ |
| $SO_2CH_3$ | (2-) Cl | Br | $N(CH_3)_2$ |
| Cl | (2-) $SO_2CH_3$ | $CF_3$ | $N(CH_3)_2$ |
| Cl | (2-) $SO_2CH_3$ | $SCH_3$ | $N(CH_3)_2$ |
| Cl | (2-) $SO_2CH_3$ | $SC_2H_5$ | $N(CH_3)_2$ |
| Cl | (2-) $SO_2CH_3$ | $SC_3H_7$ | $N(CH_3)_2$ |
| Cl | (2-) $SO_2CH_3$ | $SC_3H_7$-i | $N(CH_3)_2$ |
| Cl | (2-) $SO_2CH_3$ | 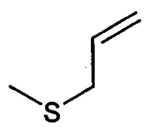 | $N(CH_3)_2$ |

(fortgesetzt)

| $R^3$ | (Position-) $R^4$ | $R^5$ | $R^6$ |
|---|---|---|---|
| Cl | (2-) $SO_2CH_3$ | 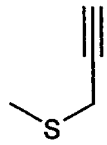 | $N(CH_3)_2$ |
| Cl | (2-) $SO_2CH_3$ | 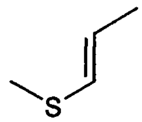 | $N(CH_3)_2$ |
| Cl | (2-) $SO_2CH_3$ | 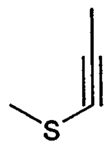 | $N(CH_3)_2$ |
| Cl | (2-) $SO_2CH_3$ | 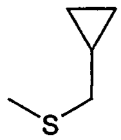 | $N(CH_3)_2$ |
| Cl | (2-) $SO_2CH_3$ | $SCH=C=CH_2$ | $N(CH_3)_2$ |
| Cl | (2-) $SO_2CH_3$ | $SCH_2CN$ | $N(CH_3)_2$ |
| Cl | (2-) $SO_2CH_3$ | $SCH_2CH_2CN$ | $N(CH_3)_2$ |
| Cl | (2-) $SO_2CH_3$ | $OCH_3$ | $N(CH_3)_2$ |
| Cl | (2-) $SO_2CH_3$ | $OC_2H_5$ | $N(CH_3)_2$ |
| Cl | (2-) $SO_2CH_3$ | $OC_3H_7$ | $N(CH_3)_2$ |
| Cl | (2-) $SO_2CH_3$ | $OC_3H_7$-i | $N(CH_3)_2$ |
| Cl | (2-) $SO_2CH_3$ | $OC_4H_9$ | $N(CH_3)_2$ |
| Cl | (2-) $SO_2CH_3$ | $OCH_2CF_3$ | $N(CH_3)_2$ |
| Cl | (2-) $SO_2CH_3$ | 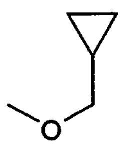 | $N(CH_3)_2$ |
| Cl | (2-) $SO_2CH_3$ | $OC_6H_5$ | $N(CH_3)_2$ |
| Cl | (2-) $SO_2CH_3$ | H | $N(CH_3)_2$ |
| Cl | (2-) $SO_2CH_3$ | $CH_3$ | $N(CH_3)_2$ |
| Cl | (2-) $SO_2CH_3$ | $C_2H_5$ | $N(CH_3)_2$ |
| Cl | (2-) $SO_2CH_3$ | $C_3H_7$ | $N(CH_3)_2$ |
| Cl | (2-) $SO_2CH_3$ | $C_3H_7$-i | $N(CH_3)_2$ |
| Cl | (2-) $SO_2CH_3$ | $C_4H_9$ | $N(CH_3)_2$ |
| Cl | (2-) $SO_2CH_3$ | $C_4H_9$-i | $N(CH_3)_2$ |
| Cl | (2-) $SO_2CH_3$ | $C_4H_9$-s | $N(CH_3)_2$ |
| Cl | (2-) $SO_2CH_3$ | $C_4H_9$-t | $N(CH_3)_2$ |

(fortgesetzt)

| $R^3$ | (Position-) $R^4$ | $R^5$ | $R^6$ |
|---|---|---|---|
| Cl | (2-) $SO_2CH_3$ | 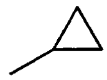 | $N(CH_3)_2$ |
| Cl | (2-) $SO_2CH_3$ | 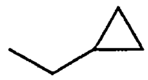 | $N(CH_3)_2$ |
| Cl | (2-) $SO_2CH_3$ | $CH=CHCH_3$ | $N(CH_3)_2$ |
| Cl | (2-) $SO_2CH_3$ | 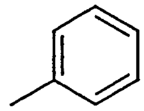 | $N(CH_3)_2$ |
| Cl | (2-) $SO_2CH_3$ | 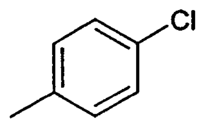 | $N(CH_3)_2$ |
| Cl | (2-) $SO_2CH_3$ | 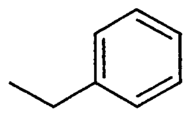 | $N(CH_3)_2$ |
| Cl | (2-) $SO_2CH_3$ | $N(CH_3)_2$ | $N(CH_3)_2$ |
| Cl | (2-) $SO_2CH_3$ | 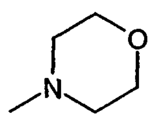 | $N(CH_3)_2$ |
| Cl | (2-) $SO_2CH_3$ | Cl | $N(CH_3)_2$ |
| Cl | (2-) $SO_2CH_3$ | Br | $N(CH_3)_2$ |
| Cl | (2-) Cl | $CH_3$ | $OCH_3$ |
| Cl | (2-) Cl | $C_2H_5$ | $OCH_3$ |
| Cl | (2-) Cl | $C_3H_7$ | $OCH_3$ |
| Cl | (2-) Cl | $SCH_3$ | $OCH_3$ |
| Cl | (2-) Cl | $SC_2H_5$ | $OCH_3$ |
| Cl | (2-) Cl | $OCH_3$ | $OCH_3$ |
| Cl | (2-) Cl | $OC_2H_5$ | $OCH_3$ |
| Cl | (2-) Cl | $CH_3$ | $OC_2H_5$ |
| Cl | (2-) Cl | $C_2H_5$ | $OC_2H_5$ |
| Cl | (2-) Cl | $C_3H_7$ | $OC_2H_5$ |
| Cl | (2-) Cl | $SCH_3$ | $OC_2H_5$ |
| Cl | (2-) Cl | $SC_2H_5$ | $OC_2H_5$ |
| Cl | (2-) Cl | $OCH_3$ | $OC_2H_5$ |
| Cl | (2-) Cl | $OC_2H_5$ | $OC_2H_5$ |
| Cl | (2-) $SO_2CH_3$ | $CH_3$ | $OCH_3$ |

(fortgesetzt)

| $R^3$ | (Position-) $R^4$ | $R^5$ | $R^6$ |
|---|---|---|---|
| Cl | (2-) $SO_2CH_3$ | $C_2H_5$ | $OCH_3$ |
| Cl | (2-) $SO_2CH_3$ | $C_3H_7$ | $OCH_3$ |
| Cl | (2-) $SO_2CH_3$ | $SCH_3$ | $OCH_3$ |
| Cl | (2-) $SO_2CH_3$ | $SC_2H_5$ | $OCH_3$ |
| Cl | (2-) $SO_2CH_3$ | $OCH_3$ | $OCH_3$ |
| Cl | (2-) $SO_2CH_3$ | $OC_2H_5$ | $OCH_3$ |
| Cl | (2-) $SO_2CH_3$ | $CH_3$ | $OC_2H_5$ |
| Cl | (2-) $SO_2CH_3$ | $C_2H_5$ | $OC_2H_5$ |
| Cl | (2-) $SO_2CH_3$ | $C_3H_7$ | $OC_2H_5$ |
| Cl | (2-) $SO_2CH_3$ | $SCH_3$ | $OC_2H_5$ |
| Cl | (2-) $SO_2CH_3$ | $SC_2H_5$ | $OC_2H_5$ |
| Cl | (2-) $SO_2CH_3$ | $OCH_3$ | $OC_2H_5$ |
| Cl | (2-) $SO_2CH_3$ | $OC_2H_5$ | $OC_2H_5$ |
| $SO_2CH_3$ | (2-) Cl | Cl | $OCH_3$ |
| $SO_2CH_3$ | (2-) Cl | Br | $OCH_3$ |
| $SO_2CH_3$ | (2-) Cl | $CH_3$ | $OCH_3$ |
| $SO_2CH_3$ | (2-) Cl | $C_2H_5$ | $OCH_3$ |
| $SO_2CH_3$ | (2-) Cl | $C_3H_7$ | $OCH_3$ |
| $SO_2CH_3$ | (2-) Cl | $SCH_3$ | $OCH_3$ |
| $SO_2CH_3$ | (2-) Cl | $SC_2H_5$ | $OCH_3$ |
| $SO_2CH_3$ | (2-) Cl | $OCH_3$ | $OC_2H_5$ |
| $SO_2CH_3$ | (2-) Cl | $OC_2H_5$ | $OC_2H_5$ |
| $SO_2CH_3$ | (2-) Cl | $CH_3$ | $OC_2H_5$ |
| $SO_2CH_3$ | (2-) Cl | $C_2H_5$ | $OC_2H_5$ |
| $SO_2CH_3$ | (2-) Cl | $C_3H_7$ | $OC_2H_5$ |
| $SO_2CH_3$ | (2-) Cl | $SCH_3$ | $OC_2H_5$ |
| $SO_2CH_3$ | (2-) Cl | $SC_2H_5$ | $OC_2H_5$ |
| $SO_2CH_3$ | (2-) Cl | $OCH_3$ | $OC_2H_5$ |
| $CF_3$ | (2-) Cl | Br | $CH_3$ |
| $CF_3$ | (2-) Cl | $SCH_3$ | $CH_3$ |
| $CF_3$ | (2-) Cl | $OCH_3$ | $CH_3$ |
| $CF_3$ | (2-) Cl | $N(CH_3)_2$ | $CH_3$ |
| $CF_3$ | (2-) Cl | $CF_3$ | $CH_3$ |
| $CF_3$ | (2-)$NO_2$ | Br | $CH_3$ |
| $CF_3$ | (2-)$NO_2$ | $SCH_3$ | $CH_3$ |
| $CF_3$ | (2-)$NO_2$ | $OCH_3$ | $CH_3$ |
| $CF_3$ | (2-)$NO_2$ | $N(CH_3)_2$ | $CH_3$ |
| $CF_3$ | (2-) $NO_2$ | $CF_3$ | $CH_3$ |

(fortgesetzt)

| $R^3$ | (Position-) $R^4$ | $R^5$ | $R^6$ |
|---|---|---|---|
| $CF_3$ | (2-) $CH_3$ | Br | $CH_3$ |
| $CF_3$ | (2-) $CH_3$ | $SCH_3$ | $CH_3$ |
| $CF_3$ | (2-) $CH_3$ | $OCH_3$ | $CH_3$ |
| $CF_3$ | (2-) $CH_3$ | $N(CH_3)_2$ | $CH_3$ |
| $CF_3$ | (2-) $CH_3$ | $CF_3$ | $CH_3$ |
| $CF_3$ | (2-) $OCH_3$ | Br | $CH_3$ |
| $CF_3$ | (2-) $OCH_3$ | $SCH_3$ | $CH_3$ |
| $CF_3$ | (2-) $OCH_3$ | $OCH_3$ | $CH_3$ |
| $CF_3$ | (2-) $OCH_3$ | $N(CH_3)_2$ | $CH_3$ |
| $CF_3$ | (2-) $OCH_3$ | $CF_3$ | $CH_3$ |
| $SO_2CH_3$ | (2-) $NO_2$ | Br | $CH_3$ |
| $SO_2CH_3$ | (2-) NOz | $SCH_3$ | $CH_3$ |
| $SO_2CH_3$ | (2-) NOz | $OCH_3$ | $CH_3$ |
| $SO_2CH_3$ | (2-) $NO_2$ | $N(CH_3)_2$ | $CH_3$ |
| $SO_2CH_3$ | (2-) $NO_2$ | $CF_3$ | $CH_3$ |
| $SO_2CH_3$ | (2-) $CF_3$ | Br | $CH_3$ |
| $SO_2CH_3$ | (2-) $CF_3$ | $SCH_3$ | $CH_3$ |
| $SO_2CH_3$ | (2-) $CF_3$ | $OCH_3$ | $CH_3$ |
| $SO_2CH_3$ | (2-) $CF_3$ | $N(CH_3)_2$ | $CH_3$ |
| $SO_2CH_3$ | (2-) $CF_3$ | $CF_3$ | $CH_3$ |
| $SO_2CH_3$ | (2-) $SO_2CH_3$ | Br | $CH_3$ |
| $SO_2CH_3$ | (2-) $SO_2CH_3$ | $SCH_3$ | $CH_3$ |
| $SO_2CH_3$ | (2-) $SO_2CH_3$ | $OCH_3$ | $CH_3$ |
| $SO_2CH_3$ | (2-) $SO_2CH_3$ | $N(CH_3)_2$ | $CH_3$ |
| $SO_2CH_3$ | (2-) $SO_2CH_3$ | $CF_3$ | $CH_3$ |
| CN | (2-) Cl | Br | $CH_3$ |
| CN | (2-) Cl | $SCH_3$ | $CH_3$ |
| CN | (2-) Cl | $OCH_3$ | $CH_3$ |
| CN | (2-) Cl | $N(CH_3)_2$ | $CH_3$ |
| CN | (2-) Cl | $CF_3$ | $CH_3$ |
| CN | (2-) $NO_2$ | Br | $CH_3$ |
| CN | (2-) $NO_2$ | $SCH_3$ | $CH_3$ |
| CN | (2-) $NO_2$ | $OCH_3$ | $CH_3$ |
| CN | (2-) $NO_2$ | $N(CH_3)_2$ | $CH_3$ |
| CN | (2-) $NO_2$ | $CF_3$ | $CH_3$ |
| CN | (2-) $CF_3$ | Br | $CH_3$ |
| CN | (2-) $CF_3$ | $SCH_3$ | $CH_3$ |
| CN | (2-) $CF_3$ | $OCH_3$ | $CH_3$ |

(fortgesetzt)

| $R^3$ | (Position-) $R^4$ | $R^5$ | $R^6$ |
|---|---|---|---|
| CN | (2-) $CF_3$ | $N(CH_3)_2$ | $CH_3$ |
| CN | (2-) $CF_3$ | $CF_3$ | $CH_3$ |
| CN | (2-) $SO_2CH_3$ | Br | $CH_3$ |
| CN | (2-) $SO_2CH_3$ | $SCH_3$ | $CH_3$ |
| CN | (2-) $SO_2CH_3$ | $OCH_3$ | $CH_3$ |
| CN | (2-) $SO_2CH_3$ | $N(CH_3)_2$ | $CH_3$ |
| CN | (2-) $SO_2CH_3$ | $CF_3$ | $CH_3$ |
| Br | (2-) $NO_2$ | Br | $CH_3$ |
| Br | (2-) $NO_2$ | $SCH_3$ | $CH_3$ |
| Br | (2-) $NO_2$ | $OCH_3$ | $CH_3$ |
| Br | (2-) $NO_2$ | $N(CH_3)_2$ | $CH_3$ |
| Br | (2-) $NO_2$ | $CF_3$ | $CH_3$ |
| Br | (2-) $CF_3$ | Br | $CH_3$ |
| Br | (2-) $CF_3$ | $SCH_3$ | $CH_3$ |
| Br | (2-) $CF_3$ | $OCH_3$ | $CH_3$ |
| Br | (2-) $CF_3$ | $N(CH_3)_2$ | $CH_3$ |
| Br | (2-) $CF_3$ | $CF_3$ | $CH_3$ |
| Br | (2-) $SO_2CH_3$ | Br | $CH_3$ |
| Br | (2-) $SO_2CH_3$ | $SCH_3$ | $CH_3$ |
| Br | (2-) $SO_2CH_3$ | $OCH_3$ | $CH_3$ |
| Br | (2-) $SO_2CH_3$ | $N(CH_3)_2$ | $CH_3$ |
| Br | (2-) $SO_2CH_3$ | $CF_3$ | $CH_3$ |
| Br | (2-) $CH_3$ | Br | $CH_3$ |
| Br | (2-) $CH_3$ | $SCH_3$ | $CH_3$ |
| Br | (2-) $CH_3$ | $OCH_3$ | $CH_3$ |
| Br | (2-) $CH_3$ | $N(CH_3)_2$ | $CH_3$ |
| Br | (2-) $CH_3$ | $CF_3$ | $CH_3$ |
| Cl | (2-) $OCH_3$ | $CF_3$ | $CH_3$ |
| Cl | (2-) $OCH_3$ | $SCH_3$ | $CH_3$ |
| Cl | (2-) $OCH_3$ | $SC_2H_5$ | $CH_3$ |
| Cl | (2-) $OCH_3$ | $SC_3H_7$ | $CH_3$ |
| Cl | (2-) $OCH_3$ | $SC_3H_7$-i | $CH_3$ |
| Cl | (2-) $OCH_3$ | 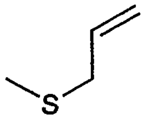 | $CH_3$ |

(fortgesetzt)

| $R^3$ | (Position-) $R^4$ | $R^5$ | $R^6$ |
|---|---|---|---|
| Cl | (2-) $OCH_3$ | 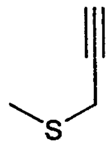 | $CH_3$ |
| Cl | (2-) $OCH_3$ | 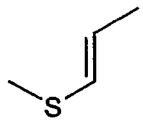 | $CH_3$ |
| Cl | (2-) $OCH_3$ | 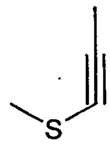 | $CH_3$ |
| Cl | (2-) $OCH_3$ | 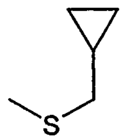 | $CH_3$ |
| Cl | (2-) $OCH_3$ | $SCH=C=CH_2$ | $CH_3$ |
| Cl | (2-) $OCH_3$ | $SCH_2CN$ | $CH_3$ |
| Cl | (2-) $OCH_3$ | $SCH_2CH_2CN$ | $CH_3$ |
| Cl | (2-) $OCH_3$ | $OCH_3$ | $CH_3$ |
| Cl | (2-) $OCH_3$ | $OC_2H_5$ | $CH_3$ |
| Cl | (2-) $OCH_3$ | $OC_3H_7$ | $CH_3$ |
| Cl | (2-) $OCH_3$ | $OC_3H_7$-i | $CH_3$ |
| Cl | (2-) $OCH_3$ | $OC_4H_9$ | $CH_3$ |
| Cl | (2-) $OCH_3$ | $OCH_2CF_3$ | $CH_3$ |
| Cl | (2-) $OCH_3$ | 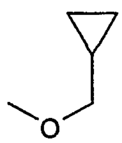 | $CH_3$ |
| Cl | (2-) $OCH_3$ | $OC_6H_5$ | $CH_3$ |
| Cl | (2-) $OCH_3$ | H | $CH_3$ |
| Cl | (2-) $OCH_3$ | $CH_3$ | $CH_3$ |
| Cl | (2-) $OCH_3$ | $C_2H_5$ | $CH_3$ |
| Cl | (2-) $OCH_3$ | $C_3H_7$ | $CH_3$ |
| Cl | (2-) $OCH_3$ | $C_3H_7$-i | $CH_3$ |
| Cl | (2-) $OCH_3$ | $C_4H_9$ | $CH_3$ |
| Cl | (2-) $OCH_3$ | $C_4H_9$-i | $CH_3$ |
| Cl | (2-) $OCH_3$ | $C_4H_9$-s | $CH_3$ |
| Cl | (2-) $OCH_3$ | $C_4H_9$-t | $CH_3$ |

(fortgesetzt)

| $R^3$ | (Position-) $R^4$ | $R^5$ | $R^6$ |
|---|---|---|---|
| Cl | (2-) $OCH_3$ | 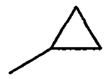 | $CH_3$ |
| Cl | (2-) $OCH_3$ | 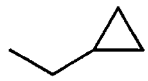 | $CH_3$ |
| Cl | (2-) $OCH_3$ | $CH{=}CHCH_3$ | $CH_3$ |
| Cl | (2-) $OCH_3$ | 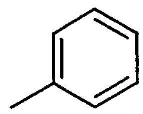 | $CH_3$ |
| Cl | (2-) $OCH_3$ | 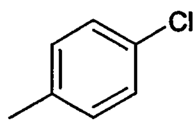 | $CH_3$ |
| Cl | (2-) $OCH_3$ | 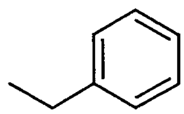 | $CH_3$ |
| Cl | (2-) $OCH_3$ | $N(CH_3)_2$ | $CH_3$ |
| Cl | (2-) $OCH_3$ | 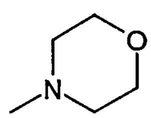 | $CH_3$ |
| Cl | (2-) $OCH_3$ | Cl | $CH_3$ |
| Cl | (2-) $OCH_3$ | Br | $CH_3$ |
| $SO_2CH_3$ | (2-) $OCH_3$ | $CF_3$ | $CH_3$ |
| $SO_2CH_3$ | (2-) $OCH_3$ | $SCH_3$ | $CH_3$ |
| $SO_2CH_3$ | (2-) $OCH_3$ | $SC_2H_5$ | $CH_3$ |
| $SO_2CH_3$ | (2-) $OCH_3$ | $SC_3H_7$ | $CH_3$ |
| $SO_2CH_3$ | (2-) $OCH_3$ | $SC_3H_7$-i | $CH_3$ |
| $SO_2CH_3$ | (2-) $OCH_3$ | 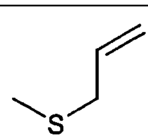 | $CH_3$ |
| $SO_2CH_3$ | (2-) $OCH_3$ | 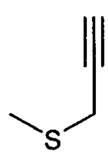 | $CH_3$ |
| $SO_2CH_3$ | (2-) $OCH_3$ | 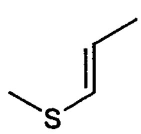 | $CH_3$ |

(fortgesetzt)

| $R^3$ | (Position-) $R^4$ | $R^5$ | $R^6$ |
|---|---|---|---|
| $SO_2CH_3$ | (2-) $OCH_3$ | 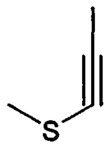 | $CH_3$ |
| $SO_2CH_3$ | (2-) $OCH_3$ | 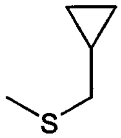 | $CH_3$ |
| $SO_2CH_3$ | (2-) $OCH_3$ | $SCH=C=CH_2$ | $CH_3$ |
| $SO_2CH_3$ | (2-) $OCH_3$ | $SCH_2CN$ | $CH_3$ |
| $SO_2CH_3$ | (2-) $OCH_3$ | $SCH_2CH_2CN$ | $CH_3$ |
| $SO_2CH_3$ | (2-) $OCH_3$ | $OCH_3$ | $CH_3$ |
| $SO_2CH_3$ | (2-) $OCH_3$ | $OC_2H_5$ | $CH_3$ |
| $SO_2CH_3$ | (2-) $OCH_3$ | $OC_3H_7$ | $CH_3$ |
| $SO_2CH_3$ | (2-) $OCH_3$ | $OC_3H_7$-i | $CH_3$ |
| $SO_2CH_3$ | (2-) $OCH_3$ | $OC_4H_9$ | $CH_3$ |
| $SO_2CH_3$ | (2-) $OCH_3$ | $OCH_2CF_3$ | $CH_3$ |
| $SO_2CH_3$ | (2-) $OCH_3$ | 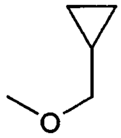 | $CH_3$ |
| $SO_2CH_3$ | (2-) $OCH_3$ | $OC_6H_5$ | $CH_3$ |
| $SO_2CH_3$ | (2-) $OCH_3$ | H | $CH_3$ |
| $SO_2CH_3$ | (2-) $OCH_3$ | $CH_3$ | $CH_3$ |
| $SO_2CH_3$ | (2-) $OCH_3$ | $C_2H_5$ | $CH_3$ |
| $SO_2CH_3$ | (2-) $OCH_3$ | $C_3H_7$ | $CH_3$ |
| $SO_2CH_3$ | (2-) $OCH_3$ | $C_3H_7$-i | $CH_3$ |
| $SO_2CH_3$ | (2-) $OCH_3$ | $C_4H_9$ | $CH_3$ |
| $SO_2CH_3$ | (2-) $OCH_3$ | $C_4H_9$-i | $CH_3$ |
| $SO_2CH_3$ | (2-) $OCH_3$ | $C_4H_9$-s | $CH_3$ |
| $SO_2CH_3$ | (2-) $OCH_3$ | $C_4H_9$-t | $CH_3$ |
| $SO_2CH_3$ | (2-) $OCH_3$ | 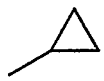 | $CH_3$ |
| $SO_2CH_3$ | (2-) $OCH_3$ | 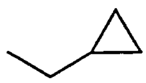 | $CH_3$ |
| $SO_2CH_3$ | (2-) $OCH_3$ | $CH=CHCH_3$ | $CH_3$ |

(fortgesetzt)

| $R^3$ | (Position-) $R^4$ | $R^5$ | $R^6$ |
|---|---|---|---|
| $SO_2CH_3$ | (2-) $OCH_3$ | 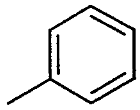 | $CH_3$ |
| $SO_2CH_3$ | (2-) $OCH_3$ | 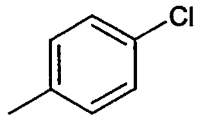 | $CH_3$ |
| $SO_2CH_3$ | (2-) $OCH_3$ | 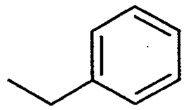 | $CH_3$ |
| $SO_2CH_3$ | (2-) $OCH_3$ | | $CH_3$ |
| $SO_2CH_3$ | (2-) $OCH_3$ | 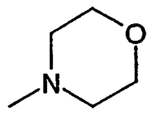 | $CH_3$ |
| $SO_2CH_3$ | (2-) $OCH_3$ | Cl | $CH_3$ |
| $SO_2CH_3$ | (2-) $OCH_3$ | Br | $CH_3$ |
| Cl | (2-) $OCH_3$ | $CF_3$ | 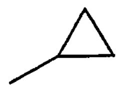 |
| Cl | (2-) $OCH_3$ | $SCH_3$ | 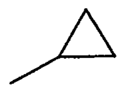 |
| Cl | (2-) $OCH_3$ | $SC_2H_5$ | 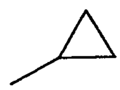 |
| Cl | (2-) $OCH_3$ | $SC_3H_7$ | 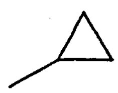 |
| Cl | (2-) $OCH_3$ | $SC_3H_7$-i | 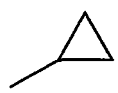 |
| Cl | (2-) $OCH_3$ | 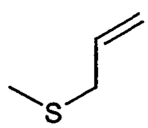 | 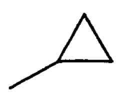 |
| Cl | (2-) $OCH_3$ | 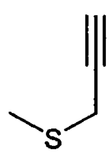 | 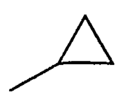 |

(fortgesetzt)

| $R^3$ | (Position-) $R^4$ | $R^5$ | $R^6$ |
|---|---|---|---|
| Cl | (2-) $OCH_3$ | 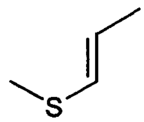 | 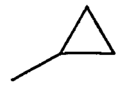 |
| Cl | (2-) $OCH_3$ | 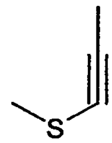 | 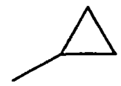 |
| Cl | (2-) $OCH_3$ | 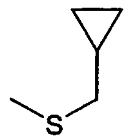 | 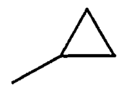 |
| Cl | (2-) $OCH_3$ | $SCH=C=CH_2$ | 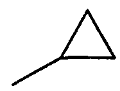 |
| Cl | (2-) $OCH_3$ | $SCH_2CN$ | 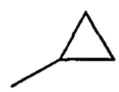 |
| Cl | (2-) $OCH_3$ | $SCH_2CH_2CN$ | 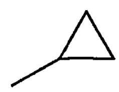 |
| Cl | (2-) $OCH_3$ | $OCH_3$ | 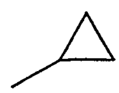 |
| Cl | (2-) $OCH_3$ | $OC_2H_5$ | 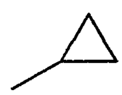 |
| Cl | (2-) $OCH_3$ | $OC_3H_7$ | 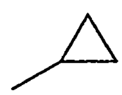 |
| Cl | (2-) $OCH_3$ | $OC_3H_7$-i | 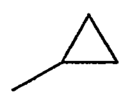 |
| Cl | (2-) $OCH_3$ | $OC_4H_9$ | 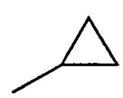 |
| Cl | (2-) $OCH_3$ | $OCH_2CF_3$ | 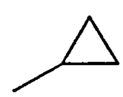 |

(fortgesetzt)

| $R^3$ | (Position-) $R^4$ | $R^5$ | $R^6$ |
|---|---|---|---|
| Cl | (2-) $OCH_3$ | 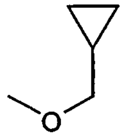 | 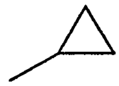 |
| Cl | (2-) $OCH_3$ | $OC_6H_5$ | 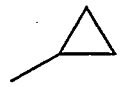 |
| Cl | (2-) $OCH_3$ | H | 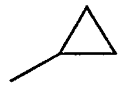 |
| Cl | (2-) $OCH_3$ | $CH_3$ | 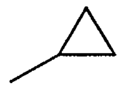 |
| Cl | (2-) $OCH_3$ | $C_2H_5$ | 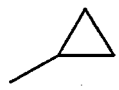 |
| Cl | (2-) $OCH_3$ | $C_3H_7$ | 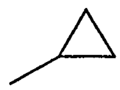 |
| Cl | (2-) $OCH_3$ | $C_3H_7$-i | 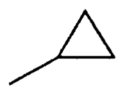 |
| Cl | (2-) $OCH_3$ | $C_4H_9$ | 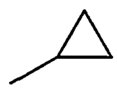 |
| Cl | (2-) $OCH_3$ | $C_4H_9$-i | 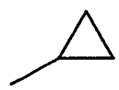 |
| Cl | (2-) $OCH_3$ | $C_4H_9$-s | 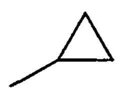 |
| Cl | (2-) $OCH_3$ | $C_4H_9$-t | 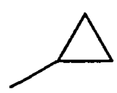 |
| Cl | (2-) $OCH_3$ | 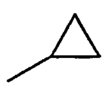 | 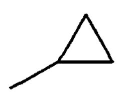 |
| Cl | (2-) $OCH_3$ | 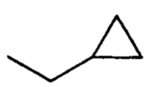 | 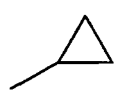 |
| Cl | (2-) $OCH_3$ | CH=CHCH$_3$ | 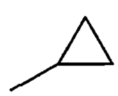 |

(fortgesetzt)

| $R^3$ | (Position-) $R^4$ | $R^5$ | $R^6$ |
|---|---|---|---|
| Cl | (2-) $OCH_3$ | 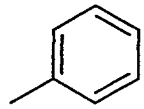 | 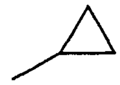 |
| Cl | (2-) $OCH_3$ | 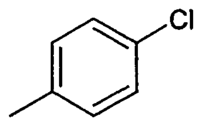 | 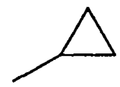 |
| Cl | (2-) $OCH_3$ | 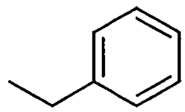 | 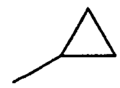 |
| Cl | (2-) $OCH_3$ | $N(CH_3)_2$ | 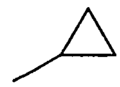 |
| Cl | (2-) $OCH_3$ | 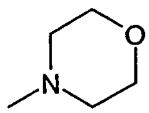 | 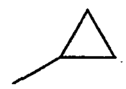 |
| Cl | (2-) $OCH_3$ | Cl | 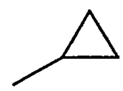 |
| Cl | (2-) $OCH_3$ | Br | 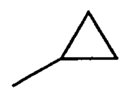 |
| $SO_2CH_3$ | (2-) $OCH_3$ | $CF_3$ | 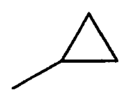 |
| $SO_2CH_3$ | (2-) $OCH_3$ | $SCH_3$ | 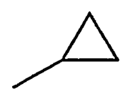 |
| $SO_2CH_3$ | (2-) $OCH_3$ | $SC_2H_5$ | 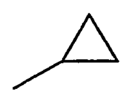 |
| $SO_2CH_3$ | (2-) $OCH_3$ | $SC_3H_7$ | 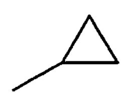 |
| $SO_2CH_3$ | (2-) $OCH_3$ | $SC_3H_7$-i | 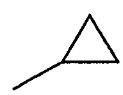 |
| $SO_2CH_3$ | (2-) $OCH_3$ | 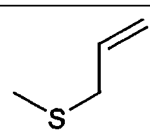 | 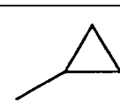 |

(fortgesetzt)

| $R^3$ | (Position-) $R^4$ | $R^5$ | $R^6$ |
|---|---|---|---|
| $SO_2CH_3$ | (2-) $OCH_3$ | 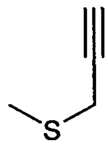 | 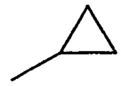 |
| $SO_2CH_3$ | (2-) $OCH_3$ | 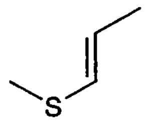 | 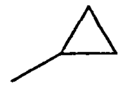 |
| $SO_2CH_3$ | (2-) $OCH_3$ | 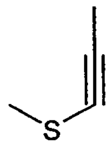 | 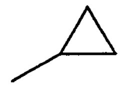 |
| $SO_2CH_3$ | (2-) $OCH_3$ | 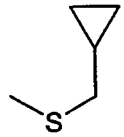 | 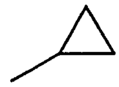 |
| $SO_2CH_3$ | (2-) $OCH_3$ | $SCH=C=CH_2$ | 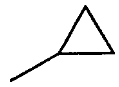 |
| $SO_2CH_3$ | (2-) $OCH_3$ | $SCH_2CN$ | 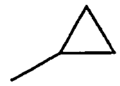 |
| $SO_2CH_3$ | (2-) $OCH_3$ | $SCH_2CH_2CN$ | 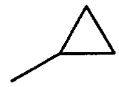 |
| $SO_2CH_3$ | (2-) $OCH_3$ | $OCH_3$ | 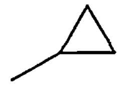 |
| $SO_2CH_3$ | (2-) $OCH_3$ | $OC_2H_5$ | 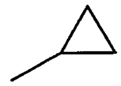 |
| $SO_2CH_3$ | (2-) $OCH_3$ | $OC_3H_7$ | 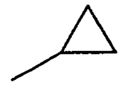 |
| $SO_2CH_3$ | (2-) $OCH_3$ | $OC_3H_7$-i | 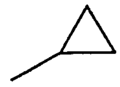 |
| $SO_2CH_3$ | (2-) $OCH_3$ | $OC_4H_9$ | 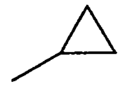 |

(fortgesetzt)

| $R^3$ | (Position-) $R^4$ | $R^5$ | $R^6$ |
|---|---|---|---|
| $SO_2CH_3$ | (2-) $OCH_3$ | $OCH_2CF_3$ | 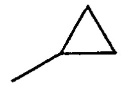 |
| $SO_2CH_3$ | (2-) $OCH_3$ | 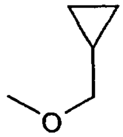 | 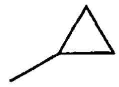 |
| $SO_2CH_3$ | (2-) $OCH_3$ | $OC_6H_5$ | 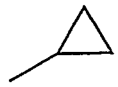 |
| $SO_2CH_3$ | (2-) $OCH_3$ | H | 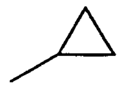 |
| $SO_2CH_3$ | (2-) $OCH_3$ | $CH_3$ | 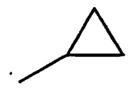 |
| $SO_2CH_3$ | (2-) $OCH_3$ | $C_2H_5$ | 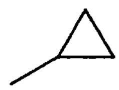 |
| $SO_2CH_3$ | (2-) $OCH_3$ | $C_3H_7$ | 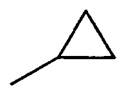 |
| $SO_2CH_3$ | (2-) $OCH_3$ | $C_3H_7$-i | 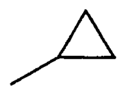 |
| $SO_2CH_3$ | (2-) $OCH_3$ | $C_4H_9$ | 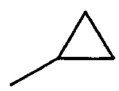 |
| $SO_2CH_3$ | (2-) $OCH_3$ | $C_4H_9$-i | 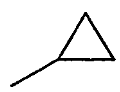 |
| $SO_2CH_3$ | (2-) $OCH_3$ | $C_4H_9$-s | 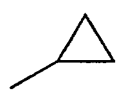 |
| $SO_2CH_3$ | (2-) $OCH_3$ | $C_4H_9$-t | 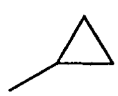 |
| $SO_2CH_3$ | (2-) $OCH_3$ | 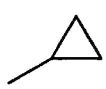 | 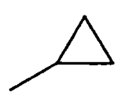 |

(fortgesetzt)

| $R^3$ | (Position-) $R^4$ | $R^5$ | $R^6$ |
|---|---|---|---|
| $SO_2CH_3$ | (2-) $OCH_3$ | 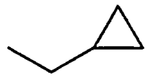 | 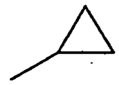 |
| $SO_2CH_3$ | (2-) $OCH_3$ | $CH{=}CHCH_3$ | 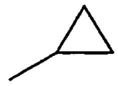 |
| $SO_2CH_3$ | (2-) $OCH_3$ | 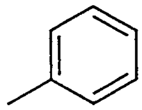 | 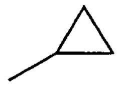 |
| $SO_2CH_3$ | (2-) $OCH_3$ | 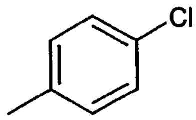 | 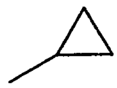 |
| $SO_2CH_3$ | (2-) $OCH_3$ | 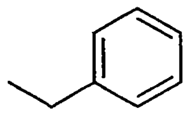 | 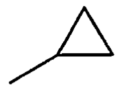 |
| $SO_2CH_3$ | (2-) $OCH_3$ | $N(CH_3)_2$ | 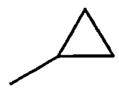 |
| $SO_2CH_3$ | (2-) $OCH_3$ | 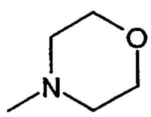 | 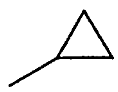 |
| $SO_2CH_3$ | (2-) $OCH_3$ | Cl | 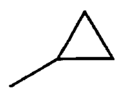 |
| $SO_2CH_3$ | (2-) $OCH_3$ | Br | 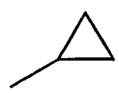 |
| Cl | (2-) $OCH_3$ | $CF_3$ | $N(CH_3)_2$ |
| Cl | (2-) $OCH_3$ | $SCH_3$ | $N(CH_3)_2$ |
| Cl | (2-) $OCH_3$ | $SC_2H_5$ | $N(CH_3)_2$ |
| Cl | (2-) $OCH_3$ | $SC_3H_7$ | $N(CH_3)_2$ |
| Cl | (2-) $OCH_3$ | $SC_3H_7$-i | $N(CH_3)_2$ |
| Cl | (2-) $OCH_3$ | 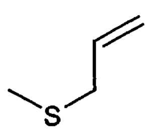 | $N(CH_3)_2$ |

(fortgesetzt)

| $R^3$ | (Position-) $R^4$ | $R^5$ | $R^6$ |
|---|---|---|---|
| Cl | (2-) $OCH_3$ | 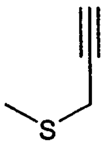 | $N(CH_3)_2$ |
| Cl | (2-) $OCH_3$ | 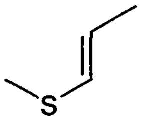 | $N(CH_3)_2$ |
| Cl | (2-) $OCH_3$ | 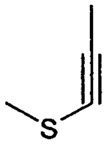 | $N(CH_3)_2$ |
| Cl | (2-) $OCH_3$ | 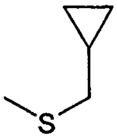 | $N(CH_3)_2$ |
| Cl | (2-) $OCH_3$ | $SCH=C=CH_2$ | $N(CH_3)_2$ |
| Cl | (2-) $OCH_3$ | $SCH_2CN$ | $N(CH_3)_2$ |
| Cl | (2-) $OCH_3$ | $SCH_2CH_2CN$ | $N(CH_3)_2$ |
| Cl | (2-) $OCH_3$ | $OCH_3$ | $N(CH_3)_2$ |
| Cl | (2-) $OCH_3$ | $OC_2H_5$ | $N(CH_3)_2$ |
| Cl | (2-) $OCH_3$ | $OC_3H_7$ | $N(CH_3)_2$ |
| Cl | (2-) $OCH_3$ | $OC_3H_7$-i | $N(CH_3)_2$ |
| Cl | (2-) $OCH_3$ | $OC_4H_9$ | $N(CH_3)_2$ |
| Cl | (2-) $OCH_3$ | $OCH_2CF_3$ | $N(CH_3)_2$ |
| Cl | (2-) $OCH_3$ | 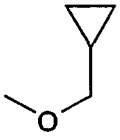 | $N(CH_3)_2$ |
| Cl | (2-) $OCH_3$ | $OC_6H_5$ | $N(CH_3)_2$ |
| Cl | (2-) $OCH_3$ | H | $N(CH_3)_2$ |
| Cl | (2-) $OCH_3$ | $CH_3$ | $N(CH_3)_2$ |
| Cl | (2-) $OCH_3$ | $C_2H_5$ | $N(CH_3)_2$ |
| Cl | (2-) $OCH_3$ | $C_3H_7$ | $N(CH_3)_2$ |
| Cl | (2-) $OCH_3$ | $C_3H_7$-i | $N(CH_3)_2$ |
| Cl | (2-) $OCH_3$ | $C_4H_9$ | $N(CH_3)_2$ |
| Cl | (2-) $OCH_3$ | $C_4H_9$-i | $N(CH_3)_2$ |
| Cl | (2-) $OCH_3$ | $C_4H_9$-s | $N(CH_3)_2$ |
| Cl | (2-) $OCH_3$ | $C_4H_9$-t | $N(CH_3)_2$ |

(fortgesetzt)

| $R^3$ | (Position-) $R^4$ | $R^5$ | $R^6$ |
|---|---|---|---|
| Cl | (2-) $OCH_3$ | 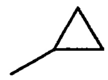 | $N(CH_3)_2$ |
| Cl | (2-) $OCH_3$ | 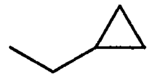 | $N(CH_3)_2$ |
| Cl | (2-) $OCH_3$ | $CH=CHCH_3$ | $N(CH_3)_2$ |
| Cl | (2-) $OCH_3$ | 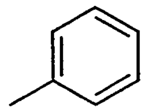 | $N(CH_3)_2$ |
| Cl | (2-) $OCH_3$ | 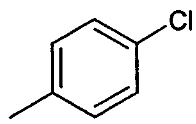 | $N(CH_3)_2$ |
| Cl | (2-) $OCH_3$ | 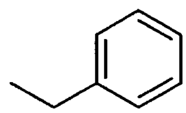 | $N(CH_3)_2$ |
| Cl | (2-) $OCH_3$ | $N(CH_3)_2$ | $N(CH_3)_2$ |
| Cl | (2-) $OCH_3$ | 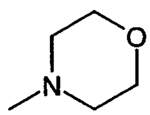 | $N(CH_3)_2$ |
| Cl | (2-) $OCH_3$ | Cl | $N(CH_3)_2$ |
| Cl | (2-) $OCH_3$ | Br | $N(CH_3)_2$ |
| $SO_2CH_3$ | (2-) $OCH_3$ | $CF_3$ | $N(CH_3)_2$ |
| $SO_2CH_3$ | (2-) $OCH_3$ | $SCH_3$ | $N(CH_3)_2$ |
| $SO_2CH_3$ | (2-) $OCH_3$ | $SC_2H_5$ | $N(CH_3)_2$ |
| $SO_2CH_3$ | (2-) $OCH_3$ | $SC_3H_7$ | $N(CH_3)_2$ |
| $SO_2CH_3$ | (2-) $OCH_3$ | $SC_3H_7$-i | $N(CH_3)_2$ |
| $SO_2CH_3$ | (2-) $OCH_3$ | 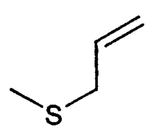 | $N(CH_3)_2$ |
| $SO_2CH_3$ | (2-) $OCH_3$ | 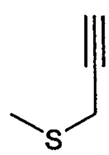 | $N(CH_3)_2$ |
| $SO_2CH_3$ | (2-) $OCH_3$ | 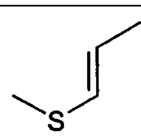 | $N(CH_3)_2$ |

(fortgesetzt)

| $R^3$ | (Position-) $R^4$ | $R^5$ | $R^6$ |
|---|---|---|---|
| $SO_2CH_3$ | (2-) $OCH_3$ | 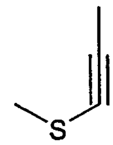 | $N(CH_3)_2$ |
| $SO_2CH_3$ | (2-) $OCH_3$ | 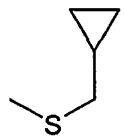 | $N(CH_3)_2$ |
| $SO_2CH_3$ | (2-) $OCH_3$ | $SCH=C=CH_2$ | $N(CH_3)_2$ |
| $SO_2CH_3$ | (2-) $OCH_3$ | $SCH_2CN$ | $N(CH_3)_2$ |
| $SO_2CH_3$ | (2-) $OCH_3$ | $SCH_2CH_2CN$ | $N(CH_3)_2$ |
| $SO_2CH_3$ | (2-) $OCH_3$ | $OCH_3$ | $N(CH_3)_2$ |
| $SO_2CH_3$ | (2-) $OCH_3$ | $OC_2H_5$ | $N(CH_3)_2$ |
| $SO_2CH_3$ | (2-) $OCH_3$ | $OC_3H_7$ | $N(CH_3)_2$ |
| $SO_2CH_3$ | (2-) $OCH_3$ | $OC_3H_7$-i | $N(CH_3)_2$ |
| $SO_2CH_3$ | (2-) $OCH_3$ | $OC_4H_9$ | $N(CH_3)_2$ |
| $SO_2CH_3$ | (2-) $OCH_3$ | $OCH_2CF_3$ | $N(CH_3)_2$ |
| $SO_2CH_3$ | (2-) $OCH_3$ | 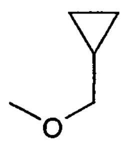 | $N(CH_3)_2$ |
| $SO_2CH_3$ | (2-) $OCH_3$ | $OC_6H_5$ | $N(CH_3)_2$ |
| $SO_2CH_3$ | (2-) $OCH_3$ | H | $N(CH_3)_2$ |
| $SO_2CH_3$ | (2-) $OCH_3$ | $CH_3$ | $N(CH_3)_2$ |
| $SO_2CH_3$ | (2-) $OCH_3$ | $C_2H_5$ | $N(CH_3)_2$ |
| $SO_2CH_3$ | (2-) $OCH_3$ | $C_3H_7$ | $N(CH_3)_2$ |
| $SO_2CH_3$ | (2-) $OCH_3$ | $C_3H_7$-i | $N(CH_3)_2$ |
| $SO_2CH_3$ | (2-) $OCH_3$ | $C_4H_9$ | $N(CH_3)_2$ |
| $SO_2CH_3$ | (2-) $OCH_3$ | $C_4H_9$-i | $N(CH_3)_2$ |
| $SO_2CH_3$ | (2-) $OCH_3$ | $C_4H_9$-s | $N(CH_3)_2$ |
| $SO_2CH_3$ | (2-) $OCH_3$ | $C_4H_9$-t | $N(CH_3)_2$ |
| $SO_2CH_3$ | (2-) $OCH_3$ | 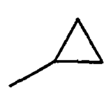 | $N(CH_3)_2$ |
| $SO_2CH_3$ | (2-) $OCH_3$ | 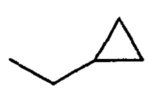 | $N(CH_3)_2$ |
| $SO_2CH_3$ | (2-) $OCH_3$ | $CH=CHCH_3$ | $N(CH_3)_2$ |

(fortgesetzt)

| $R^3$ | (Position-) $R^4$ | $R^5$ | $R^6$ |
|---|---|---|---|
| $SO_2CH_3$ | (2-) $OCH_3$ | 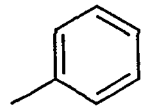 | $N(CH_3)_2$ |
| $SO_2CH_3$ | (2-) $OCH_3$ | 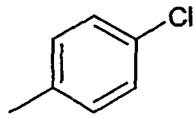 | $N(CH_3)_2$ |
| $SO_2CH_3$ | (2-) $OCH_3$ | 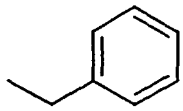 | $N(CH_3)_2$ |
| $SO_2CH_3$ | (2-) $OCH_3$ | $N(CH_3)_2$ | $N(CH_3)_2$ |
| $SO_2CH_3$ | (2-) $OCH_3$ | 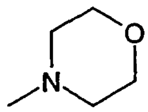 | $N(CH_3)_2$ |
| $SO_2CH_3$ | (2-) $OCH_3$ | Cl | $N(CH_3)_2$ |
| $SO_2CH_3$ | (2-) $OCH_3$ | Br | $N(CH_3)_2$ |
| Cl | (2-) $OCH_3$ | $CH_3$ | $OCH_3$ |
| Cl | (2-) $OCH_3$ | $C_2H_5$ | $OCH_3$ |
| Cl | (2-) $OCH_3$ | $C_3H_7$ | $OCH_3$ |
| Cl | (2-) $OCH_3$ | $SCH_3$ | $OCH_3$ |
| Cl | (2-) $OCH_3$ | $SC_2H_5$ | $OCH_3$ |
| Cl | (2-) $OCH_3$ | $OCH_3$ | $OCH_3$ |
| Cl | (2-) $OCH_3$ | $OC_2H_5$ | $OCH_3$ |
| Cl | (2-) $OCH_3$ | $CH_3$ | $OC_2H_5$ |
| Cl | (2-) $OCH_3$ | $C_2H_5$ | $OC_2H_5$ |
| Cl | (2-) $OCH_3$ | $C_3H_7$ | $OC_2H_5$ |
| Cl | (2-) $OCH_3$ | $SCH_3$ | $OC_2H_5$ |
| Cl | (2-) $OCH_3$ | $SC_2H_5$ | $OC_2H_5$ |
| Cl | (2-) $OCH_3$ | $OCH_3$ | $OC_2H_5$ |
| Cl | (2-) $OCH_3$ | $OC_2H_5$ | $OC_2H_5$ |
| $SO_2CH_3$ | (2-) $OCH_3$ | Cl | $OCH_3$ |
| $SO_2CH_3$ | (2-) $OCH_3$ | Br | $OCH_3$ |
| $SO_2CH_3$ | (2-) $OCH_3$ | $CH_3$ | $OCH_3$ |
| $SO_2CH_3$ | (2-) $OCH_3$ | $C_2H_5$ | $OCH_3$ |
| $SO_2CH_3$ | (2-) $OCH_3$ | $C_3H_7$ | $OCH_3$ |
| $SO_2CH_3$ | (2-) $OCH_3$ | $SCH_3$ | $OCH_3$ |
| $SO_2CH_3$ | (2-) $OCH_3$ | $SC_2H_5$ | $OCH_3$ |
| $SO_2CH_3$ | (2-) $OCH_3$ | $OCH_3$ | $OC_2H_5$ |

(fortgesetzt)

| $R^3$ | (Position-) $R^4$ | $R^5$ | $R^6$ |
|---|---|---|---|
| $SO_2CH_3$ | (2-) $OCH_3$ | $OC_2H_5$ | $OC_2H_5$ |
| $SO_2CH_3$ | (2-) $OCH_3$ | $CH_3$ | $OC_2H_5$ |
| $SO_2CH_3$ | (2-) $OCH_3$ | $C_2H_5$ | $OC_2H_5$ |
| $SO_2CH_3$ | (2-) $OCH_3$ | $C_3H_7$ | $OC_2H_5$ |
| $SO_2CH_3$ | (2-) $OCH_3$ | $SCH_3$ | $OC_2H_5$ |
| $SO_2CH_3$ | (2-) $OCH_3$ | $SC_2H_5$ | $OC_2H_5$ |
| $SO_2CH_3$ | (2-) $OCH_3$ | $OCH_3$ | $OC_2H_5$ |
| Cl | (2-) F | $OCH_3$ | $CH_3$ |
| Cl | (2-) F | $OCH_3$ | $C_2H_5$ |
| Cl | (2-) F | $C_2H_5$ | $OCH_3$ |
| Cl | (2-) F | $C_2H_5$ | $C_2H_5$ |
| Cl | (2-) F | OC | $CH_3$ |
| Cl | (2-) F | $_2H_5$ $OC_2H_5$ | $C_2H_5$ |
| Cl | (2-) F | $OCH_3$ | 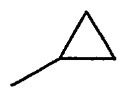 |
| Cl | (2-) F | $OC_2H_5$ | 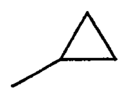 |
| Cl | (2-) F | $SCH_3$ | $CH_3$ |
| Cl | (2-) F | $SCH_3$ | 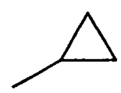 |
| Cl | (2-) F | $CH_3$ | $CH_3$ |
| Cl | (2-) F | $CH_3$ | 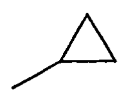 |

Gruppe 7

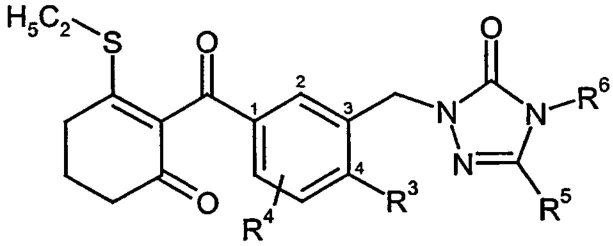

(IB-2)

$R^3$, $R^4$, $R^5$ und $R^6$ haben hierbei beispielhaft die vorausgehend in Gruppe 6 angegebenen Bedeutungen.

Gruppe 8

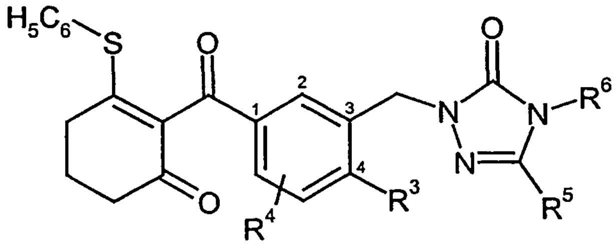

(IB-3)

$R^3$, $R^4$, $R^5$ und $R^6$ haben hierbei beispielhaft die vorausgehend in Gruppe 6 angegebenen Bedeutungen.

Gruppe 9

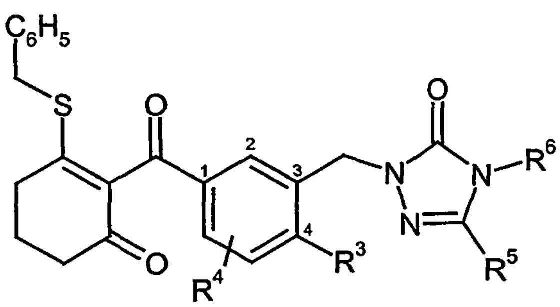

(IB-4)

$R^3$, $R^4$, $R^5$ und $R^6$ haben hierbei beispielhaft die vorausgehend in Gruppe 6 angegebenen Bedeutungen.

Gruppe 10

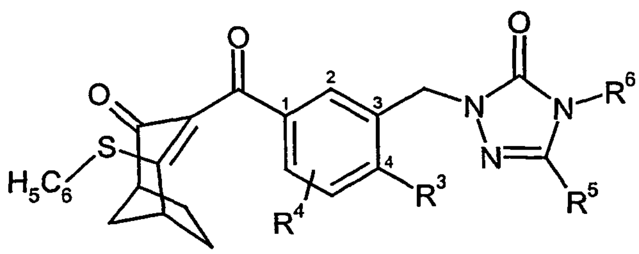

(IB-5)

$R^3$, $R^4$, $R^5$ und $R^6$ haben hierbei beispielhaft die vorausgehend in Gruppe 6 angegebenen Bedeutungen.

Die neuen substituierten Benzoylcyclohexenone der Formel (I) zeichnen sich durch starke und selektive herbizide Wirksamkeit aus.

Man erhält die neuen substituierten Benzoylcyclohexenone der Formel (I), wenn man substituierte Cyclohexandione der Formel (II)

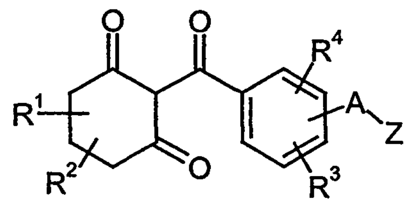

(II)

in welcher
A, $R^1$, $R^2$, $R^3$, $R^4$ und Z die oben angegebene Bedeutung haben,
mit einem Halogenierungsmittel, gegebenenfalls in Gegenwart eines Reaktionshilfsmittel und gegebenenfalls in Gegenwart eines Verdünnungsmittel, umsetzt und die hierbei (in der ersten Umsetzungsstufe) erhaltenen Halogencyclohexenone der Formel (III)

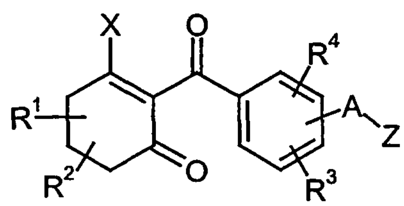

(III)

in welcher

A, $R^1$, $R^2$, $R^3$, $R^4$ und Z die oben angegebene Bedeutung haben,

X für Halogen, bevorzugt für Fluor oder Chlor, besonders bevorzugt für Chlor steht,

- nach Zwischenisolierung oder ohne Zwischenisolierung ("in situ") -

in einer zweiten Umsetzungsstufe mit Mercaptoverbindungen der Formel (IV)

HS-Y (IV)

in welcher

Y die oben angegebene Bedeutung hat,

gegebenenfalls in Gegenwart eines Reaktionshilfsmittel und gegebenenfalls in Gegenwart eines Verdünnungsmittel, umsetzt,
und gegebenenfalls im Anschluss daran an den so erhaltenen Verbindungen der Formel (I) im Rahmen der Substituentendefinition auf übliche Weise elektrophile oder nucleophile Substitutionsreaktionen bzw. Oxidations- oder Reduktionsreaktionen durchführt oder die Verbindungen der Formel (I) auf übliche Weise in Salze überführt.
Verwendet man beispielsweise 2-[4-Chlor-2-[(3,4-Dimethyl-5-oxo-4,5-dihydro-1H-1,2,4-triazol-1-yl)-methyl]-benzoyl]-cyclohexan-1,3-dion und Phosgen sowie anschließend Thiophenol als Ausgangsstoffe, so kann der Reaktionsablauf beim erfindungsgemäßen Verfahren durch das folgende Formelschema skizziert werden:

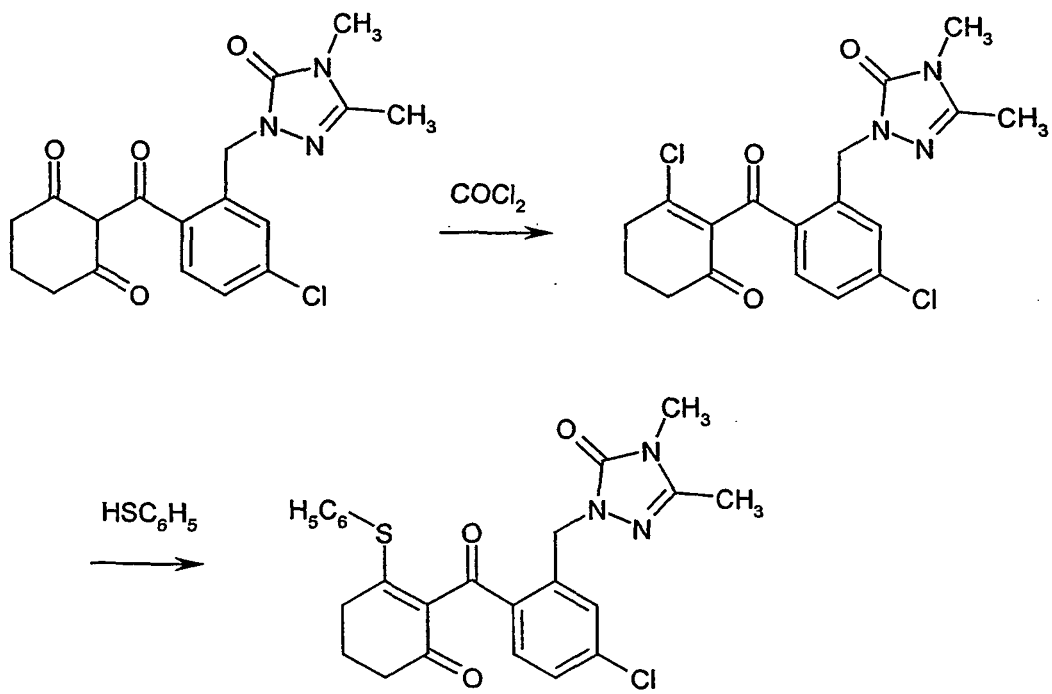


Die beim erfindungsgemäßen Verfahren zur Herstellung von Verbindungen der allgemeinen Formel (I) als Ausgangsstoffe zu verwendenden substituierten Benzoylcyclohexandione sind durch die Formel (II) allgemein definiert. In der allgemeinen Formel (II) haben A, $R^1$, $R^2$, $R^3$, $R^4$ und Z vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt, ganz besonders bevorzugt oder am meisten bevorzugt für A, $R^1$, $R^2$, $R^3$, $R^4$ und Z angegeben worden sind.

Die Ausgangsstoffe der allgemeinen Formel (II) sind bekannt und/oder können nach an sich bekannten Verfahren hergestellt werden (vgl. WO-A-00/05221).

Das erfindungsgemäße Verfahren zur Herstellung der Verbindungen der allgemeinen Formel (I) wird in der ersten Umsetzungsstufe unter Verwendung eines Halogenierungsmittels durchgeführt. Es können hierbei die üblichen Halogenierungsmittel verwendet werden, die zur Umwandlung von Enolen in entsprechende Halogenalkene geeignet sind. Halogen steht hierbei vorzugsweise für Fluor, Chlor oder Brom, insbesondere für Chlor. Es werden also vorzugsweise Chlorierungsmittel oder Bromierungsmittel eingesetzt. Hierzu gehören vorzugsweise Phosgen, Oxalsäuredichlorid, Oxalsäuredibromid, Phosphor(III)-chlorid, Phosphor(III)-bromid, Phosphorylchlorid, Thionylchlorid und Thionylbromid.

Das erfindungsgemäße Verfahren zur Herstellung der Verbindungen der allgemeinen Formel (I) wird in der ersten Umsetzungsstufe vorzugsweise unter Verwendung eines Reaktionshilfsmittels durchgeführt. Es kommen hierbei die für Halogenierungsreaktionen üblichen Reaktionshilfsmittel in Betracht. Hierzu gehören vorzugsweise Acetonitril, N,N-Dimethyl-formamid, N,N-Diethyl-formamid, N,N-Dipropyl-formamid und N,N-Dibutyl-formamid sowie N-Methyl-pyrrolidon.

Die beim erfindungsgemäßen Verfahren zur Herstellung von Verbindungen der allgemeinen Formel (I) weiter als Ausgangsstoffe zu verwendenden Mercaptoverbindungen sind durch die Formel (IV) allgemein definiert. In der allgemeinen Formel (IV) hat Y vorzugsweise insbesondere diejenige Bedeutung, die bereits oben im Zusammenhang mit der Beschreibung der erfindungsgemäßen Verbindungen der allgemeinen Formel (I) als bevorzugt, besonders bevorzugt oder ganz besonders bevorzugt für Y angegeben worden ist.

Die Ausgangsstoffe der allgemeinen Formel (IV) sind bekannte organische Synthesechemikalien.

Das erfindungsgemäße Verfahren zur Herstellung der Verbindungen der allgemeinen Formel (I) wird in der zweiten Umsetzungsstufe vorzugsweise unter Verwendung eines Reaktionshilfsmittels durchgeführt. Als Reaktionshilfsmittel kommen hierbei im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erste und die zweite Stufe des erfindungsgemäßen Verfahrens zur Herstellung der Verbindungen der allgemeinen Formel (I) werden vorzugsweise unter Verwendung von Verdünnungsmitteln durchgeführt. Als Verdünnungsmittel kommen hierbei vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Düsopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether oder Ethylenglykolmonoethylether.

Die Reaktionstemperaturen können bei der Durchführung der ersten und der zweiten Stufe des erfindungsgemäßen Verfahrens in einem größeren Bereich variiert werden. Im allgemeinen arbeitet man bei Temperaturen zwischen -20˚C und +150˚C, vorzugsweise zwischen 0˚C und 120˚C.

Das erfindungsgemäße Verfahren wird in beiden Stufen im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahren werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart geeigneter Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

<u>Dikotyle Kulturen der Gattungen:</u> Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

<u>Monokotyle Unkräuter der Gattungen:</u> Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

<u>Monokotyle Kulturen der Gattungen:</u> Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-,

Wein-, Citrus-, Nuß-, Bananen-, Kaffee-, Tee-, Gumini-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen

Safener enthalten.

Für die Mischungen kommen bekannte Herbizide infrage, beispielsweise Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlomitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfaron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Procarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

## Herstellungsbeispiele:

### Beispiel 1

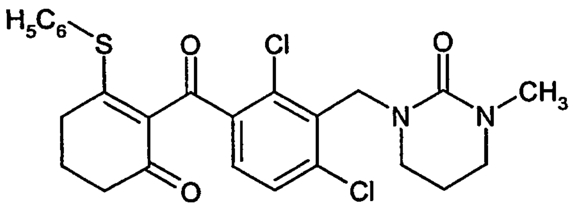

Eine Mischung aus 1,3 g (3 mMol) 2-[2,4-Dichlor-3-[(3-methyl-2-oxo-tetrahydro-1(2H)-pyrimidinyl)-methyl]-benzoyl]-cyclohexan-1,3-dion, 1,0 g (7,5 mMol) Oxalsäuredichlorid, 2 Tropfen N,N-Dimethyl-formamid und 30 ml Methylenchlorid wird 30 Minuten unter Rückfluss erhitzt und anschließend im Wasserstrahlvakuum eingeengt. Der Rückstand wird in 50 ml Tetrahydrofuran aufgenommen und mit 0,33 g (3 mMol) Thiophenol versetzt. Unter Kühlen mit Eis werden dann 0,50 g (4,5 mMol) Triethylamin tropfenweise dazu gegeben. Nach Entfernen der Eiskühlung wird die Mischung 2 Stunden bei Raumtemperatur gerührt und anschließend im Wasserstrahlvakuum eingeengt. Der Rückstand wird in Methylenchlorid aufgenommen, je einmal mit 1N-Salzsäure, Wasser und gesättigter wässriger Natriumchlorid-Lösung gewaschen, mit Natriumsulfat getrocknet und filtriert. Das Filtrat wird im Wasserstrahlvakuum eingeengt, der Rückstand mit Diethylether digeriert und das kristallin angefallene Produkt durch Absaugen isoliert.

Man erhält 0,91 g (61% der Theorie) 1-[2,6-Dichlor-3-[(6-oxo-2-phenylthio-1-cyclohexen-1-yl)-carbonyl]-benzyl]-3-methyl-tetrahydro-2(1H)-pyrimidinon vom Schmelzpunkt 140°C.

Analog zu Beispiel 1 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) - bzw. der allgemeinen Formel (ID) - hergestellt werden.

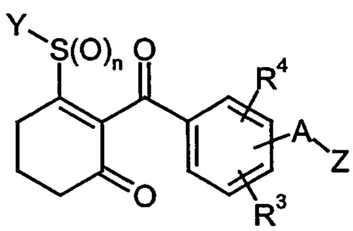

(ID)

Tabelle 1: Beispiele für die Verbindungen der Formel (ID)

| Bsp.-Nr | n | (Position) $R^3$ | (Position) $R^4$ | (Position) -A-Z | Y | Physikal. Daten |
|---|---|---|---|---|---|---|
| 2 | 0 | (2) Cl | (4) Cl | (3) 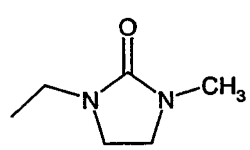 | $C_6H_5$ | Fp.:169°C logP = 3,02 [a)] |
| 3 | 0 | (2) Cl | (4) Cl | (3) 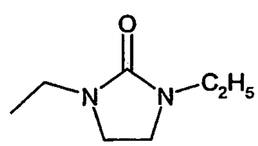 | $C_6H_5$ | Fp.:149°C logP = 3,30 [a)] |

(fortgesetzt)

| Bsp.-Nr | n | (Position) $R^3$ | (Position) $R^4$ | (Position) -A-Z | Y | Physikal. Daten |
|---|---|---|---|---|---|---|
| 4 | 0 | (2) Cl | (4) Cl | (3) 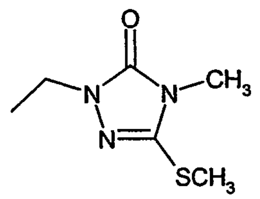 | $C_6H_5$ | Fp.:159°C logP = 3,29 [a)] |
| 5 | 0 | (2) Cl | (4) Cl | (3) 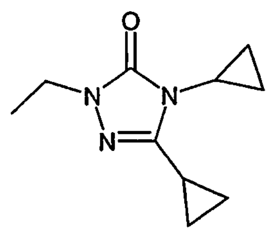 | $C_6H_5$ | Fp.:163°C logP = 3,50 [a)] |
| 6 | 0 | (2) Cl | (4) $SO_2CH_3$ | (3) 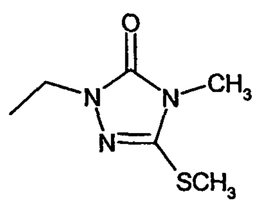 | $C_6H_5$ | Fp.:195°C logP = 2,84 [a)] |
| 7 | 0 | (2) Cl | (4) $SO_2CH_3$ | (3) 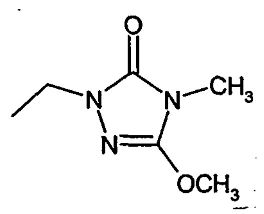 | $C_6H_5$ | Fp.: 234°C logP = 2,67 [a)] |
| 8 | 0 | (2) Cl | (4) $SO_2CH_3$ | (3) 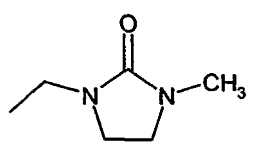 | $C_6H_5$ | Fp.: 203°C logP = 2,56 [a)] |
| 9 | 0 | (2) Cl | (4) $SO_2CH_3$ | (3) 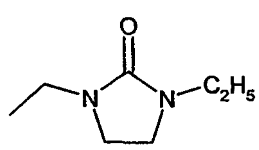 | $C_6H_5$ | Fp.: 184°C logP = 2,79 [a)] |

(fortgesetzt)

| Bsp.-Nr | n | (Position) $R^3$ | (Position) $R^4$ | (Position) -A-Z | Y | Physikal. Daten |
|---|---|---|---|---|---|---|
| 10 | 0 | (2) Cl | (4) $SO_2CH_3$ | (3) 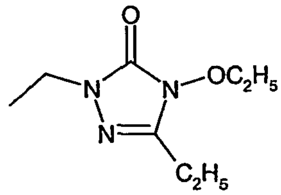 | $C_6H_5$ | Fp.: 228°C logP = 3,13 [a)] |
| 11 | 0 | (2) Cl | (4) Cl | (3) 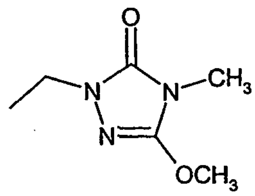 | $C_6H_5$ | Fp.:176°C logP = 3,06[a)] |
| 12 | 0 | (2) Cl | (4) Cl | (3) 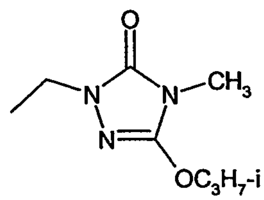 | $C_6H_5$ | Fp.: 145°C logP = 3,69 [a)] |
| 13 | 0 | (2) Cl | (4) Cl | (3) 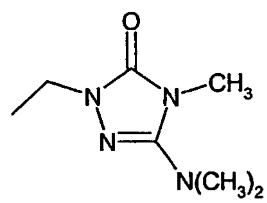 | $C_6H_5$ | Fp.: 207°C logP = 3,10 [a)] |
| 14 | 2 | (2) Cl | (4) Cl | (3) 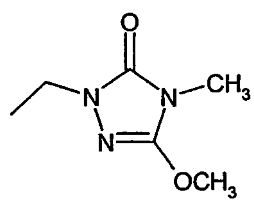 | $C_6H_5$ | Fp.:180°C logP = 2,62 [a)] |
| 15 | 1 | (2) Cl | (4) Cl | (3) 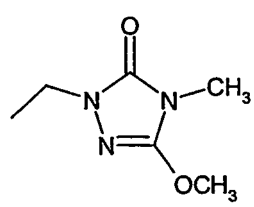 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 16 | 0 | (4) $CF_3$ | - | (2) 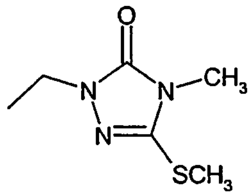 | $C_6H_5$ | logP = 3,58 [a)] |
| 17 | 0 | (4) $CF_3$ | - | (2) 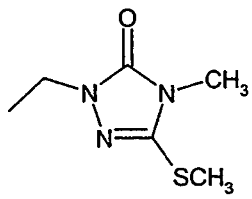 | $CH_3$ | logP = 2,63[a)] |
| 18 | 0 | (4) $CF_3$ | - | (2) 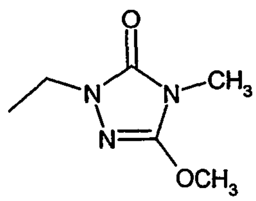 | $C_6H_5$ | logP = 3,30 [a)] |
| 19 | 0 | (4) $CF_3$ | - | (2) 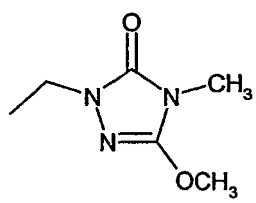 | $CH_3$ | logP = 2,41 [a)] |
| 20 | 0 | (4) $CF_3$ | - | (2) 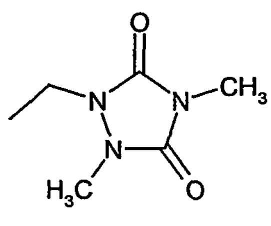 | $C_6H_5$ | logP = 3,25 [a)] |
| 21 | 0 | (2) Cl | (4) Cl | (3) 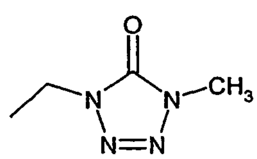 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 22 | 0 | (2) Cl | (4) $SO_2CH_3$ | (3) 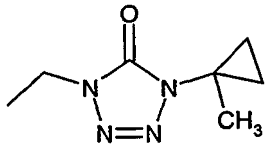 | $C_6H_5$ | |
| 23 | 0 | (2) Cl | (4) $SO_2CH_3$ | (3) 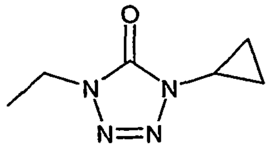 | $C_6H_5$ | |
| 24 | 0 | (2) Cl | (4) Cl | (3) 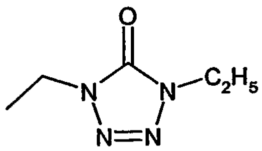 | $C_6H_5$ | |
| 25 | 0 | (2) Cl | (4) Cl | (3) 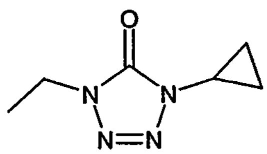 | $C_6H_5$ | |
| 26 | 0 | (2) Cl | (4) $SO_2CH_3$ | (3) 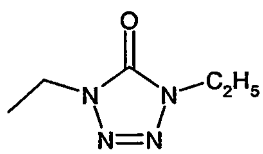 | $C_6H_5$ | |
| 27 | 0 | (2) Cl | (4) Cl | (3) 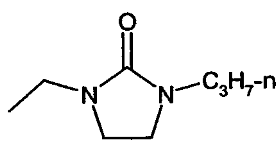 | $C_6H_5$ | |
| 28 | 0 | (2) Cl | (4) Cl | (3) 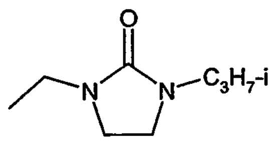 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 29 | 0 | (2) Cl | (4) Cl | (3) 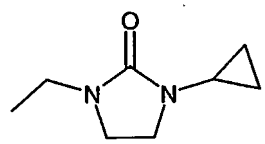 | $C_6H_5$ | |
| 30 | 0 | (2) Cl | (4) Cl | (3) 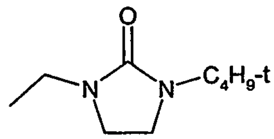 | $C_6H_5$ | |
| 31 | 0 | (2) Cl | (4) Cl | (3) 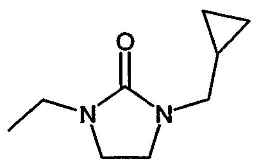 | $C_6H_5$ | |
| 32 | 0 | (2) Cl | (4) Cl | (3) 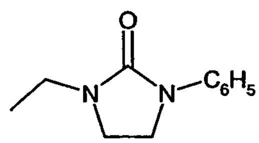 | $C_6H_5$ | |
| 33 | 0 | (2) Cl | (4) Cl | (3) 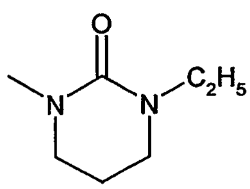 | $C_6H_5$ | |
| 34 | 0 | (2) Cl | (4) Cl | (3) 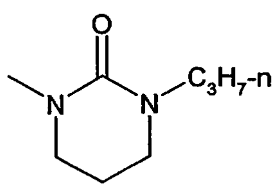 | $C_6H_5$ | |
| 35 | 0 | (2) Cl | (4) Cl | (3) 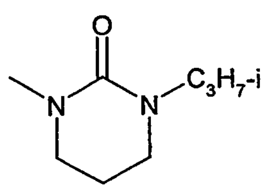 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 36 | 0 | (2) Cl | (4) Cl | (3) 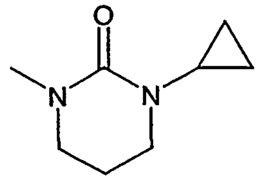 | $C_6H_5$ | |
| 37 | 0 | (2) Cl | (4) Cl | (3) 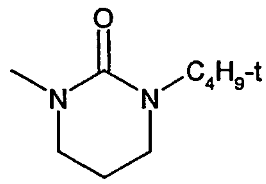 | $C_6H_5$ | |
| 38 | 0 | (2) Cl | (4) Cl | (3) 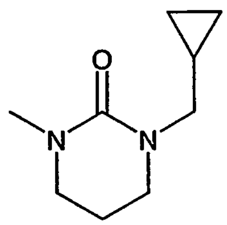 | $C_6H_5$ | |
| 39 | 0 | (2) Cl | (4) $SO_2CH_3$ | (3) 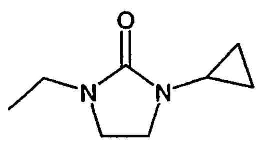 | $C_6H_5$ | |
| 40 | 0 | (2) Cl | (4) $SO_2CH_3$ | (3) 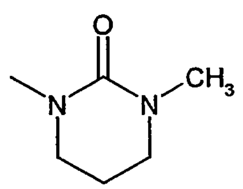 | $C_6H_5$ | |
| 41 | 0 | (2) Cl | (4) $SO_2CH_3$ | (3) 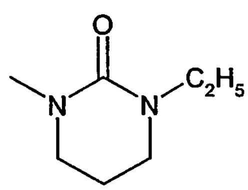 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 42 | 0 | (2) $OCH_3$ | (4) Cl | (3) 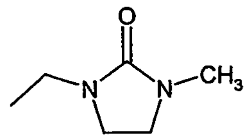 | $C_6H_5$ | |
| 43 | 0 | (2) $OCH_3$ | (4) Cl | (3) 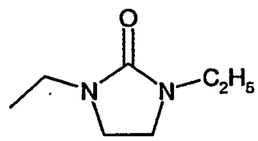 | $C_6H_5$ | |
| 44 | 0 | (2) $OCH_3$ | (4) Cl | (3) 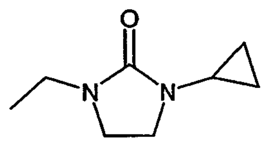 | $C_6H_5$ | |
| 45 | 0 | (2) $OCH_3$ | (4) Cl | (3) 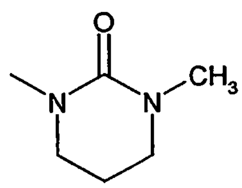 | $C_6H_5$ | |
| 46 | 0 | (2) $OCH_3$ | (4) Cl | (3) 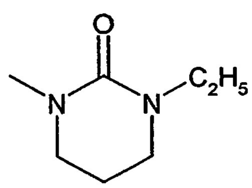 | $C_6H_5$ | |
| 47 | 0 | (2) Cl | (4) $CF_3$ | (3) 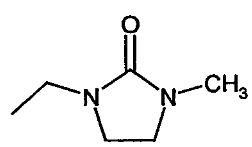 | $C_6H_5$ | |
| 48 | 0 | (2) Cl | (4) $CF_3$ | (3) 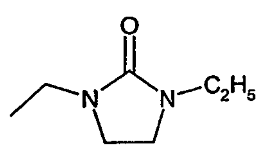 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 49 | 0 | (2) Cl | (4) $CF_3$ | (3) 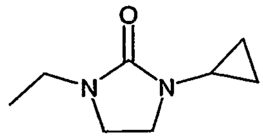 | $C_6H_5$ | |
| 50 | 0 | (2) Cl | (4) $CF_3$ | (3) 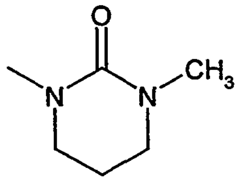 | $C_6H_5$ | |
| 51 | 0 | (2) Cl | (4) $CF_3$ | (3) 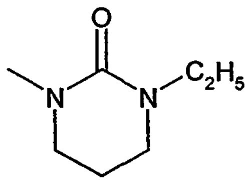 | $C_6H_5$ | |
| 52 | 0 | (2) Cl | - | (4) 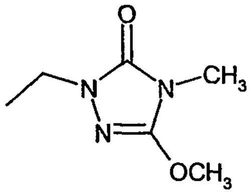 | $C_6H_5$ | |
| 53 | 0 | (2) $OCH_3$ | (4) Cl | (3) 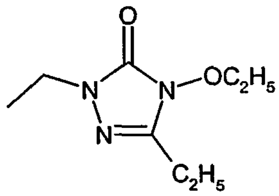 | $C_6H_5$ | |
| 54 | 0 | (2) $OCH_3$ | (4) Cl | (3) 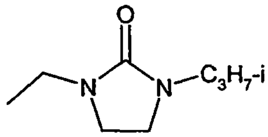 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 55 | 0 | (2) $OCH_3$ | (4) Cl | (3) 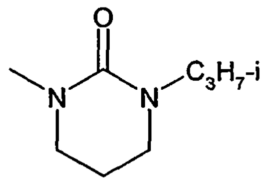 | $C_6H_5$ | |
| 56 | 0 | (2) Cl | (4) $SO_2CH_3$ | (3) 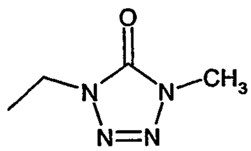 | $C_2H_5$ | Fp.: 85°C |
| 57 | 0 | (2) Cl | (4) $SO_2CH_3$ | (3) 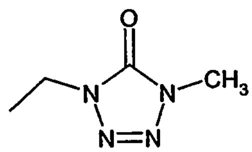 | $C_6H_5$ | Fp.: 226°C |
| 58 | 0 | (2) Cl | - | (4) 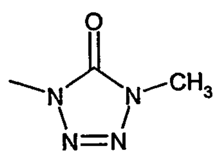 | $C_6H_5$ | Fp.:183°C |
| 59 | 0 | (2) Cl | (4) $SO_2CH_3$ | (3) 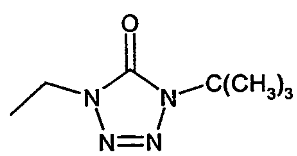 | $C_6H_5$ | Fp.:159°C |
| 60 | 0 | (2) $NO_2$ | - | (4) 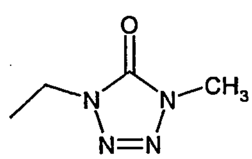 | $C_6H_5$ | Fp.:77°C |
| 61 | 0 | (4) $CF_3$ | - | (2) 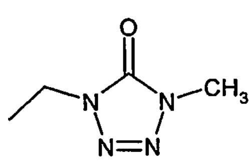 | $C_6H_5$ | Fp.:167°C |

(fortgesetzt)

| Bsp.-Nr | n | (Position) $R^3$ | (Position) $R^4$ | (Position) -A-Z | Y | Physikal. Daten |
|---|---|---|---|---|---|---|
| 62 | 0 | (2) Cl | (4) Cl | (3) 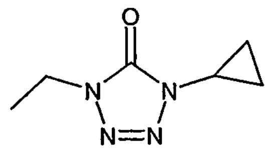 | $C_2H_5$ | $n_D^{20} = 1.6122$ |
| 63 | 0 | (2) Cl | (4) Cl | (3) 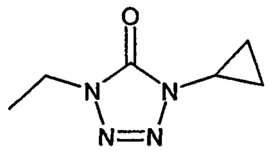 | $CH_3$ | $n_D^{20} = 1.6018$ |
| 64 | 0 | (2) Cl | (4) $SO_2CH_3$ | (3) 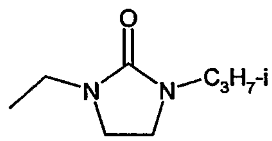 | $C_6H_5$ | Fp.: 116˚C |
| 65 | 0 | (2) Cl | - | (4) 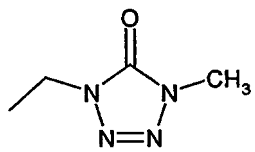 | $C_6H_5$ | Fp.:88˚C |
| 66 | 0 | (2) Cl | - | (4) 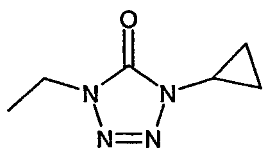 | $C_6H_5$ | Fp.:59˚C |
| 67 | 0 | (2) Cl | (4) $SCH_3$ | (3) 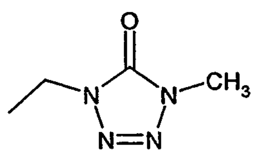 | $CH_3$ | Fp.:82˚C |
| 68 | 0 | (2) Cl | (4) $SC_2H_5$ | (3) 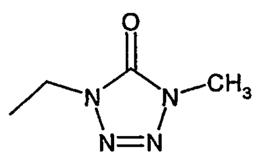 | $C_2H_5$ | Fp.: 64˚C |

(fortgesetzt)

| Bsp.-Nr | n | (Position) $R^3$ | (Position) $R^4$ | (Position) -A-Z | Y | Physikal. Daten |
|---|---|---|---|---|---|---|
| 69 | 0 | (2) Cl | (4) $SO_2CH_3$ | (3) 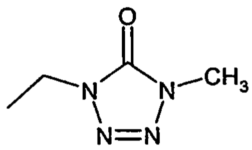 | $CH_3$ | Fp.:100˚C |
| 70 | 0 | (2) Cl | (4) $SO_2CH_3$ | (3) 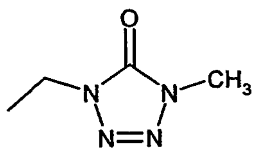 | 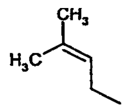 | Fp.: 96˚C |
| 71 | 0 | (4) F | - | (2) 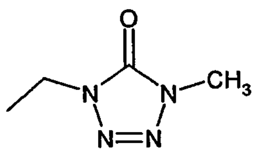 | $C_6H_5$ | |
| 72 | 0 | (4) Cl | - | (2) 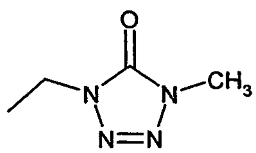 | $C_6H_5$ | |
| 73 | 0 | (4) Br | - | (2) 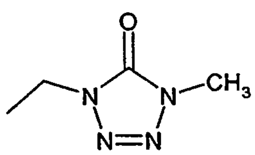 | $C_6H_5$ | |
| 74 | 0 | (4) I | - | (2) 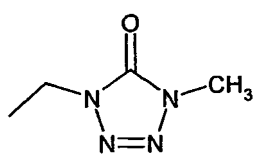 | $C_6H_5$ | |
| 75 | 0 | (4) CN | - | (2) 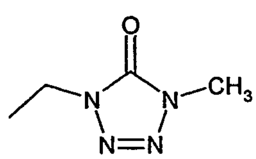 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 76 | 0 | (4) $CF_3$ | - | (2) 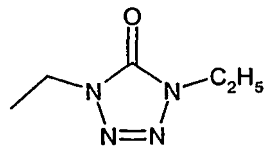 | $C_6H_5$ | |
| 77 | 0 | (4) $CF_3$ | - | (2) 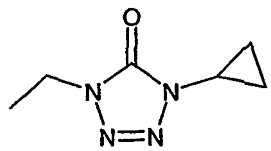 | $C_6H_5$ | |
| 78 | 0 | (2) Cl | (4) Cl | (3) 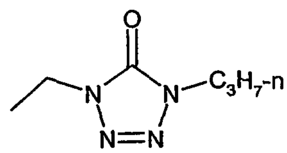 | $C_6H_5$ | |
| 79 | 0 | (2) Cl | (4) Cl | (3) 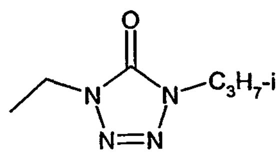 | $C_6H_5$ | |
| 80 | 0 | (2) Cl | (4) Cl | (3) 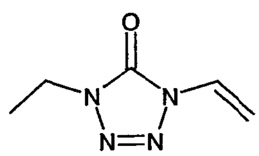 | $C_6H_5$ | |
| 81 | 0 | (2) Cl | (4) Cl | (3) 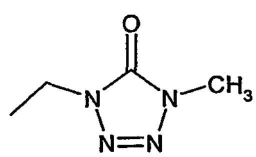 | $CH_3$ | |
| 82 | 0 | (2) Cl | (4) Cl | (3) 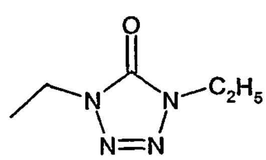 | $CH_3$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 83 | 0 | (2) Cl | (4) Cl | (3) 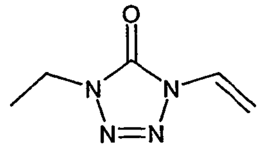 | $CH_3$ | |
| 84 | 0 | (2) Cl | (4) Cl | (3) 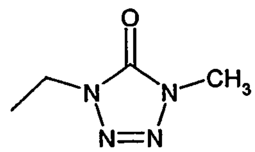 | $C_2H_5$ | |
| 85 | 0 | (2) Cl | (4) Cl | (3) 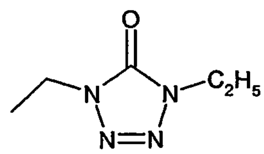 | $C_2H_5$ | |
| 86 | 0 | (2) Cl | (4) Cl | (3) 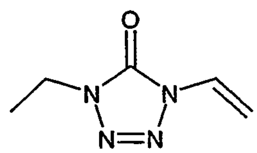 | $C_2H_5$ | |
| 87 | 0 | (2) Cl | (4) $SO_2CH_3$ | (3) 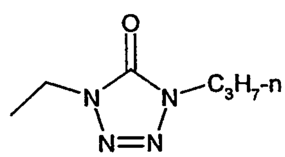 | $C_6H_5$ | |
| 88 | 0 | (2) Cl | (4) $SO_2CH_3$ | (3) 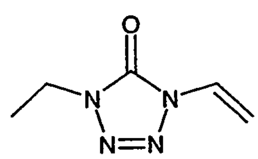 | $C_6H_5$ | |
| 89 | 0 | (2) Cl | (4) $SO_2CH_3$ | (3) 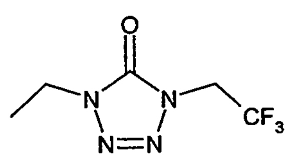 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 90 | 0 | (2) Cl | (4) $SO_2CH_3$ | (3) 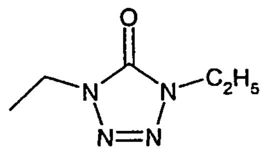 | $CH_3$ | |
| 91 | 0 | (2) Cl | (4) $SO_2CH_3$ | (3) 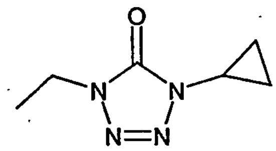 | $CH_3$ | |
| 92 | 0 | (2) Cl | (4) $SO_2CH_3$ | (3) 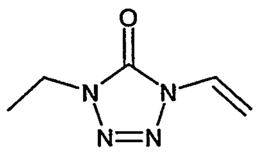 | $CH_3$ | |
| 93 | 0 | (2) Cl | (4) $SO_2CH_3$ | (3) 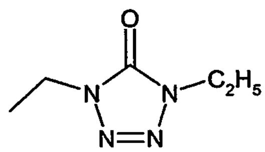 | $C_2H_5$ | |
| 94 | 0 | (2) Cl | (4) $SO_2CH_3$ | (3) 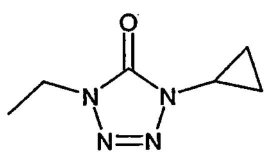 | $C_2H_5$ | |
| 95 | 0 | (2) Cl | (4) $SO_2CH_3$ | (3) 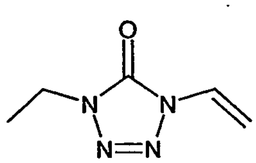 | $C_2H_5$ | |
| 96 | 0 | (2) Br | (4) Br | (3) 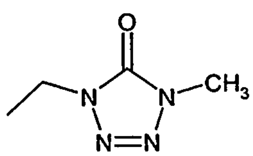 | $C_6H_5$ | Fp.:94°C |

(fortgesetzt)

| Bsp.-Nr | n | (Position) $R^3$ | (Position) $R^4$ | (Position) -A-Z | Y | Physikal. Daten |
|---|---|---|---|---|---|---|
| 97 | 0 | (2) Br | (4) Br | (3) 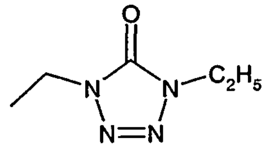 | $C_6H_5$ | |
| 98 | 0 | (2) Br | (4) Br | (3) 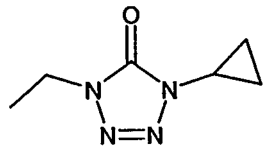 | $C_6H_5$ | Fp.:86°C |
| 99 | 0 | (2) Br | (4) Br | (3) 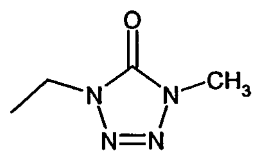 | $CH_3$ | |
| 100 | 0 | (2) Br | (4) Br | (3) 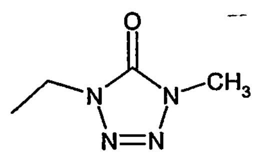 | $C_2H_5$ | |
| 101 | 0 | (2) $CH_3$ | (4) $SO_2CH_3$ | (3) 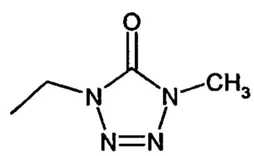 | $C_6H_5$ | |
| 102 | 0 | (2) $CH_3$ | (4) $SO_2CH_3$ | (3) 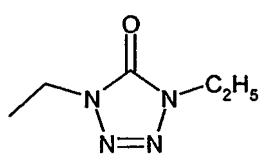 | $C_6H_5$ | |
| 103 | 0 | (2) $CH_3$ | (4) $SO_2CH_3$ | (3) 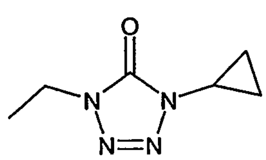 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 104 | 0 | (2) $CH_3$ | (4) $SO_2CH_3$ | (3) 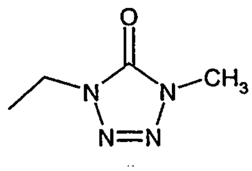 | $CH_3$ | |
| 105 | 0 | (2) $CH_3$ | (4) $SO_2CH_3$ | (3) 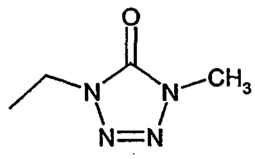 | $C_2H_5$ | |
| 106 | 0 | (2) $CH_3$ | (4) $SO_2CH_3$ | (3) 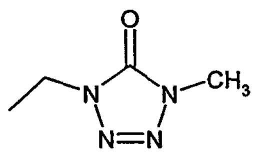 | $C_6H_5$ | |
| 107 | 0 | (2) $OCH_3$ | (4) Cl | (3) 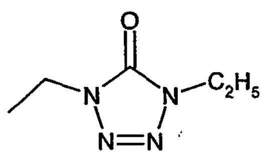 | $C_6H_5$ | |
| 108 | 0 | (2) $OCH_3$ | (4) Cl | (3) 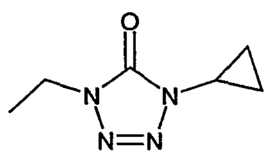 | $C_6H_5$ | |
| 109 | 0 | (2) $OCH_3$ | (4) Cl | (3) 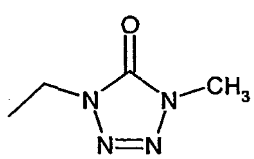 | $CH_3$ | |
| 110 | 0 | (2) $OCH_3$ | (4) Cl | (3) 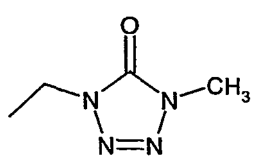 | $C_2H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 111 | 0 | (2) Br | - | (4) 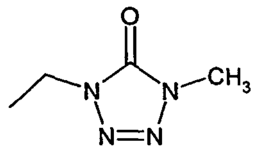 | $C_6H_5$ | |
| 112 | 0 | (2) I | - | (4) 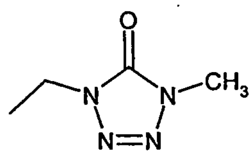 | $C_6H_5$ | |
| 113 | 0 | (2) $CF_3$ | - | (4) 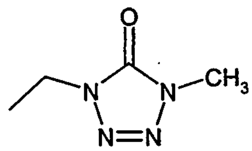 | $C_6H_5$ | |
| 114 | 0 | (2) CN | - | (4) 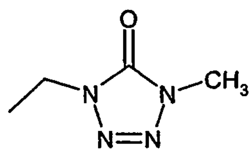 | $C_6H_5$ | |
| 115 | 0 | (2) $NO_2$ | - | (4) 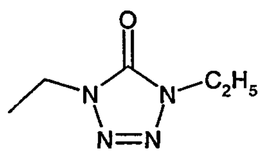 | $C_6H_5$ | |
| 116 | 0 | (2) $NO_2$ | - | (4) 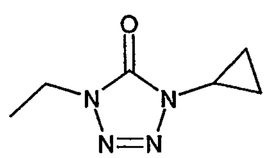 | $C_6H_5$ | |
| 117 | 0 | (2) $NO_2$ | - | (4) 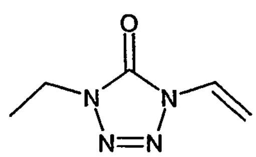 | $C_6H_5$ | |

(fortgesetzt)

| Bsp.-Nr | n | (Position) $R^3$ | (Position) $R^4$ | (Position) -A-Z | Y | Physikal. Daten |
|---|---|---|---|---|---|---|
| 118 | 0 | (2) $NO_2$ | - | (4) 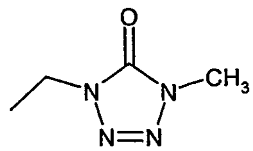 | $CH_3$ | |
| 119 | 0 | (2) $NO_2$ | - | (4) 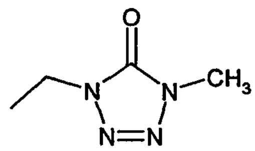 | $C_2H_5$ | |
| 120 | 0 | (2) Cl | (4) Cl | (3) 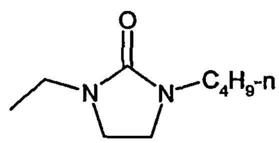 | $C_6H_5$ | Fp.:154°C logP = 4,02 [a)] |
| 121 | 0 | (2) Cl | (4) Cl | (3) 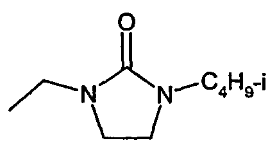 | $C_6H_5$ | Fp.: 156°C logP = 3,98 [a)] |
| 122 | 0 | (2) Cl | (4) $SO_2CH_3$ | (3) 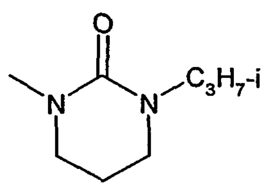 | $C_6H_5$ | logP = 3,11 [a)] |

Die Bestimmung der in Tabelle 1 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.

(a) Eluenten für die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit [a)] markiert.

(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit [b)] markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

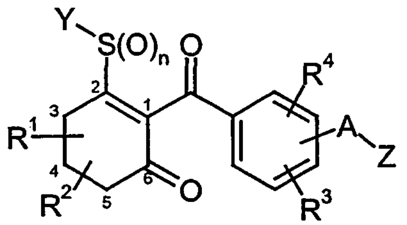

(I)

Tabelle 2: Weitere Beispiele für die Verbindungen der Formel (I)

| Bsp.-Nr. | n | (Position) $R^1$, $R^2$ | (Position) $R^3$ | (Position) $R^4$ | (Position) -A-Z | Y | Physikal. Daten |
|---|---|---|---|---|---|---|---|
| 123 | 0 | (4) $CH_3$, (4) $CH_3$ | (2) Cl | (4) Cl | (3) 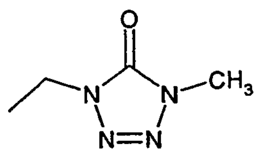 | $C_6H_5$ | Fp.: 77˚C |
| 124 | 0 | (5) $CH_3$, (5) $CH_3$ | (2) Cl | (4) Cl | (3) 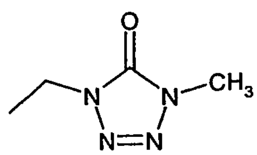 | $C_6H_5$ | Fp.: 85˚C |
| 125 | 0 | (4) $CH_3$, (4) $CH_3$ | (2) Cl | (4) $SO_2CH_3$ | (3) 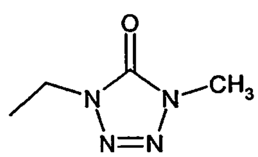 | $C_6H_5$ | Fp.: 99˚C |
| 126 | 0 | (5) $CH_3$, (5) $CH_3$ | (2) Cl | (4) $SO_2CH_3$ | (3) 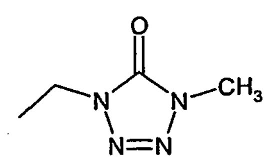 | $C_6H_5$ | Fp.: 104˚C |
| 127 | 0 | (3) $-CH_2CH_2-$ (5) | (2) Cl | (4) Cl | (3) 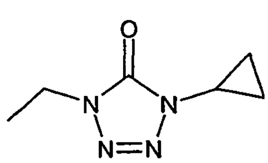 | $C_6H_5$ | Fp.: 84˚C |

(fortgesetzt)

| Bsp.-Nr. | n | (Position) $R^1$, $R^2$ | (Position) $R^3$ | (Position) $R^4$ | (Position) -A-Z | Y | Physikal. Daten |
|---|---|---|---|---|---|---|---|
| 128 | 0 | (3) $-CH_2CH_2-$ (5) | (4) Br | - | (2) 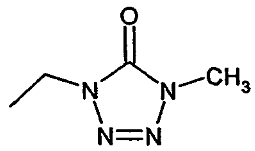 | $C_6H_5$ | Fp.: 186°C |
| 129 | 0 | (3) $-CH_2CH_2-$ (5) | (4) $CF_3$ | - | (2) 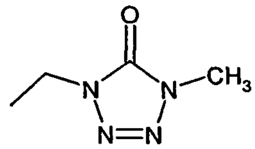 | $C_6H_5$ | Fp.: 197°C |
| 130 | 0 | (3) $-CH_2CH_2-$ (5) | (2) $NO_2$ | - | (4) 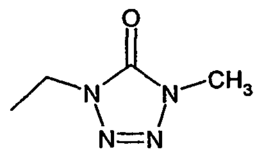 | $C_6H_5$ | Fp.:84°C |
| 131 | 0 | (3) $-CH_2CH_2-$ (5) | (2) Cl | (4) Cl | (3) 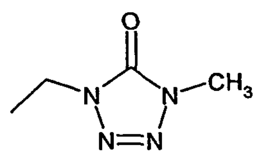 | $C_6H_5$ | |
| 132 | 0 | (3) $-CH_2CH_2-$ (5) | (2) Cl | (4) Cl | (3) 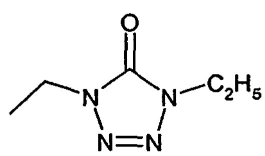 | $C_6H_5$ | |
| 133 | 0 | (3) $-CH_2CH_2-$ (5) | (2) Cl | (4) Cl | (3) 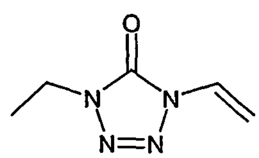 | $C_6H_5$ | |
| 134 | 0 | (3) $-CH_2CH_2-$ (5) | (2) Cl | (4) $SO_2CH_3$ | (3) 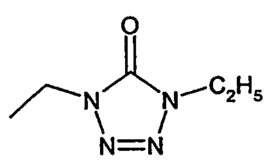 | $C_6H_5$ | |

(fortgesetzt)

| Bsp.-Nr. | n | (Position) $R^1$, $R^2$ | (Position) $R^3$ | (Position) $R^4$ | (Position) -A-Z | Y | Physikal. Daten |
|---|---|---|---|---|---|---|---|
| 135 | 0 | (3) $-CH_2CH_2-$ (5) | (2) Cl | (4) $SO_2CH_3$ | (3) 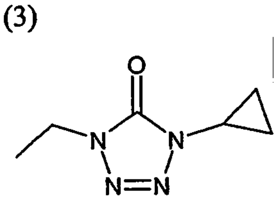 | $C_6H_5$ | |
| 136 | 0 | (3) $-CH_2CH_2-$ (5) | (2) Cl | (4) $SO_2CH_3$ | (3) 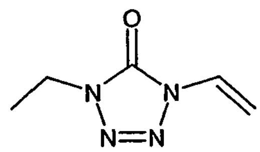 | $C_6H_5$ | |
| 137 | 0 | (3) $-CH_2CH_2-$ (5) | (2) Cl | (4) $SO_2CH_3$ | (3) 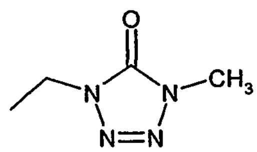 | $CH_3$ | |
| 138 | 0 | (3) $-CH_2CH_2-$ (5) | (2) Cl | (4) $SO_2CH_3$ | (3) 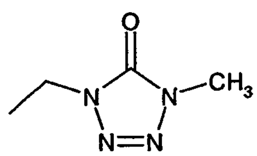 | $C_2H_5$ | |
| 139 | 0 | (3) $-CH_2CH_2-$ (5) | (2) Br | (4) Br | (3) 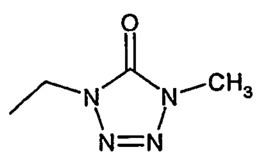 | $C_6H_5$ | |
| 140 | 0 | (3) $-CH_2CH_2-$ (5) | (2) Br | (4) Br | (3) 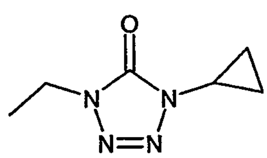 | $C_6H_5$ | |
| 141 | 0 | (3) $-CH_2CH_2-$ (5) | (2) $CH_3$ | (4) $SO_2CH_3$ | (3) 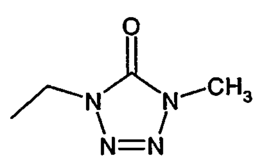 | $C_6H_5$ | |

(fortgesetzt)

| Bsp.-Nr. | n | (Position) $R^1$, $R^2$ | (Position) $R^3$ | (Position) $R^4$ | (Position) -A-Z | Y | Physikal. Daten |
|---|---|---|---|---|---|---|---|
| 142 | 0 | (3) $-CH_2CH_2-$ (5) | (2) $CH_3$ | (4) $SO_2CH_3$ | (3) 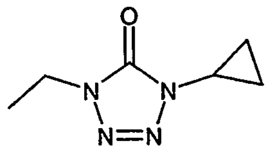 | $C_6H_5$ | |
| 143 | 0 | (3) $-CH_2CH_2-$ (5) | (2) $OCH_3$ | (4) Cl | (3) 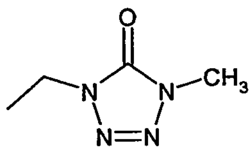 | $C_6H_5$ | |
| 144 | 0 | (3) $-CH_2CH_2-$ (5) | (2) $OCH_3$ | (4) Cl | (3) 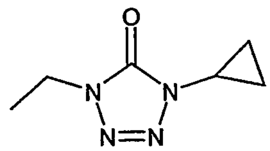 | $C_6H_5$ | |

**Anwendungsbeispiele**

**Beispiel A**

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden so mit der Wirkstoffzubereitung besprüht, dass die jeweils gewünschte Wirkstoffmenge pro Flächeneinheit ausgebracht wird. Die Wirkstoffkonzentration in der Spritzbrühe wird so gewählt, dass in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt beispielsweise die Verbindung gemäß Herstellungsbeispiel 16 bei zum Teil guter Verträglichkeit gegenüber Kulturpflanzen, wie z.B. Mais, starke Wirkung gegen Unkräuter.

**Beispiel B**

**Post-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.
Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.
Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigen beispielsweise die Verbindungen gemäß Herstellungsbeispiel 1, 2, 5, 16, 17, 18, 19 und 20, bei teilweise guter Verträglichkeit gegenüber Kulturpflanzen, wie z.B. Mais und Weizen, starke Wirkung gegen Unkräuter.

## Patentansprüche

**1.** Substituierte Benzoylcyclohexenone der Formel (I),

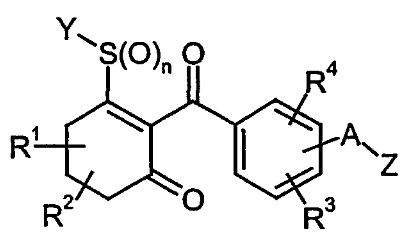

(I)

in welcher

n für die Zahlen 0, 1 oder 2 steht,
A für eine Einfachbindung oder für Alkandiyl (Alkylen) mit 1 bis 6 Kohlenstoffatomen steht,
$R^1$ für Wasserstoff, Phenyl oder gegebenenfalls durch Cyano, Halogen, $C_1$-$C_4$-Alkoxy oder $C_1$-$C_4$-Alkylthio substituiertes Alkyl mit 1 bis 6 Kohlenstoffatomen steht,
$R^2$ für Wasserstoff oder gegebenenfalls durch Cyano, Halogen, $C_1$-$C_4$-Alkoxy oder $C_1$-$C_4$-Alkylthio substituiertes Alkyl mit 1 bis 6 Kohlenstoffatomen steht, oder zusammen mit $R^1$ für Alkandiyl (Alkylen) mit 1 bis 6 Kohlenstoffatomen steht, oder zusammen mit $R^1$ - in diesem Fall an das gleiche Kohlenstoffatom gebunden - und dem Kohlenstoffatom, an welches $R^1$ und $R^2$ dann gebunden sind, für eine (C=O)-Gruppierung steht,
$R^3$ für Wasserstoff, Nitro, Cyano, Carboxy, Carbamoyl, Thiocarbamoyl, Halogen, oder für jeweils gegebenenfalls durch Cyano, Halogen oder $C_1$-$C_4$-Alkoxy substituiertes Alkyl, Alkoxy, Alkylthio, Alkylsulfinyl, Alkylsulfonyl, Alkylamino, Dialkylamino oder Dialkylaminosulfonyl mit jeweils 1 bis 6 Kohlenstoffatomen in den Alkylgruppen steht,
$R^4$ für Wasserstoff, Nitro, Cyano, Carboxy, Carbamoyl, Thiocarbamoyl, Halogen, oder für jeweils gegebenenfalls durch Cyano, Halogen oder $C_1$-$C_4$-Alkoxy substituiertes Alkyl, Alkoxy, Alkylthio, Alkylsulfinyl, Alkylsulfonyl,

Alkylamino, Dialkylamino oder Dialkylaminosulfonyl mit jeweils 1 bis 6 Kohlenstoffatomen in den Alkylgruppen steht, und

Y für Wasserstoff, für gegebenenfalls durch Cyano, Halogen oder $C_1$-$C_4$-Alkoxy substituiertes Alkyl mit 1 bis 10 Kohlenstoffatomen, für jeweils gegebenenfalls durch Cyano, Halogen, $C_1$-$C_4$-Alkyl-carbonyl oder $C_1$-$C_4$-Alkoxy-carbonyl substituiertes Alkenyl oder Alkinyl mit jeweils 2 bis 10 Kohlenstoffatomen, oder für jeweils gegebenenfalls durch Nitro, Amino, Cyano, Carboxy, Carbamoyl, Thiocarbamoyl, Halogen, $C_1$-$C_4$-Alkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl, $C_1$-$C_4$-Halogenalkylsulfonyl, $C_1$-$C_4$-Alkylcarbonyl, $C_1$-$C_4$-Alkoxy-carbonyl, $C_1$-$C_4$-Alkyl-carbonyl-amino, $C_1$-$C_4$-Alkoxy-carbonyl-amino, $C_1$-$C_4$-Alkyl-sulfonyl-amino substituiertes Aryl oder Arylalkyl mit jeweils 6 oder 10 Kohlenstoffatomen in den Arylgruppen und gegebenenfalls 1 bis 4 Kohlenstoffatomen im Alkylteil steht, und

Z für eine gegebenenfalls substituierte 4- bis 12-gliedrige, gesättigte oder ungesättigte, monocyclische oder bicyclische, heterocyclische Gruppierung steht, welche 1 bis 4 Heteroatome (bis zu 4 Stickstoffatome und gegebenenfalls - alternativ oder additiv - ein Sauerstoffatom oder ein Schwefelatom, oder eine SO-Gruppierung oder eine $SO_2$-Gruppierung) enthält, und welche zusätzlich ein bis drei Oxo-Gruppen (C=O) und/oder Thioxo-Gruppen (C=S) als Bestandteile des Heterocyclus enthält,

- einschließlich aller möglichen tautomeren und gegebenenfalls möglichen stereoisomeren Formen der Verbindungen der allgemeinen Formel (I), und der möglichen Salze oder Metall-koordinierten Derivate der Verbindungen der Formel (I).

**2.** Verbindungen gemäß Anspruch 1, **dadurch gekennzeichnet, dass**

n für die Zahlen 0 oder 2 steht,

A für eine Einfachbindung oder für Alkandiyl (Alkylen) mit 1 bis 4 Kohlenstoffatomen steht,

$R^1$ für Wasserstoff, Phenyl oder gegebenenfalls durch Cyano, Halogen, $C_1$-$C_4$-Alkoxy oder $C_1$-$C_4$-Alkylthio substituiertes Alkyl mit 1 bis 5 Kohlenstoffatomen steht,

$R^2$ für Wasserstoff oder gegebenenfalls durch Cyano, Halogen, $C_1$-$C_4$-Alkoxy oder $C_1$-$C_4$-Alkylthio substituiertes Alkyl mit 1 bis 5 Kohlenstoffatomen, oder zusammen mit $R^1$ für Alkandiyl (Alkylen) mit 1 bis 5 Kohlenstoffatomen steht,

$R^3$ für Wasserstoff, Nitro, Cyano, Carboxy, Carbamoyl, Thiocarbamoyl, Halogen, oder für jeweils gegebenenfalls durch Cyano, Halogen oder $C_1$-$C_4$-Alkoxy substituiertes Alkyl, Alkoxy, Alkylthio, Alkylsulfinyl, Alkylsulfonyl, Alkylamino, Dialkylamino oder Dialkylaminosulfonyl mit jeweils 1 bis 5 Kohlenstoffatomen in den Alkylgruppen steht,

$R^4$ für Wasserstoff, Nitro, Cyano, Carboxy, Carbamoyl, Thiocarbamoyl, Halogen, oder für jeweils gegebenenfalls durch Cyano, Halogen oder $C_1$-$C_4$-Alkoxy substituiertes Alkyl, Alkoxy, Alkylthio, Alkylsulfinyl, Alkylsulfonyl, Alkylamino, Dialkylamino oder Dialkylaminosulfonyl mit jeweils 1 bis 5 Kohlenstoffatomen in den Alkylgruppen steht,

Y für Wasserstoff, für gegebenenfalls durch Cyano, Halogen oder $C_1$-$C_4$-Alkoxy substituiertes Alkyl mit 1 bis 6 Kohlenstoffatomen, für jeweils gegebenenfalls durch Cyano, Halogen, $C_1$-$C_4$-Alkyl-carbonyl oder $C_1$-$C_4$-Alkoxy-carbonyl substituiertes Alkenyl oder Alkinyl mit jeweils 2 bis 6 Kohlenstoffatomen, oder für jeweils gegebenenfalls durch Nitro, Amino, Cyano, Carboxy, Carbamoyl, Thiocarbamoyl, Halogen, $C_1$-$C_4$-Alkyl, $C_1$-$C_4$-Halogenalkyl, $C_1$-$C_4$-Alkoxy-$C_1$-$C_4$-alkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl, $C_1$-$C_4$-Halogenalkylsulfonyl, $C_1$-$C_4$-Alkylcarbonyl, $C_1$-$C_4$-Alkoxy-carbonyl, $C_1$-$C_4$-Alkyl-carbonyl-amino, $C_1$-$C_4$-Alkoxycarbonyl-amino, $C_1$-$C_4$-Alkyl-sulfonyl-amino substituiertes Aryl oder Arylalkyl mit jeweils 6 oder 10 Kohlenstoffatomen in den Arylgruppen und gegebenenfalls 1 bis 4 Kohlenstoffatomen im Alkylteil steht,

Z für eine der nachstehenden heterocyclischen Gruppierungen steht,

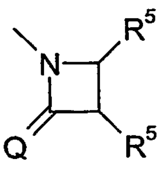 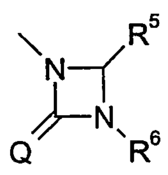 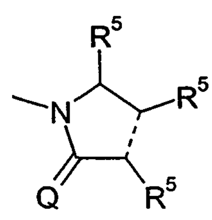 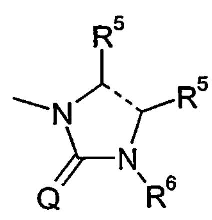

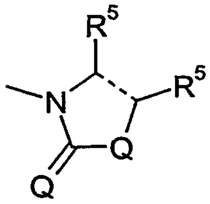
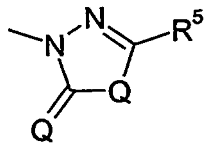
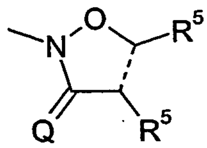
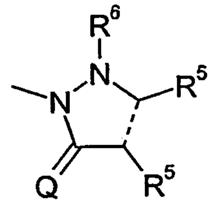

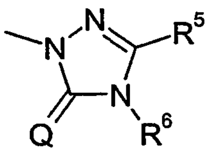
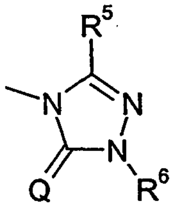
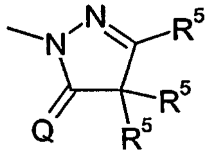
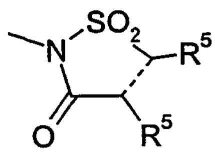

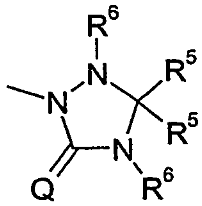
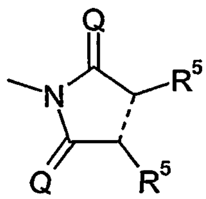
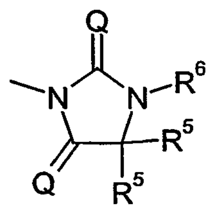
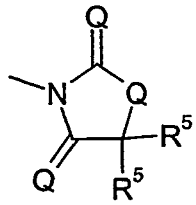

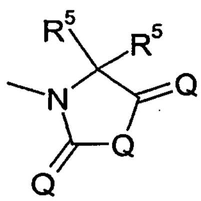
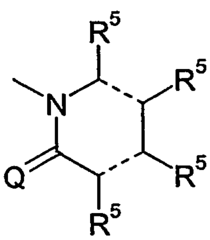
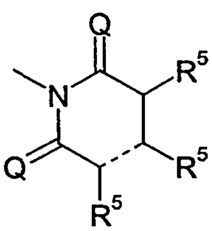
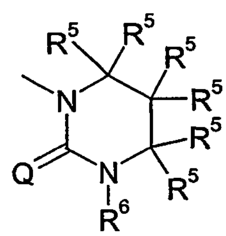

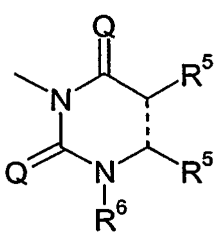
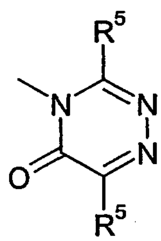
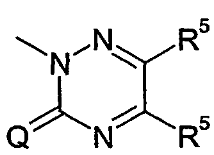
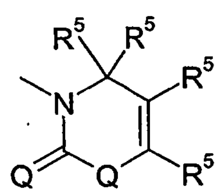

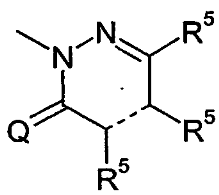
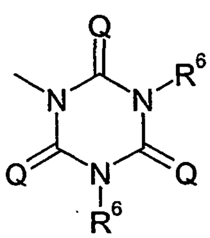
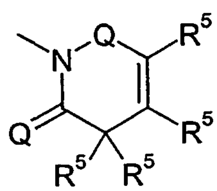
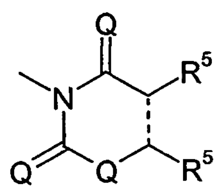

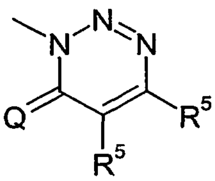 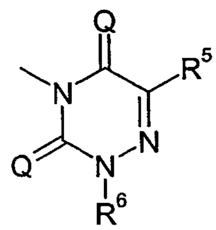 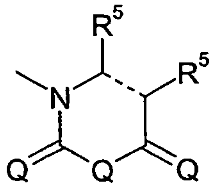 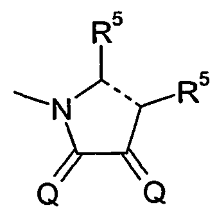

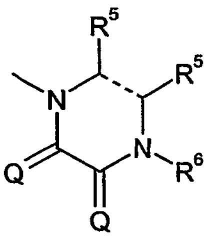 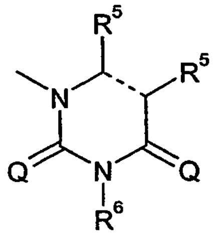 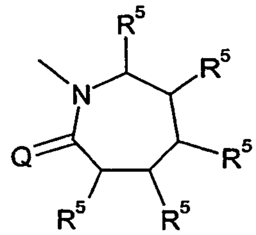 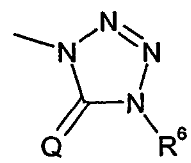

worin jeweils die gestrichelt gezeichnete Bindung eine Einfachbindung oder eine Doppelbindung ist, und worin jeder Heterocyclus bevorzugt nur zwei Substituenten der Definition $R^5$ und/oder $R^6$ in beliebiger Kombination trägt,

Q für Sauerstoff oder Schwefel steht,
$R^5$ für Wasserstoff, Hydroxy, Mercapto, Cyano, Halogen, für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl, Alkylcarbonyl, Alkoxy, Alkoxycarbonyl, Alkylthio, Alkylsulfinyl oder Alkylsulfonyl mit jeweils bis zu 6 Kohlenstoffatomen in den Alkylgruppen, für jeweils gegebenenfalls durch Halogen substituiertes Alkylamino oder Dialkylamino mit jeweils bis zu 6 Kohlenstoffatomen in den Alkylgruppen, für jeweils gegebenenfalls durch Halogen substituiertes Alkenyl, Alkinyl, Alkenyloxy, Alkenylthio, Alkinylthio oder Alkenylamino mit jeweils bis zu 6 Kohlenstoffatomen in den Alkenyl- bzw. Alkinylgruppen, für jeweils gegebenenfalls durch Halogen substituiertes Cycloalkyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in den Cycloalkylgruppen und gegebenenfalls bis zu 4 Kohlenstoffatomen im Alkylteil, oder für jeweils gegebenenfalls durch Halogen, $C_1$-$C_4$-Akyl oder $C_1$-$C_4$-Akoxy substituiertes Phenyl, Phenyloxy, Phenylthio, Phenylamino, Benzyl, Benzyloxy, Benzylthio oder Benzylamino steht, für Pyrrolidino, Piperidino oder Morpholino steht, oder - für den Fall, dass zwei benachbarte Reste $R^5$ und $R^5$ sich an einer Doppelbindung befinden - auch zusammen mit dem benachbarten Rest $R^5$ für eine Benzogruppierung steht, und
$R^6$ für Wasserstoff, Hydroxy, Amino, Alkylidenamino mit bis zu 4 Kohlenstoffatomen, für jeweils gegebenenfalls durch Halogen oder $C_1$-$C_4$-Alkoxy substituiertes Alkyl, Alkoxy, Alkylamino, Dialkylamino oder Alkanoylamino mit jeweils bis zu 6 Kohlenstoffatomen in den Alkylgruppen, für jeweils gegebenenfalls durch Halogen substituiertes Akenyl, Alkinyl oder Alkenyloxy mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl oder Cycloalkylamino mit jeweils 3 bis 6 Kohlenstoffatomen in den Cycloalkylgruppen und gegebenenfalls bis zu 3 Kohlenstoffatomen im Alkylteil, oder für jeweils gegebenenfalls durch Halogen, $C_1$-$C_4$-Alkyl oder $C_1$-$C_4$-Alkoxy substituiertes Phenyl oder Benzyl steht, oder zusammen mit einem benachbarten Rest $R^5$ oder $R^6$ für gegebenenfalls durch Halogen oder $C_1$-$C_4$-Alkyl substituiertes Alkandiyl mit 3 bis 5 Kohlenstoffatomen steht,

wobei die einzelnen Reste $R^5$ und $R^6$ - soweit mehrere davon an gleiche heterocyclische Gruppierungen gebunden sind, gleiche oder verschiedene Bedeutungen im Rahmen der obigen Definition haben können.

**3.** Verbindungen gemäß Anspruch 1 oder 2, **dadurch gekennzeichnet, dass**

A für eine Einfachbindung oder für Methylen, Ethyliden (Ethan-1,1-diyl) oder Dimethylen (Ethan-1,2-diyl) steht,
$R^1$ für Wasserstoff, Phenyl oder jeweils gegebenenfalls durch Cyano, Fluor, Chlor, Brom, Methoxy, Ethoxy, n-

oder i-Propoxy, Methylthio, Ethylthio, n- oder i-Propylthio substituiertes Methyl, Ethyl, n- oder i-Propyl, n-, i- oder s-Butyl steht,
$R^2$ für Wasserstoff oder jeweils gegebenenfalls durch Cyano, Fluor, Chlor, Brom, Methoxy, Ethoxy, n- oder i-Propoxy, Methylthio, Ethylthio, n- oder i-Propylthio substituiertes Methyl, Ethyl, n- oder i-Propyl, n-, i- oder s-Butyl, oder zusammen mit $R^1$ für Methylen, Ethyliden (Ethan-1,1-diyl), Dimethylen (Ethan-1,2-diyl), Propyliden (Propan-1,1-diyl) oder Trimethylen (Propan-1,3-diyl) steht,
$R^3$ für Wasserstoff, Nitro, Cyano, Carboxy, Carbamoyl, Thiocarbamoyl, Fluor, Chlor, Brom, Iod, oder für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Dimethylaminosulfonyl oder Diethylaminosulfonyl steht,
$R^4$ für Wasserstoff, Nitro, Cyano, Carboxy, Carbamoyl, Thiocarbamoyl, Fluor, Chlor, Brom, Iod, oder für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Dimethylaminosulfonyl oder Diethylaminosulfonyl steht,
Y für Wasserstoff, für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, für jeweils gegebenenfalls durch Cyano, Fluor, Chlor, Brom, Acetyl, Propionyl, n- oder i-Butyroyl, Methoxycarbonyl, Ethoxycarbonyl, n- oder i-Propoxycarbonyl substituiertes Ethenyl, Propenyl, Butenyl, Pentenyl, Ethinyl, Propinyl oder Butinyl, oder für jeweils gegebenenfalls durch Nitro, Amino, Cyano, Carboxy, Carbamoyl, Thiocarbamoyl, Fluor, Chlor, Brom, Iod, Methyl, Ethyl, n- oder i-Propyl, n-, i-, s- oder t-Butyl, Trifluormethyl, Methoxymethyl, Ethoxymethyl, Methoxyethyl, Ethoxyethyl, Methoxy, Ethoxy, n- oder i-Propoxy, n-, i-, s- oder t-Butoxy, Difluormethoxy, Trifluormethoxy, Methylthio, Ethylthio, n- oder i-Propylthio, n-, i-, s- oder t-Butylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl, Acetyl, Propionyl, n- oder i-Butyroyl, Methoxycarbonyl, Ethoxycarbonyl, n-oder i-Propoxycarbonyl, Acetylamino, Propionylamino, Methoxycarbonylamino, Ethoxycarbonylamino, Methylsulfonylamino, Ethylsulfonylamino, n- oder i-Propylsulfonylamino substituiertes Phenyl, Naphthyl, Benzyl oder Phenylethyl steht,
Z für eine der nachstehenden heterocyclischen Gruppierungen

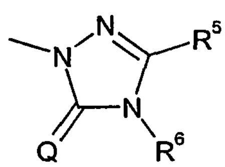
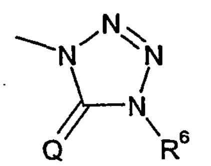

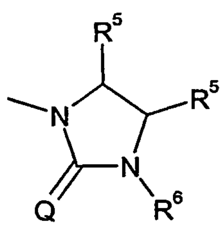
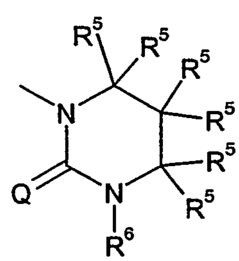

steht,

Q für Sauerstoff steht,
$R^5$ für Wasserstoff, Hydroxy, Mercapto, Cyano, Fluor, Chlor, Brom, Iod, für jeweils gegebenenfalls durch Fluor, Chlor, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl,

Ethylsulfonyl, n- oder i-Propylsulfonyl substituiertes Methyl, Ethyl, n- oder i-Propyl, n-, i-, s-oder t-Butyl, Methoxy, Ethoxy, n- oder i-Propoxy, n-, i-, s-oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, n-, i-, s- oder t-Butylamino, Dimethylamino, Diethylamino, Di-n-propylamino oder Di-i-propylamino, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenylthio, Propenylamino oder Butenylamino, für jeweils gegebenenfalls durch Fluor und/oder Chlor substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutyhnethylamino, Cyclopentylmethylamino oder Cyclohexylmethylamino, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl, Phenyloxy, Phenylthio, Phenylamino, Benzyl, Benzyloxy, Benzylthio oder Benzylamino steht, oder - für den Fall, dass zwei benachbarte Reste $R^5$ und $R^5$ sich an einer Doppelbindung befinden - zusammen mit dem benachbarten Rest $R^5$ auch für eine Benzogruppierung steht, und
$R^6$ für Wasserstoff, Hydroxy, Amino, für jeweils gegebenenfalls durch Fluor und/oder Chlor, Methoxy oder Ethoxy substituiertes Methyl, Ethyl, n- oder i-Propyl, n-, i- oder s-Butyl, Methoxy, Ethoxy, n- oder i-Propoxy, Methylamino, Ethylamino oder Dimethylamino, für jeweils gegebenenfalls durch Fluor und/oder Chlor substituiertes Ethenyl, Propenyl, Ethinyl, Propinyl oder Propenyloxy, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl oder Benzyl steht, oder zusammen mit einem benachbarten Rest $R^5$ oder $R^6$ für jeweils gegebenenfalls durch Methyl und/oder Ethyl substituiertes Propan-1,3-diyl (Trimethylen), Butan-1,4-diyl (Tetramethylen) oder Pentan-1-5-diyl (Pentamethylen) steht.

**4.** Verbindungen gemäß einem der Ansprüche 1 bis 3, **dadurch gekennzeichnet, dass**

A für eine Einfachbindung oder für Methylen steht,
$R^1$ für Wasserstoff, Phenyl oder jeweils gegebenenfalls durch Fluor oder Chlor substituiertes Methyl, Ethyl, n- oder i-Propyl steht,
$R^2$ für Wasserstoff oder jeweils gegebenenfalls durch Fluor oder Chlor substituiertes Methyl, Ethyl, n- oder i-Propyl, oder zusammen mit $R^1$ für Methylen oder Dimethylen steht,
$R^3$ für Wasserstoff, Nitro, Cyano, Fluor, Chlor, Brom, Iod, oder für jeweils gegebenenfalls durch Fluor, Chlor, Methoxy oder Ethoxy substituiertes Methyl, Ethyl, n- oder i-Propyl, Methoxy, Ethoxy, Methylthio, Ethylthio, Methylsulfinyl, Ethylsulfinyl, Methylsulfonyl, Ethylsulfonyl, Methylamino, Ethylamino, Dimethylamino oder Dimethylaminosulfonyl steht,
$R^4$ für Wasserstoff, Nitro, Cyano, Fluor, Chlor, Brom, Iod, oder für jeweils gegebenenfalls durch Fluor, Chlor, Methoxy oder Ethoxy substituiertes Methyl, Ethyl, n- oder i-Propyl, Methoxy, Ethoxy, Methylthio, Ethylthio, Methylsulfinyl, Ethylsulfinyl, Methylsulfonyl, Ethylsulfonyl, Methylamino, Ethylamino, Dimethylamino oder Dimethylaminosulfonyl steht,
Y für Wasserstoff, für jeweils gegebenenfalls durch Cyano, Fluor, Chlor, Brom, Methoxy, Ethoxy, n- oder i-Propoxy substituiertes Methyl, Ethyl, n- oder i-Propyl, n-, i-, s- oder t-Butyl, für jeweils gegebenenfalls durch Fluor, Chlor oder Brom substituiertes Propenyl, Butenyl, Pentenyl, Propinyl oder Butinyl, oder für jeweils gegebenenfalls durch Nitro, Amino, Cyano, Fluor, Chlor, Brom, Methyl, Ethyl, n- oder i-Propyl, n-, i-, s- oder t-Butyl, Trifluormethyl, Methoxymethyl, Ethoxymethyl, Methoxyethyl, Ethoxyethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethylsulfonyl, Trifluormethylsulfonyl, Acetyl, Propionyl, Methoxycarbonyl, Ethoxycarbonyl, Acetylamino, Propionylamino, Methoxycarbonylamino, Ethoxycarbonylamino, Methylsulfonylamino oder Ethylsulfonylamino substituiertes Phenyl oder Benzyl steht,
$R^5$ für Wasserstoff, Fluor, Chlor, Brom, für jeweils gegebenenfalls durch Fluor, Chlor, Methoxy, Ethoxy, n- oder i-Propoxy, Methylthio, Ethylthio, Methylsulfinyl, Ethylsulfinyl, Methylsulfonyl, Ethylsulfonyl substituiertes Methyl, Ethyl, n- oder i-Propyl, n-, i- oder s-Butyl, Methoxy, Ethoxy, n- oder i-Propoxy, n-, i- oder s-Butoxy, Methylthio, Ethylthio, n- oder i-Propylthio, n-, i- oder s-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, n-, i-

oder s-Butylamino, Dimethylamino oder Diethylamino, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio oder für jeweils gegebenenfalls durch Fluor und/oder Chlor substituiertes Cyclopropyl, Cyclopropyloxy, Cyclopropylthio, Cyclopropylamino, Cyclopropylmethyl, Cyclopropylmethoxy, Cyclopropylmethylthio oder Cyclopropylmethylamino steht, und

$R^6$ für Wasserstoff, für Amino, für jeweils gegebenenfalls durch Fluor und/oder Chlor, Methoxy oder Ethoxy substituiertes Methyl, Ethyl, Methoxy, Ethoxy, Methylamino oder Ethylamino, für Dimethylamino, oder für jeweils gegebenenfalls durch Fluor und/oder Chlor substituiertes Cyclopropyl, Cyclopropylmethyl; Phenyl oder Benzyl, oder zusammen mit einem benachbarten Rest $R^5$ oder $R^6$ für jeweils gegebenenfalls durch Methyl und/oder Ethyl substituiertes Propan-1,3-diyl (Trimethylen), Butan-1,4-diyl (Tetramethylen) oder Pentan-1,5-diyl (Pentamethylen) steht.

5. Verbindungen gemäß einem der Ansprüche 1 bis 4, **dadurch gekennzeichnet, dass**

$R^1$ für Wasserstoff steht,
$R^2$ für Wasserstoff steht,
$R^3$ für Chlor, Trifluormethyl oder Methoxy steht,
$R^4$ für Chlor, Trifluormethyl oder Methylsulfonyl steht,
$R^5$ für Wasserstoff, Methyl oder Ethyl steht, und
$R^6$ für Wasserstoff, Methyl, Ethyl, n- oder i-Propyl, n-, i-, s- oder t-Butyl, Cyclopropyl, Cyclopropylmethyl, Cyclopentyl, Cyclopentylmethyl, Cyclohexyl, Cyclohexylmethyl, Phenyl oder Benzyl steht.

6. Verbindungen gemäß einem der Ansprüche 1 bis 5, **dadurch gekennzeichnet, dass**

$R^5$ für Wasserstoff steht, und
$R^6$ für Methyl, Ethyl, n- oder i-Propyl, n-, i-, s- oder t-Butyl, Cyclopropyl, Cyclopropylmethyl oder Phenyl steht.

7. Verbindungen gemäß einem der Ansprüche 1 bis 6, **dadurch gekennzeichnet, dass**

n für die Zahl 0 steht.

8. Verbindungen gemäß einem der Ansprüche 1 bis 7, **dadurch gekennzeichnet, dass**

Z für eine der nachstehenden Gruppierungen steht

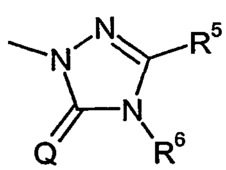 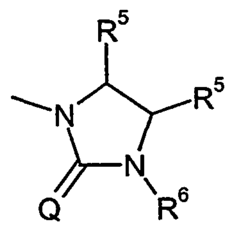 .

9. Verbindungen gemäß einem der Ansprüche 1 bis 8, **dadurch gekennzeichnet, dass**

Q für Sauerstoff steht.

10. Verbindungen gemäß einem der Ansprüche 1 bis 9, **dadurch gekennzeichnet, dass**

Y für Wasserstoff, für jeweils gegebenenfalls durch Fluor, Chlor oder Methoxy substituiertes Methyl, Ethyl, n- oder i-Propyl, n-, i-, s- oder t-Butyl, für jeweils gegebenenfalls durch Fluor oder Chlor substituiertes Propenyl, Butenyl, Pentenyl oder für gegebenenfalls durch Amino, Cyano, Fluor, Chlor, Methyl, Ethyl, Trifluormethyl, Methoxy oder Ethoxy substituiertes Phenyl, steht.

11. Verbindungen der Formel (IA)

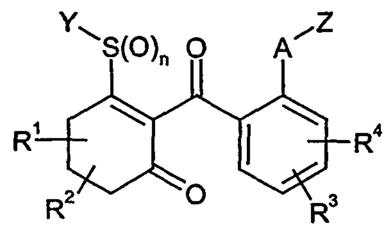

(IA)

worin n, A, $R^1$, $R^2$, $R^3$, $R^4$, Y und Z gemäß einem der Ansprüche 1 bis 9 definiert sind.

**12.** Verbindungen der Formel (IB)

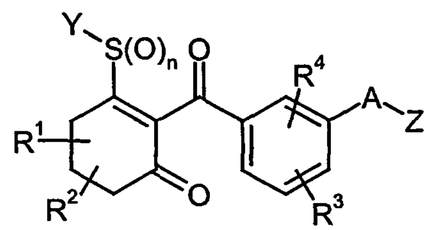

(IB)

worin n, A, $R^1$, $R^2$, $R^3$, $R^4$, Y und Z gemäß einem der Ansprüche 1 bis 9 definiert sind.

**13.** Verbindungen der Formel (IC)

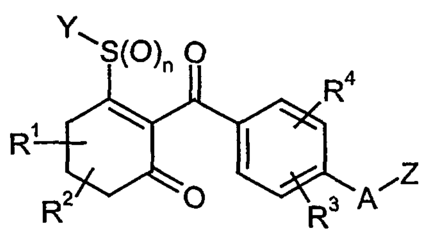

(IC)

worin n, A, $R^1$, $R^2$, $R^3$, $R^4$, Y und Z gemäß einem der Ansprüche 1 bis 9 definiert sind.

**14.** Natrium-, Kalium-, Magnesium-, Calcium-, Ammonium-, $C_1$-$C_4$-Alkylammonium-, Di-($C_1$-$C_4$-alkyl)-ammonium-, Tri-($C_1$-$C_4$-alkyl)-ammonium-, Tetra-($C_1$-$C_4$-alkyl)-ammonium, Tri-($C_1$-$C_4$-alkyl)-sulfonium-, $C_5$- oder $C_6$-Cycloalkyl-ammonium- und Di-($C_1$-$C_2$-alkyl)-benzyl-ammoni-um-Salze von Verbindungen der Formel (I) gemäß einem der Ansprüche 1 bis 13, sowie deren Komplexverbindungen mit Metallen wie Kupfer, Eisen, Kobalt, Nickel.

**15.** Verfahren zum Herstellen von Verbindungen gemäß einem der Ansprüche 1 bis 12, **dadurch gekennzeichnet, dass** man Verbindungen der Formel (II)

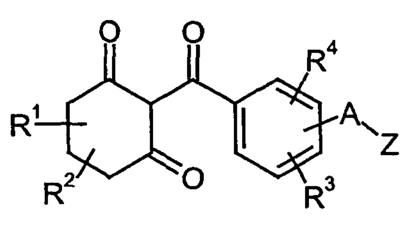

(II)

in welcher
A, $R^1$, $R^2$, $R^3$, $R^4$ und Z gemäß einem der Ansprüche 1 bis 9 definiert sind,
mit einem Halogenierungsmittel, gegebenenfalls in Gegenwart eines Reaktionshilfsmittel und gegebenenfalls in Gegenwart eines Verdünnungsmittel, umsetzt und die hierbei (in der ersten Umsetzungsstufe) erhaltenen Halogencyclohexenone der Formel (III)

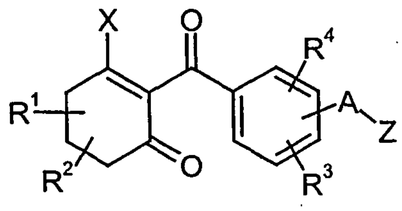

(III)

in welcher
A, $R^1$, $R^2$, $R^3$, $R^4$ und Z gemäß einem der Ansprüche 1 bis 9 definiert sind und

X für Halogen steht,
- nach Zwischenisolierung oder ohne Zwischenisolierung ("in situ") -

in einer zweiten Umsetzungsstufe mit Mercaptoverbindungen der Formel (TV)

HS-Y (IV)

in welcher

Y die oben angegebene Bedeutung hat,

gegebenenfalls in Gegenwart eines Reaktionshilfsmittel und gegebenenfalls in Gegenwart eines Verdünnungsmittel, umsetzt,
und gegebenenfalls im Anschluss daran an den so erhaltenen Verbindungen der Formel (I) im Rahmen der Substituentendefinition auf übliche Weise elektrophile oder nucleophile Substitutionsreaktionen bzw. Oxidations- oder Reduktionsreaktionen durchführt oder die Verbindungen der Formel (I) auf übliche Weise in Salze überführt.

**16.** Herbizide Mittel, **gekennzeichnet durch** den Gehalt mindestens einer Verbindung gemäß einem der Ansprüche 1 bis 12 und den üblichen Streckmitteln.

**17.** Verwendung von mindestens einer Verbindung gemäß einem der Ansprüche 1 bis 12 zur Bekämpfung von unerwünschten Pflanzen.

**18.** Verfahren zum Bekämpfen von unerwünschten Pflanzen, **dadurch gekennzeichnet, dass** man mindestens eine Verbindung gemäß einem der Ansprüche 1 bis 13 oder ein Mittel gemäß Anspruch 16 auf die unerwünschten Pflanzen und/oder ihren Lebensraum einwirken lässt.

## Claims

**1.** Substituted benzoylcyclohexenones of the formula (I),

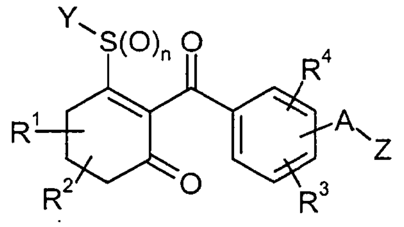

(I)

in which

n represents the number 0, 1 or 2,
A represents a single bond or represents alkanediyl (alkylene) having 1 to 6 carbon atoms,
$R^1$ represents hydrogen, phenyl or optionally cyano-, halogen-, $C_1$-$C_4$-alkoxy- or $C_1$-$C_4$-alkylthio-substituted alkyl having 1 to 6 carbon atoms,
$R^2$ represents hydrogen or optionally cyano-, halogen-, $C_1$-$C_4$-alkoxy- or $C_1$-$C_4$-alkylthio-substituted alkyl having 1 to 6 carbon atoms, or together with $R^1$ represents alkanediyl (alkylene) having 1 to 6 carbon atoms, or together with $R^1$ - in this case attached to the same carbon atom - and the carbon atom to which $R^1$ and $R^2$ are attached in this case represents a (C=O) grouping,
$R^3$ represents hydrogen, nitro, cyano, carboxyl, carbamoyl, thiocarbamoyl, halogen, or represents in each case optionally cyano-, halogen- or $C_1$-$C_4$-alkoxy-substituted alkyl, alkoxy, alkylthio, alkylsulphinyl, alkylsulphonyl, alkylamino, dialkylamino or dialkylaminosulphonyl having in each case 1 to 6 carbon atoms in the alkyl groups,
$R^4$ represents hydrogen, nitro, cyano, carboxyl, carbamoyl, thiocarbamoyl, halogen, or represents in each case optionally cyano-, halogen- or $C_1$-$C_4$-alkoxy-substituted alkyl, alkoxy, alkylthio, alkylsulphinyl, alkylsulphonyl, alkylamino, dialkylamino or dialkylaminosulphonyl having in each case 1 to 6 carbon atoms in the alkyl groups, and
Y represents hydrogen, represents optionally cyano-, halogen- or $C_1$-$C_4$-alkoxy-substituted alkyl having 1 to 10 carbon atoms, represents in each case optionally cyano-, halogen-, $C_1$-$C_4$-alkyl-carbonyl- or $C_1$-$C_4$-alkoxy-carbonyl-substituted alkenyl or alkinyl having in each case 2 to 10 carbon atoms, or represents in each case optionally nitro-, amino-, cyano-, carboxyl-, carbamoyl-, thiocarbamoyl-, halogen-, $C_1$-$C_4$-alkyl-, $C_1$-$C_4$-halogenoalkyl-, $C_1$-$C_4$-alkoxy-$C_1$-$C_4$-alkyl-, $C_1$-$C_4$-alkoxy-, $C_1$-$C_4$-halogenoalkoxy-, $C_1$-$C_4$-alkylthio-, $C_1$-$C_4$-halogenoalkylthio-,$C_1$-$C_4$-alkylsulphinyl-,$C_1$-$C_4$-halogenoalkylsulphinyl-,$C_1$-$C_4$-alkylsulphonyl-,$C_1$-$C_4$-halogenoalkylsulphonyl-, $C_1$-$C_4$-alkylcarbonyl-, $C_1$-$C_4$-alkoxy-carbonyl-, $C_1$-$C_4$-alkyl-carbonyl-amino-, $C_1$-$C_4$-alkoxy-carbonyl-amino-, $C_1$-$C_4$-alkyl-sulphonyl-amino-substituted aryl or arylalkyl having in each case 6 or 10 carbon atoms in the aryl groups and optionally 1 to 4 carbon atoms in the alkyl moiety, and
Z represents an optionally substituted 4- to 12-membered saturated or unsaturated, monocyclic or bicyclic, heterocyclic grouping which contains 1 to 4 hetero atoms (up to 4 nitrogen atoms and optionally - alternatively or additionally - one oxygen atom or one sulphur atom, or one SO grouping or one $SO_2$ grouping), and which contains additionally one to three oxo groups (C=O) and/or thioxo groups (C=S) as components of the heterocycle,
- including all possible tautomeric and, if appropriate, possible stereoisomeric forms of the compounds of the general formula (I), and the possible salts or metal-coordinated derivatives of the compounds of the formula (I).

**2.** Compounds according to Claim 1, **characterized in that**

n represents the number 0 or 2,
A represents a single bond or represents alkanediyl (alkylene) having 1 to 4 carbon atoms,
$R^1$ represents hydrogen, phenyl or optionally cyano-, halogen-, $C_1$-$C_4$-alkoxy- or $C_1$-$C_4$-alkylthio-substituted alkyl having 1 to 5 carbon atoms,
$R^2$ represents hydrogen or optionally cyano-, halogen-, $C_1$-$C_4$-alkoxy- or $C_1$-$C_4$-alkylthio-substituted alkyl having 1 to 5 carbon atoms, or together with $R^1$ represents alkanediyl (alkylene) having 1 to 5 carbon atoms,
$R^3$ represents hydrogen, nitro, cyano, carboxyl, carbamoyl, thiocarbamoyl, halogen, or represents in each case optionally cyano-, halogen- or $C_1$-$C_4$-alkoxy-substituted alkyl, alkoxy, alkylthio, alkylsulphinyl, alkylsulphonyl, alkylamino, dialkylamino or dialkylaminosulphonyl having in each case 1 to 5 carbon atoms in the alkyl groups,
$R^4$ represents hydrogen, nitro, cyano, carboxyl, carbamoyl, thiocarbamoyl, halogen, or represents in each case optionally cyano-, halogen- or $C_1$-$C_4$-alkoxy-substituted alkyl, alkoxy, alkylthio, alkylsulphinyl, alkylsulphonyl, alkylamino, dialkylamino or dialkylaminosulphonyl having in each case 1 to 5 carbon atoms in the alkyl groups,

Y represents hydrogen, represents optionally cyano-, halogen- or $C_1$-$C_4$-alkoxy-substituted alkyl having 1 to 6 carbon atoms, represents in each case optionally cyano-, halogen-, $C_1$-$C_4$-alkyl-carbonyl- or $C_1$-$C_4$-alkoxy-carbonyl-substituted alkenyl or alkinyl having in each case 2 to 6 carbon atoms, or represents in each case optionally nitro-, amino-, cyano-, carboxyl-, carbamoyl-, thiocarbamoyl-, halogen-, $C_1$-$C_4$-alkyl-, $C_1$-$C_4$-halogenoalkyl-, $C_1$-$C_4$-alkoxy-$C_1$-$C_4$-alkyl-, $C_1$-$C_4$-alkoxy-, $C_1$-$C_4$-halogenoalkoxy-, $C_1$-$C_4$-alkylthio, $C_1$-$C_4$-halogenoalkylthio-,$C_1$-$C_4$-alkylsulphinyl-,$C_1$-$C_4$-halogepoalkylsulphinyl-,$C_1$-$C_4$-alkylsulphonyl-,$C_1$-$C_4$-halogenoalkylsulphonyl-, $C_1$-$C_4$-alkylcarbonyl-, $C_1$-$C_4$-alkoxy-carbonyl-, $C_1$-$C_4$-alkyl-carbonyl-amino-, $C_1$-$C_4$-alkoxy-carbonyl-amino-, $C_1$-$C_4$-alkyl-sulphonyl-amino-substituted aryl or arylalkyl having in each case 6 or 10 carbon atoms in the aryl groups and optionally 1 to 4 carbon atoms in the alkyl moiety,

Z represents one of the heterocyclic groupings below

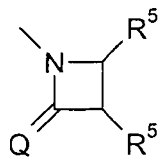 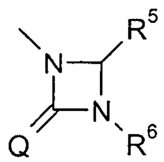 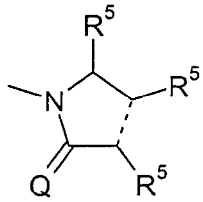 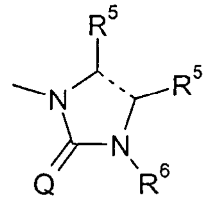

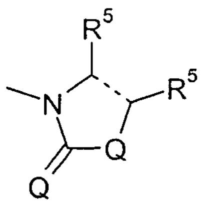 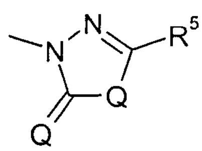 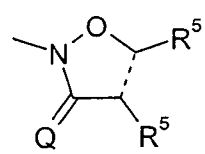 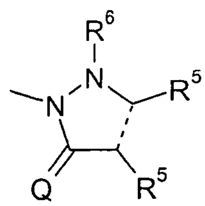

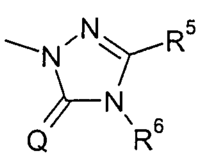 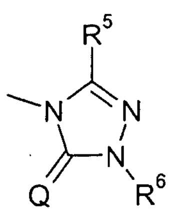 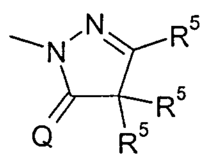 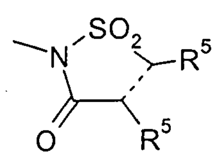

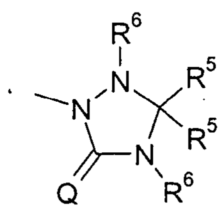 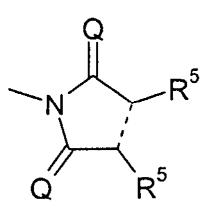 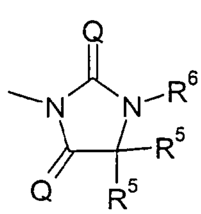 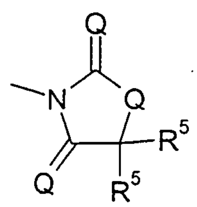

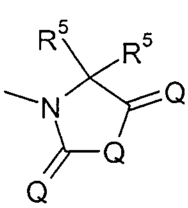 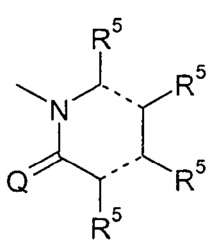 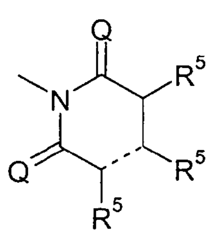 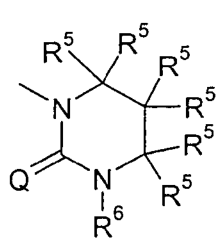

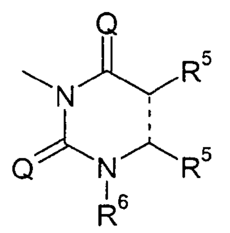 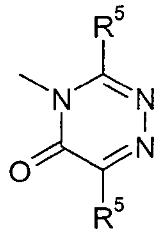 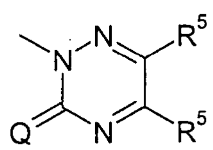 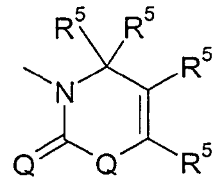

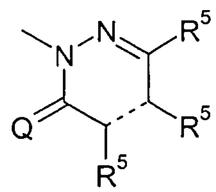 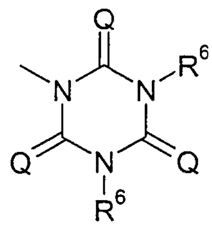 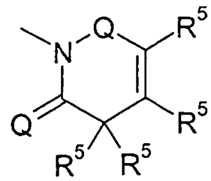 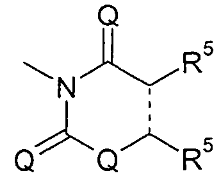

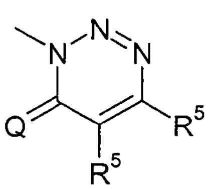 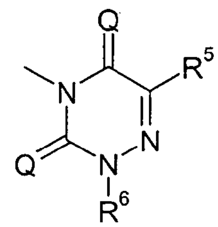 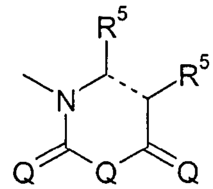 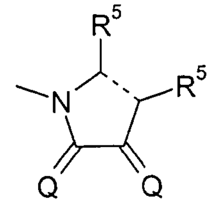

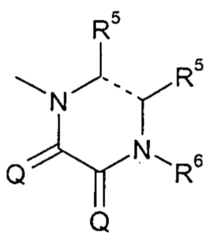 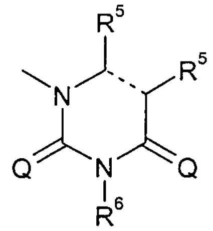 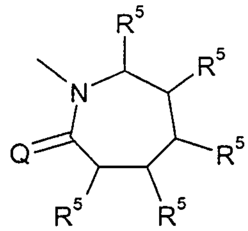 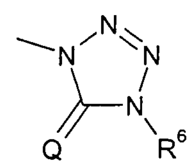

in which the bond drawn broken in each case denotes a single bond or a double bond, and in which each heterocycle preferably only carries two substituents of the definition $R^5$ and/or $R^6$ in any combination,

Q represents oxygen or sulphur,

$R^5$ represents hydrogen, hydroxyl, mercapto, cyano, halogen, represents in each case optionally cyano-, halogen-, $C_1$-$C_4$-alkoxy-, $C_1$-$C_4$-alkylthio-, $C_1$-$C_4$-alkylsulphinyl- or $C_1$-$C_4$-alkylsulphonyl-substituted alkyl, alkylcarbonyl, alkoxy, alkoxycarbonyl, alkylthio, alkylsulphinyl or alkylsulphonyl having in each case up to 6 carbon atoms in the alkyl groups, represents in each case optionally halogen-substituted alkylamino or dialkylamino having in each case up to 6 carbon atoms in the alkyl groups, represents in each case optionally halogensubstituted alkenyl, alkinyl, alkenyloxy, alkenylthio, alkinylthio or alkenylamino having in each case up to 6 carbon atoms in the alkenyl or alkinyl groups, represents in each case optionally halogensubstituted cycloalkyl, cycloalkyloxy, cycloalkylthio, cycloalkylamino, cycloalkylalkyl, cycloalkylalkoxy, cycloalkylalkylthio or cycloalkylalkylamino having in each case 3 to 6 carbon atoms in the cycloalkyl groups and optionally up to 4 carbon atoms in the alkyl moiety, or represents in each case optionally halogen-, $C_1$-$C_4$-alkyl- or $C_1$-$C_4$-alkoxy-substituted phenyl, phenyloxy, phenylthio, phenylamino, benzyl, benzyloxy, benzylthio or benzylamino, represents pyrrolidino, piperidino or morpholino, or - if two adjacent radicals $R^5$ and $R^5$ are located at a double bond - also together with the adjacent radical $R^5$ represents a benzo grouping,

and

$R^6$ represents hydrogen, hydroxyl, amino, alkylideneamino having up to 4 carbon atoms, represents in each case optionally halogen- or $C_1$-$C_4$-alkoxy-substituted alkyl, alkoxy, alkylamino, dialkylamino or alkanoylamino having in each case up to 6 carbon atoms in the alkyl groups, represents in each case optionally halogen-substituted alkenyl, alkinyl or alkenyloxy having in each case up to 6 carbon atoms in the alkenyl or alkinyl groups, represents in each case optionally halogen-substituted cycloalkyl, cycloalkylalkyl or cycloalkylamino having in each case 3 to 6 carbon atoms in the cycloalkyl groups and optionally up to 3 carbon atoms in the alkyl moiety, or represents in each case optionally halogen-, $C_1$-$C_4$-alkyl- or $C_1$-$C_4$-alkoxy-substituted phenyl or benzyl, or together with an adjacent radical $R^5$ or $R^6$ represents optionally halogen- or $C_1$-$C_4$-alkyl-substituted alkanediyl having 3 to 5 carbon atoms,

where the individual radicals $R^5$ and $R^6$ - if two or more of them are attached to the same heterocyclic groupings, may have identical or different meanings in the context of the above definition.

**3.** Compounds according to Claim 1 or 2, **characterized in that**

A represents a single bond or represents methylene, ethylidene (ethane-1,1-diyl) or dimethylene (ethane-1,2-diyl),

$R^1$ represents hydrogen, phenyl or in each case optionally cyano-, fluorine-, chlorine-, bromine-, methoxy-, ethoxy-, n- or i-propoxy-, methylthio-, ethylthio-, n- or i-propylthio-substituted methyl, ethyl, n- or i-propyl, n-, i- or s-butyl,

$R^2$ represents hydrogen or represents in each case optionally cyano-, fluorine-, chlorine-, bromine-, methoxy-, ethoxy-, n- or i-propoxy-, methylthio-, ethylthio-, n- or i-propylthio-substituted methyl, ethyl, n- or 1-propyl, n-, i- or s-butyl, or together with $R^1$ represents methylene, ethylidene (ethane-1,1-diyl), dimethylene (ethane-1,2-diyl), propylidene (propane-1,1-diyl) or trimethylene (propane-1,3-diyl),

$R^3$ represents hydrogen, nitro, cyano, carboxyl, carbamoyl, thiocarbamoyl, fluorine, chlorine, bromine, iodine, or represents in each case optionally cyano-, fluorine-, chlorine-, bromine-, methoxy-, ethoxy-, n- or i-propoxy-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, dimethylamino, diethylamino, dimethylaminosulphonyl or diethylaminosulphonyl,

$R^4$ represents hydrogen, nitro, cyano, carboxyl, carbamoyl, thiocarbamoyl, fluorine, chlorine, bromine, iodine, or represents in each case optionally cyano-, fluorine-, chlorine-, bromine-, methoxy-, ethoxy-, n- or i-propoxy-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, dimethylamino, diethylamino, dimethylaminosulphonyl or diethylaminosulphonyl,

Y represents hydrogen, represents in each case optionally cyano-, fluorine-, chlorine-, bromine-, methoxy-, ethoxy-, n- or i-propoxysubstituted methyl, ethyl, n- or i-propyl, n-, i-, s- or t-butyl, represents in each case optionally cyano-, fluorine-, chlorine-, bromine-, acetyl-, propionyl-, n- or i-butyroyl-, methoxycarbonyl-, ethoxycarbonyl-, n- or i-propoxycarbonyl-substituted ethenyl, propenyl, butenyl, pentenyl, ethinyl, propinyl or butinyl, or represents in each case optionally nitro-, amino-, cyano-, carboxyl-, carbamoyl-, thiocarbamoyl-, fluorine-, chlorine-, bromine-, iodine-, methyl-, ethyl-, n- or i-propyl-, n-, i-, s- or t-butyl-, trifluoromethyl-, methoxymethyl-, ethoxymethyl-, methoxyethyl-, ethoxyethyl-, methoxy-, ethoxy-, n- or i-propoxy-, n-, i-, s- or t-butoxy-, difluoromethoxy-, trifluoromethoxy-, methylthio-, ethylthio-, n- or i-propylthio-, n-, i-, s- or t-butylthio-, difluoromethylthio-, trifluoromethylthio-, methylsulphinyl-, ethylsulphinyl-, trifluoromethylsulphinyl-, methylsulphonyl-, ethylsulphonyl-, trifluoromethylsulphonyl-, acetyl-, propionyl-, n- or i-butyroyl-, methoxycarbonyl-, ethoxycarbonyl-, n- or i-propoxycarbonyl-, acetylamino-, propionylamino-, methoxycarbonylamino-, ethoxycarbonylamino-, methylsulphonylamino-, ethylsulphonylamino-, n- or i-propylsulphonylamino-substituted phenyl, naphthyl, benzyl or phenylethyl,

Z represents one of the heterocyclic groupings below

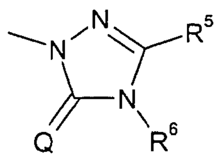
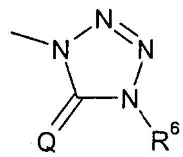

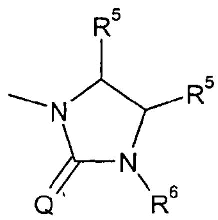
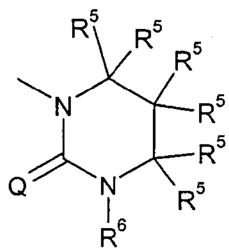

Q represents oxygen,

$R^5$ represents hydrogen, hydroxyl, mercapto, cyano, fluorine, chlorine, bromine, iodine, represents in each case optionally fluorine-, chlorine-, methoxy-, ethoxy-, n- or i-propoxy-, n-, i-, s- or t-butoxy-, methylthio-, ethylthio-, n- or i-propylthio-, n-, i-, s- or t-butylthio-, methylsulphinyl-, ethylsulphinyl-, n- or i-propylsulphinyl-, methylsulphonyl-, ethylsulphonyl-, n- or i-propylsulphonyl-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, represents methylamino, ethylamino, n- or i-propylamino, n-, i-, s- or t-butylamino, dimethylamino, diethylamino, di-n-propylamino or di-i-propylamino, represents in each case optionally fluorine- and/or chlorine-substituted ethenyl, propenyl, butenyl, ethinyl, propinyl, butinyl, propenyloxy, butenyloxy, propenylthio, butenylthio, propenylamino or butenylamino, represents in each case optionally fluorine- and/or chlorine-substituted cyclopropyl, cyclobutyl, cyclopentyl, cyclohexyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentylmethylthio, cyclohexylmethylthio, cyclopropylmethylamino, cyclobutylmethylamino, cyclopentylmethylamino or cyclohexylmethylamino, or represents in each case optionally fluorine-, chlorine-, methyl-, ethyl-, n- or i-propyl-, n-, i-, s- or t-butyl-, methoxy-, ethoxy-, n- or i-propoxy-substituted phenyl, phenyloxy, phenylthio, phenylamino, benzyl, benzyloxy, benzylthio or benzylamino, or - in the case that two adjacent radicals $R^5$ and $R^5$ are located at a double bond - together with the adjacent radical $R^5$ also represents a benzo grouping, and

$R^6$ represents hydrogen, hydroxyl, amino, represents in each case optionally fluorine- and/or chlorine-, methoxy- or ethoxysubstituted methyl, ethyl, n- or i-propyl, n-, i- or s-butyl, methoxy, ethoxy, n- or i-propoxy, methylamino, ethylamino or dimethylamino, represents in each case optionally fluorine- and/or chlorine-substituted ethenyl, propenyl, ethinyl, propinyl or propenyloxy, represents in each case optionally fluorine- and/or chlorine-substituted cyclopropyl, cyclobutyl, cyclopentyl, cyclohexyl, cyclopropylmethyl, cyclobutylmethyl, cyclopentylmethyl or cyclohexylmethyl, or represents in each case optionally fluorine-, chlorine-, methyl-, ethyl-, n- or i-propyl-, n-, i-, s- or t-butyl-, methoxy-, ethoxy-, n- or i-propoxy-substituted phenyl or benzyl, or together with an adjacent radical $R^5$ or $R^6$ represents in each case optionally methyl- and/or ethyl-substituted propane-1,3-diyl (trimethylene), butane-1,4-diyl (tetramethylene) or pentane-1,5-diyl (pentamethylene).

**4.** Compounds according to any of Claims 1 to 3, **characterized in that**

A represents a single bond or represents methylene,

$R^1$ represents hydrogen, phenyl or in each case optionally fluorine- or chlorine-substituted methyl, ethyl, n- or i-propyl,

$R^2$ represents hydrogen or in each case optionally fluorine- or chlorine-substituted methyl, ethyl, n- or i-propyl, or together with $R^1$ represents methylene or dimethylene,

$R^3$ represents hydrogen, nitro, cyano, fluorine, chlorine, bromine, iodine, or represents in each case optionally fluorine-, chlorine-, methoxy- or ethoxy-substituted methyl, ethyl, n- or i-propyl, methoxy, ethoxy, methylthio, ethylthio, methylsulphinyl, ethylsulphinyl, methylsulphonyl, ethylsulphonyl, methylamino, ethylamino, dimethylamino or dimethylaminosulphonyl,

$R^4$ represents hydrogen, nitro, cyano, fluorine, chlorine, bromine, iodine, or represents in each case optionally fluorine-, chlorine-, methoxy- or ethoxy-substituted methyl, ethyl, n- or i-propyl, methoxy, ethoxy, methylthio, ethylthio, methylsulphinyl, ethylsulphinyl, methylsulphonyl, ethylsulphonyl, methylamino, ethylamino, dimethylamino or dimethylaminosulphonyl,

Y represents hydrogen, represents in each case optionally cyano-, fluorine-, chlorine-, bromine-, methoxy-, ethoxy-, n- or i-propoxy-substituted methyl, ethyl, n- or i-propyl, n-, i-, s- or t-butyl, represents in each case optionally fluorine-, chlorine- or bromine-substituted propenyl, butenyl, pentenyl, propinyl or butinyl, or represents in each case optionally nitro-, amino-, cyano-, fluorine-, chlorine-, bromine-, methyl-, ethyl-, n- or i-propyl-, n-, i-, s- or t-butyl-, trifluoromethyl-, methoxymethyl-, ethoxymethyl-, methoxyethyl-, ethoxyethyl-, methoxy-, ethoxy-, n- or i-propoxy-, difluoromethoxy-, trifluoromethoxy-, methylthio-, ethylthio-, n- or i-propylthio-, difluoromethylthio-, trifluoromethylthio-, methylsulphinyl-, ethylsulphinyl-, trifluoromethylsulphinyl-, methylsulphonyl, ethylsulphonyl-, trifluoromethylsulphonyl-, acetyl-, propionyl-, methoxycarbonyl-, ethoxycarbonyl-, acetylamino-, propionylamino-, methoxycarbonylamino-, ethoxycarbonylamino-, methylsulphonylamino- or ethylsulphonylamino-substituted phenyl or benzyl,

$R^5$ represents hydrogen, fluorine, chlorine, bromine, represents in each case optionally fluorine-, chlorine-, methoxy-, ethoxy-, n- or i-propoxy-, methylthio-, ethylthio-, methylsulphinyl-, ethylsulphinyl-, methylsulphonyl-, ethylsulphonyl-substituted methyl, ethyl, n- or i-propyl, n-, i- or s-butyl, methoxy, ethoxy, n- or i-propoxy, n-, i- or s-butoxy, methylthio, ethylthio, n- or i-propylthio, n-, i- or s-butylthio, methylsulphinyl, ethylsulphinyl, n- or i-propylsulphinyl, methylsulphonyl, ethylsulphonyl, n- or i-propylsulphonyl, represents methylamino, ethylamino, n- or i-propylamino, n-, i- or s-butylamino, dimethylamino or diethylamino, represents in each case optionally fluorine- and/or chlorine-substituted ethenyl, propenyl, butenyl, ethinyl, propinyl, butinyl, propenyloxy, butenyloxy, propenylthio or represents in each case optionally fluorine- and/or chlorine-substituted cyclopropyl, cyclopropyloxy, cyclopropylthio, cyclopropylamino, cyclopropylmethyl, cyclopropylmethoxy, cyclopropylmethylthio or cyclopropylmethylamino, and

$R^6$ represents hydrogen, represents amino, represents in each case optionally fluorine- and/or chlorine-, methoxy- or ethoxy-substituted methyl, ethyl, methoxy, ethoxy, methylamino or ethylamino, represents dimethylamino, or represents in each case optionally fluorine- and/or chlorine-substituted cyclopropyl, cyclopropylmethyl, phenyl or benzyl, or together with an adjacent radical $R^5$ or $R^6$ represents in each case optionally methyl- and/or ethyl-substituted propane-1,3-diyl (trimethylene), butane-1,4-diyl (tetramethylene) or pentane-1,5-diyl (pentamethylene).

**5.** Compounds according to any of Claims 1 to 4, **characterized in that**

$R^1$ represents hydrogen,
$R^2$ represents hydrogen,
$R^3$ represents chlorine, trifluoromethyl or methoxy,
$R^4$ represents chlorine, trifluoromethyl or methylsulphonyl,
$R^5$ represents hydrogen, methyl or ethyl, and
$R^6$ represents hydrogen, methyl, ethyl, n- or i-propyl, n-, i-, s- or t-butyl, cyclopropyl, cyclopropylmethyl, cyclopentyl, cyclopentylmethyl, cyclohexyl, cyclohexylmethyl, phenyl or benzyl.

**6.** Compounds according to any of Claims 1 to 5, **characterized in that**

$R^5$ represents hydrogen, and
$R^6$ represents methyl, ethyl, n- or i-propyl, n-, i-, s- or t-butyl, cyclopropyl, cyclopropylmethyl or phenyl.

**7.** Compounds according to any of Claims 1 to 6, **characterized in that**

n represents the number 0.

**8.** Compounds according to any of Claims 1 to 7, **characterized in that**

Z represents one of the groupings below

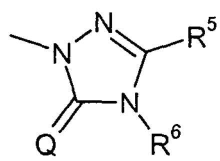 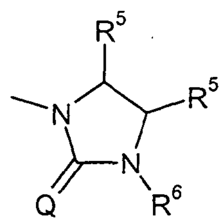.

**9.** Compounds according to any of Claims 1 to 8, **characterized in that**

Q represents oxygen.

**10.** Compounds according to any of Claims 1 to 9, **characterized in that**

Y represents hydrogen, represents in each case optionally fluorine-, chlorine- or methoxy-substituted methyl, ethyl, n- or i-propyl, n-, i-, s- or t-butyl, represents in each case optionally fluorine- or chlorine-substituted propenyl, butenyl, pentenyl or represents optionally amino-, cyano-, fluorine-, chlorine-, methyl-, ethyl-, trifluoromethyl-, methoxy- or ethoxy-substituted phenyl.

**11.** Compounds of the formula (IA)

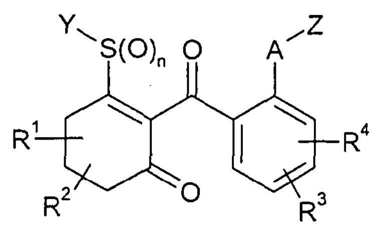 (IA)

in which n, A, $R^1$, $R^2$, $R^3$, $R^4$, Y and Z are as defined in any of Claims 1 to 9.

**12.** Compounds of the formula (IB)

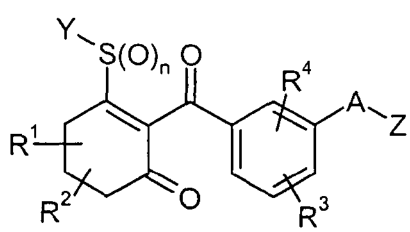 (IB)

in which n, A, $R^1$, $R^2$, $R^3$, $R^4$, Y and Z are as defined in any of Claims 1 to 9.

**13.** Compounds of the formula (IC)

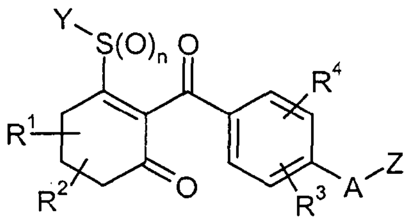

(IC)

in which n, A, $R^1$, $R^2$, $R^3$, $R^4$, Y and Z are as defined in any of Claims 1 to 9.

**14.** Sodium, potassium, magnesium, calcium, ammonium, $C_1$-$C_4$-alkylammonium, di-($C_1$-$C_4$-alkyl)-ammonium, tri-($C_1$-$C_4$-alkyl)-ammonium-, tetra-($C_1$-$C_4$-alkyl)-ammonium, tri-($C_1$-$C_4$-alkyl)-sulphonium, $C_5$- or $C_6$-cycloalkyl-ammonium and di-($C_1$-$C_2$-alkyl)-benzyl-ammonium salts of compounds of the formula (I) according to any of Claims 1 to 13, and their complex compounds with metals such as copper, iron, cobalt, nickel.

**15.** Process for preparing compounds according to any of Claims 1 to 12, **characterized in that** compounds of the formula (II)

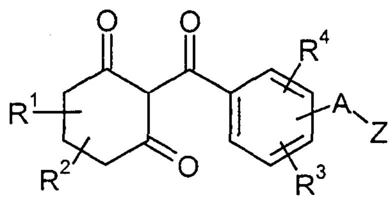

(II)

in which
A, $R^1$, $R^2$, $R^3$, $R^4$ and Z are as defined in any of Claims 1 to 9
are reacted with a halogenating agent, if appropriate in the presence of a reaction auxiliary and if appropriate in the presence of a diluent, and the halogenocyclohexenones obtained in this way (in the first reaction step), of the formula (III)

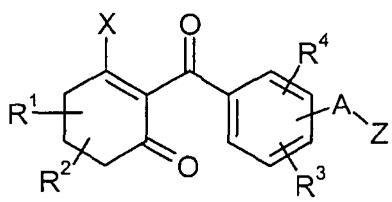

(III)

in which
A, $R^1$, $R^2$, $R^3$, $R^4$ and Z are as defined in any of Claims 1 to 9 and

X represents halogen
- after intermediate isolation or without intermediate isolation ("in situ") -

are reacted in a second reaction step with mercapto compounds of the formula (IV)

HS-Y (IV)

in which

Y is as defined above,

if appropriate in the presence of a reaction auxiliary and if appropriate in the presence of a diluent,
and, if appropriate, the compounds of the formula (I) obtained in this manner are subsequently subjected in a customary manner, within the scope of the definition of the substituents, to electrophilic or nucleophilic substitution reactions or oxidation or reduction reactions, or the compounds of the formula (I) are converted in a customary manner into salts.

**16.** Herbicidal compositions, **characterized in that** they comprise at least one compound according to any of Claims 1 to 12 and customary extenders.

**17.** Use of at least one compound according to any of Claims 1 to 12 for controlling undesirable plants.

**18.** Method for controlling undesirable plants, **characterized in that** at least one compound according to any of Claims 1 to 13 or a composition according to Claim 16 is allowed to act on the undesirable plants and/or their habitat.

## Revendications

**1.** Benzoylcyclohexénones substituées de formule (I),

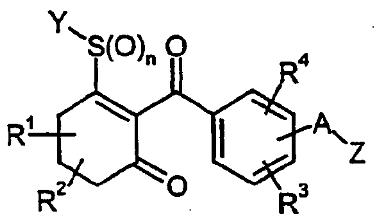

(I)

dans laquelle

n représente les nombres 0, 1 ou 2,
A représente une liaison simple ou un groupe alcanediyle (alkylène) contenant 1 à 6 atomes de carbone,
$R^1$ représente un atome d'hydrogène, un groupe phényle ou un groupe alkyle contenant 1 à 6 atomes de carbone éventuellement substitué par un groupe cyano, halogéno, alcoxy en $C_1$ à $C_4$ ou alkylthio en $C_1$ à $C_4$,
$R^2$ représente un atome d'hydrogène ou un groupe alkyle contenant 1 à 6 atomes de carbone éventuellement substitué par un groupe cyano, halogéno, alcoxy en $C_1$ à $C_4$ ou alkylthio en $C_1$ à $C_4$, ou, conjointement avec $R^1$, représente un groupe alcanediyle (alkylène) contenant 1 à 6 atomes de carbone, ou, conjointement avec $R^1$ - dans ce cas lié au même atome de carbone - et avec l'atome de carbone auquel $R^1$ et $R^2$ sont liés, un groupement (C=O),
$R^3$ représente un atome d'hydrogène, un groupe nitro, cyano, carboxy, carbamoyle, thiocarbamoyle, halogéno, ou un groupe alkyle, alcoxyle, alkylthio, alkylsulfinyle, alkylsulfonyle, alkylamino, dialkylamino ou dialkylaminosulfonyle, contenant chacun 1 à 6 atomes de carbone dans le groupe alkyle, éventuellement substitué par un groupe cyano, halogéno ou alcoxy en $C_1$ à $C_4$,
$R^4$ représente un atome d'hydrogène, un groupe nitro, cyano, carboxy, carbamoyle, thiocarbamoyle, halogéno, ou un groupe alkyle, alcoxyle, alkylthio, alkylsulfinyle, alkylsulfonyle, alkylamino, dialkylamino ou dialkylaminosulfonyle, contenant chacun 1 à 6 atomes de carbone dans le groupe alkyle, éventuellement substitué par un groupe cyano, halogéno ou alcoxy en $C_1$ à $C_4$, et
Y représente un atome d'hydrogène, un groupe alkyle contenant 1 à 10 atomes de carbone éventuellement substitué par un groupe cyano, halogéno ou alcoxy en $C_1$ à $C_4$, un groupe alcényle ou alcynyle contenant chacun 2 à 10 atomes de carbone éventuellement substitué par un groupe cyano, halogéno, (alkyle en $C_1$ à $C_4$)-carbonyle ou (alcoxy en $C_1$ à $C_4$)-carbonyle, ou un groupe aryle ou arylalkyle, contenant chacun 6 ou 10 atomes de carbone dans le groupe aryle et éventuellement 1 à 4 atomes de carbone dans la fraction alkyle, éventuellement substitué par un groupe nitro, amino, cyano, carboxy, carbamoyle, thiocarbamoyle, halogéno, alkyle en $C_1$ à $C_4$, halogéno- (alkyle en $C_1$ à $C_4$), (alcoxy en $C_1$ à $C_4$)-(alkyle en $C_1$ à $C_4$), alcoxy en $C_1$ à $C_4$, halogéno-(alkyle en $C_1$ à $C_4$), alkylthio en $C_1$ à $C_4$, halogéno-(alkylthio en $C_1$ à $C_4$), (alkyle en $C_1$ à $C_4$)-sulfinyle, halogéno-(alkyle en $C_1$ à $C_4$)-sulfinyle, (alkyle en $C_1$ à $C_4$)-sulfonyle, halogéno-(alkyle en $C_1$ à $C_4$)-sulfonyle, (alkyle en $C_1$ à $C_4$)-carbonyle, (alcoxy en $C_1$ à $C_4$)-carbonyle, (alkyle en $C_1$ à $C_4$)-carbonyl-amino, (alcoxy en

$C_1$ à $C_4$)-carbonyl-amino, (alkyle en $C_1$ à $C_4$)-sulfonyl-amino, et
Z représente un groupe hétérocyclique, monocyclique ou bicyclique, saturé ou insaturé, de 4 à 12 éléments, éventuellement substitué, qui contient 1 à 4 hétéroatomes (jusqu'à 4 atomes d'azote et éventuellement - en variante ou en plus - un atome d'oxygène ou un atome de soufre, ou un groupe SO ou un groupe $SO_2$), et qui contient en plus un à trois groupes oxo (C=O) et/ou thioxo (C=S) en tant que constituants de l'hétérocycle,
y compris toutes les formes tautomères possibles et éventuellement toutes les formes stéréoisomères possibles des composés de formule générale (I), et des sels ou des dérivés liés de manière coordonnée à un métal possibles des composés de formule (I).

2. Composés selon la revendication 1, **caractérisés en ce que**

n représente les nombres 0 ou 2,
A représente une liaison simple ou un groupe alcanediyle (alkylène) contenant 1 à 4 atomes de carbone,
$R^1$ représente un atome d'hydrogène, un groupe phényle ou un groupe alkyle contenant 1 à 5 atomes de carbone éventuellement substitué par un groupe cyano, halogéno, alcoxy en $C_1$ à $C_4$ ou alkylthio en $C_1$ à $C_4$,
$R^2$ représente un atome d'hydrogène ou un groupe alkyle contenant 1 à 5 atomes de carbone éventuellement substitué par un groupe cyano, halogéno, alcoxy en $C_1$ à $C_4$ ou alkylthio en $C_1$ à $C_4$, ou, conjointement avec $R^1$, représente un groupe alcanediyle (alkylène) contenant 1 à 5 atomes de carbone,
$R^3$ représente un atome d'hydrogène, un groupe nitro, cyano, carboxy, carbamoyle, thiocarbamoyle, halogéno, ou un groupe alkyle, alcoxyle, alkylthio, alkylsulfinyle, alkylsulfonyle, alkylamino, dialkylamino ou dialkylaminosulfonyle, contenant chacun 1 à 5 atomes de carbone dans le groupe alkyle, éventuellement substitué par un groupe cyano, halogéno ou alcoxy en $C_1$ à $C_4$,
$R^4$ représente un atome d'hydrogène, un groupe nitro, cyano, carboxy, carbamoyle, thiocarbamoyle, halogéno, ou un groupe alkyle, alcoxyle, alkylthio, alkylsulfinyle, alkylsulfonyle, alkylamino, dialkylamino ou dialkylaminosulfonyle, contenant chacun 1 à 5 atomes de carbone dans le groupe alkyle, éventuellement substitué par un groupe cyano, halogéno ou alcoxy en $C_1$ à $C_4$,
Y représente un atome d'hydrogène, un groupe alkyle contenant 1 à 6 atomes de carbone éventuellement substitué par un groupe cyano, halogéno ou alcoxy en $C_1$ à $C_4$, un groupe alcényle ou alcynyle contenant chacun 2 à 6 atomes de carbone éventuellement substitué par un groupe cyano, halogéno, (alkyle en $C_1$ à $C_4$)-carbonyle ou (alcoxy en $C_1$ à $C_4$)-carbonyle, ou un groupe aryle ou arylalkyle, contenant chacun 6 ou 10 atomes de carbone dans le groupe aryle et éventuellement 1 à 4 atomes de carbone dans la fraction alkyle, éventuellement substitué par un groupe nitro, amino, cyano, carboxy, carbamoyle, thiocarbamoyle, halogéno, alkyle en $C_1$ à $C_4$, halogéno-(alkyle en $C_1$ à $C_4$), (alcoxy en $C_1$ à $C_4$)-(alkyle en $C_1$ à $C_4$), alcoxy en $C_1$ à $C_4$, halogéno-(alkyle en $C_1$ à $C_4$), alkylthio en $C_1$ à $C_4$, halogéno-(alkylthio en $C_1$ à $C_4$), (alkyle en $C_1$ à $C_4$)-sulfinyle, halogéno-(alkyle en $C_1$ à $C_4$)-sulfinyle, (alkyle en $C_1$ à $C_4$)-sulfonyle, halogéno-(alkyle en $C_1$ à $C_4$)-sulfonyle, (alkyle en $C_1$ à $C_4$)-carbonyle, (alcoxy en $C_1$ à $C_4$)-carbonyle, (alkyle en $C_1$ à $C_4$)-carbonyl-amino, (alcoxy en $C_1$ à $C_4$)-carbonyl-amino, (alkyle en $C_1$ à $C_4$)-sulfonyl-amino, et
Z représente un des groupes hétérocycliques suivants,

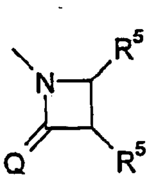 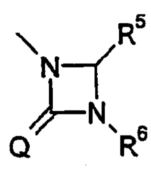 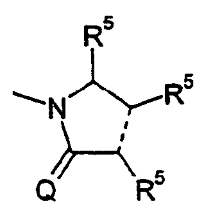 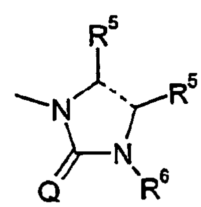

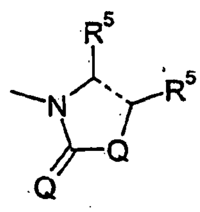 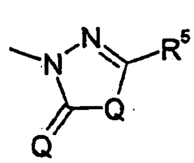 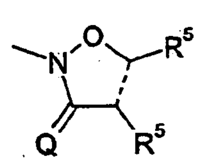 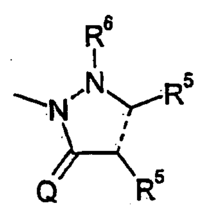

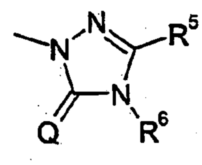 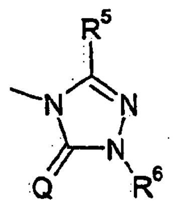 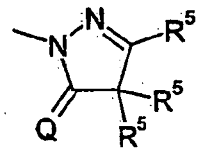 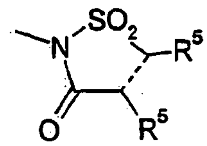

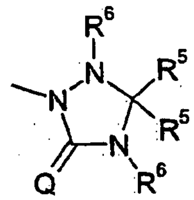 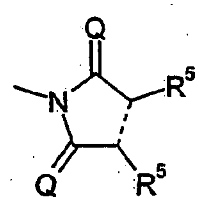 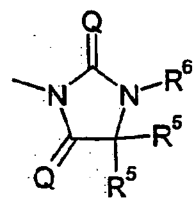 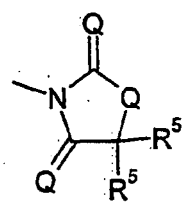

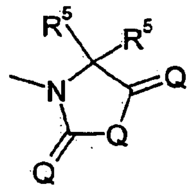 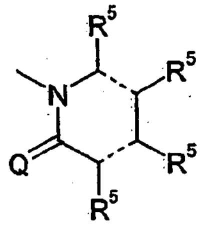 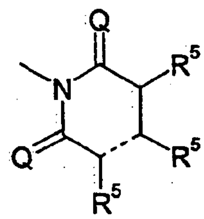 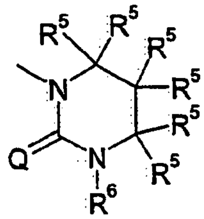

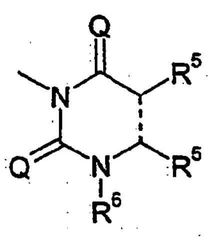 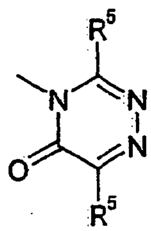 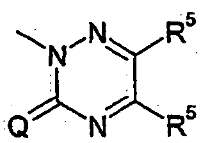 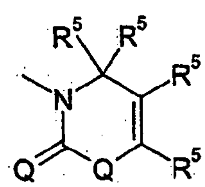

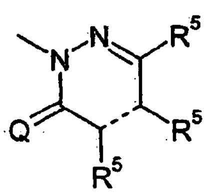 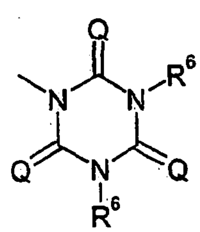 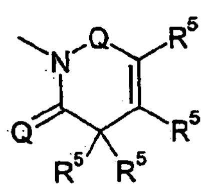 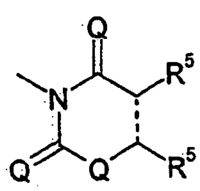

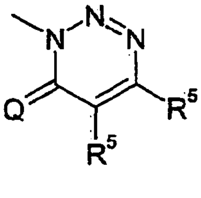 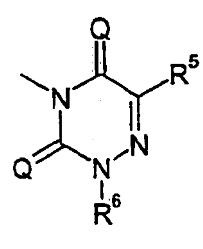 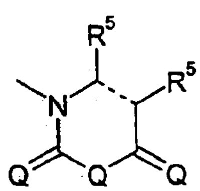 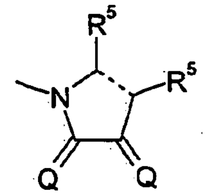

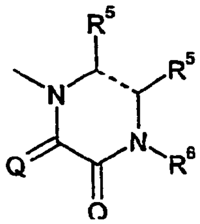 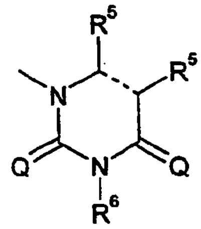 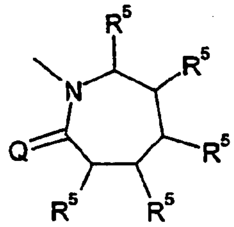 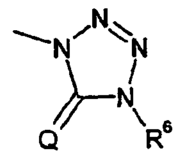

où chaque liaison en pointillés désigne une liaison simple ou une double liaison, et où chaque hétérocycle ne porte de préférence que deux substituants de la définition $R^5$ et/ou $R^6$ dans n'importe quelle combinaison,
Q représente un atome d'oxygène ou de soufre,
$R^5$ représente un atome d'hydrogène, un groupe hydroxy, mercapto, cyano, halogéno, un groupe alkyle, alkylcarbonyle, alcoxy, alcoxycarbonyle, alkylthio, alkylsulfinyle ou alkylsulfonyle, contenant chacun jusqu'à 6 atomes de carbone dans le groupe alkyle, éventuellement substitué par un groupe cyano, halogéno, alcoxy en $C_1$ à $C_4$, alkylthio en $C_1$ à $C_4$, (alkyle en $C_1$ à $C_4$)-sulfinyle ou (alkyle en $C_1$ à $C_4$)-sulfonyle, un groupe alkylamino ou dialkylamino, contenant chacun jusqu'à 6 atomes de carbone dans le groupe alkyle, éventuellement substitué par un groupe halogéno, un groupe alcényle, alcinyle, alcényloxy, alcénylthio, alcinylthio ou alcénylamino, contenant chacun jusqu'à 6 atomes de carbone dans le groupe alcényle ou dans le groupe alcinyle, éventuellement substitué par un groupe halogéno, un groupe cycloalkyle, cycloalkyloxy, cycloalkylthio, cycloalkylamino, cycloalkylalkyle, cycloalkylalcoxy, cycloalkylalkylthio ou cycloalkylalkylamino, contenant chacun 3 à 6 atomes de carbone dans le groupe cycloalkyle et éventuellement jusqu'à 4 atomes de carbone dans la fraction alkyle, éventuellement substitué par un groupe halogéno, ou un groupe phényle, phényloxy, phénylthio, phénylamino, benzyle, benzyloxy, benzylthio ou benzylamino éventuellement substitué par un groupe halogéno, alkyle en $C_1$ à $C_4$ ou alcoxy en $C_1$ à $C_4$, un groupe pyrrolidino, pipéridino ou morpholino, ou - si deux groupes vicinaux $R^5$ et $R^5$ se trouvent au niveau d'une double liaison - également avec le groupe $R^5$ vicinal, un groupe benzo, et
$R^6$ représente un atome d'hydrogène, un groupe hydroxy, amino, alkylidène-amino contenant jusqu'à 4 atomes de carbone, un groupe alkyle, alcoxy, alkylamino, dialkylamino ou alcanoylamino contenant chacun jusqu'à 6 atomes de carbone dans le groupe alkyle éventuellement substitué par un groupe halogéno ou alcoxy en $C_1$ à $C_4$, un groupe alcényle, alcinyle ou alcényloxy, contenant chacun jusqu'à 6 atomes de carbone dans le groupe alcényle ou dans le groupe alcinyle, éventuellement substitué par un groupe halogéno, un groupe cycloalkyle, cycloalkylalkyle ou cycloalkylamino, contenant chacun 3 à 6 atomes de carbone dans le groupe cycloalkyle et éventuellement jusqu'à 3 atomes de carbone dans la fraction alkyle, éventuellement substitué par un groupe halogéno, ou un groupe phényle ou benzyle éventuellement substitué par un groupe halogéno, alkyle en $C_1$ à $C_4$ ou alcoxy en $C_1$ à $C_4$, ou, conjointement avec un groupe $R^5$ ou $R^6$ vicinal, un groupe alcanediyle contenant 3 à 5 atomes de carbone éventuellement substitué par un groupe halogéno ou alkyle en $C_1$ à $C_4$,

où les groupes $R^5$ et $R^6$ individuels - si plusieurs d'entre eux sont liés aux mêmes groupes hétérocycliques - ont des significations identiques ou différentes dans le cadre de la définition ci-dessus.

**3.** Composés selon la revendication 1 ou 2, **caractérisés en ce que**

A représente une liaison simple ou un groupe méthylène, éthylidène (éthane-1,1-diyle) ou diméthylène (éthane-1,2-diyle),
$R^1$ représente un atome d'hydrogène, un groupe phényle ou un groupe méthyle, éthyle, n- ou i-propyle, n-, i- ou s-butyle éventuellement substitué par un groupe cyano, fluoro, chloro, bromo, méthoxy, éthoxy, n- ou i-propoxy, méthylthio, éthylthio, n- ou i-propylthio,
$R^2$ représente un atome d'hydrogène ou un groupe méthyle, éthyle, n- ou i-propyle, n-, i- ou s-butyle éventuellement substitué par un groupe cyano, fluoro, chloro, bromo, méthoxy, éthoxy, n- ou i-propoxy, méthylthio, éthylthio, n- ou i-propylthio, ou, conjointement avec $R^1$, un groupe méthylène, éthylidène (éthane-1,1-diyle), diméthylène (éthane-1,2-diyle), propylidène (propane-1,1-diyle) ou triméthylène (propane-1,3-diyle),
$R^3$ représente un atome d'hydrogène, un groupe nitro, cyano, carboxy, carbamoyle, thiocarbamoyle, fluoro, chloro, bromo, iodo, ou un groupe méthyle, éthyle, n- ou i-propyle, n-, i-, s- ou t-butyle, méthoxy, éthoxy, n- ou i-propoxy, n-, i-, s- ou t-butoxy, méthylthio, éthylthio, n- ou i-propylthio, n-, i-, s- ou t-butylthio, méthylsulfinyle, éthylsulfinyle, n- ou i-propylsulfinyle, méthylsulfonyle, éthylsulfonyle, n- ou i-propylsulfonyle, méthylamino, éthylamino, n- ou i-propylamino, n-, i-, s- ou t-butylamino, diméthylamino, diéthylamino, diméthylaminosulfonyle ou

diéthylaminosulfonyle éventuellement substitué par un groupe cyano, fluoro, chloro, bromo, méthoxy, éthoxy, n- ou i-propoxy,

$R^4$ représente un atome d'hydrogène, un groupe nitro, cyano, carboxy, carbamoyle, thiocarbamoyle, fluoro, chloro, bromo, iodo, ou un groupe méthyle, éthyle, n- ou i-propyle, n-, i-, s- ou t-butyle, méthoxy, éthoxy, n- ou i-propoxy, n-, i-, s- ou t-butoxy, méthylthio, éthylthio, n- ou i-propylthio, n-, i-, s- ou t-butylthio, méthylsulfinyle, éthylsulfinyle, n- ou i-propylsulfinyle, méthylsulfonyle, éthylsulfonyle, n- ou i-propylsulfonyle, méthylamino, éthylamino, n- ou i-propylamino, n-, i-, s- ou t-butylamino, diméthylamino, diéthylamino, diméthylaminosulfonyle ou diéthylaminosulfonyle éventuellement substitué par un groupe cyano, fluoro, chloro, bromo, méthoxy, éthoxy, n- ou i-propoxy,

Y représente un atome d'hydrogène, un groupe méthyle, éthyle, n- ou i-propyle, n-, i-, s- ou t-butyle éventuellement substitué par un groupe cyano, fluoro, chloro, bromo, méthoxy, éthoxy, n- ou i-propoxy, un groupe éthényle, propényle, butényle, pentényle, éthinyle, propinyle ou butinyle éventuellement substitué par un groupe cyano, fluoro, chloro, bromo, acétyle, propionyle, n- ou i-butyroyle, méthoxycarbonyle, éthoxycarbonyle, n- ou i-propoxycarbonyle, ou un groupe phényle, naphtyle, benzyle ou phényléthyle éventuellement substitué par un groupe nitro, amino, cyano, carboxy, carbamoyle, thiocarbamoyle, fluoro, chloro, bromo, iodo, méthyle, éthyle, n- ou i-propyle, n-, i-, s- ou t-butyle, trifluorométhyle, méthoxyméthyle, éthoxyméthyle, méthoxyéthyle, éthoxyéthyle, méthoxy, éthoxy, n- ou i-propoxy, n-, i-, s- ou t-butoxy, difluorométhoxy, trifluorométhoxy, méthylthio, éthylthio, n- ou i-propylthio, n-, i-, s- ou t-butylthio, difluorométhylthio, trifluorométhylthio, méthylsulfinyle, éthylsulfinyle, trifluorométhylsulfinyle, méthylsulfonyle, éthylsulfonyle, trifluorométhylsulfonyle, acétyle, propionyle, n- ou i-butyroyle, méthoxycarbonyle, éthoxycarbonyle, n- ou i-propoxycarbonyle, acétylamino, propionylamino, méthoxycarbonylamino, éthoxycarbonylamino, méthylsulfonylamino, éthylsulfonylamino, n- ou i-propylsulfonylamino,

Z représente un des groupes hétérocycliques suivants,

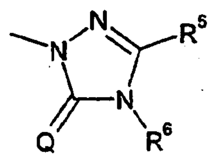

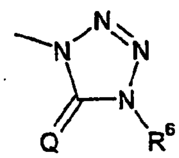

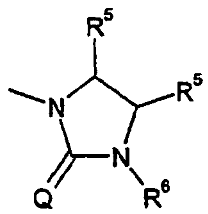

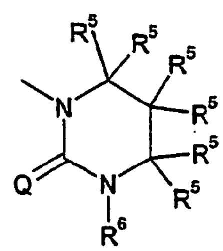

Q représente un atome d'oxygène,

$R^5$ représente un atome d'hydrogène, un groupe hydroxy, mercapto, cyano, fluoro, chloro, bromo, iodo, un groupe méthyle, éthyle, n- ou i-propyle, n-, i-, s-ou t-butyle, méthoxy, éthoxy, n- ou i-propoxy, n-, i-, s- ou t-butoxy, méthylthio, éthylthio, n- ou i-propylthio, n-, i-, s- ou t-butylthio, méthylsulfinyle, éthylsulfinyle, n- ou i-propylsulfinyle, méthylsulfonyle, éthylsulfonyle, n-ou i-propylsulfonyle éventuellement substitué par un groupe fluoro, chloro, méthoxy, éthoxy, n- ou i-propoxy, n-, i-, s- ou t-butoxy, méthylthio, éthylthio, n- ou i-propylthio, n-, i-, s- ou t-butylthio, méthylsulfinyle, éthylsulfinyle, n- ou i-propylsulfinyle, méthylsulfonyle, éthylsulfonyle, n- ou i-propylsulfonyle, un groupe méthylamino, éthylamino, n- ou i-propylamino, n-, i-, s- ou t-butylamino, diméthylamino, diéthylamino, di-n-propylamino ou di-i-propylamino, un groupe éthényle, propényle, butényle, éthinyle, propinyle, butinyle, propényloxy, butényloxy, propénylthio, buténylthio, propénylamino ou buténylamino éventuellement substitué par un groupe fluoro et/ou chloro, un groupe cyclopropyle, cyclobutyle, cyclopentyle, cyclohexyle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropyl-méthyle, cyclobutylméthyle, cyclopentyl-méthyle, cyclohexyl-méthyle, cyclopropyl-méthoxy, cyclobutylméthoxy, cyclopentyl-méthoxy, cyclohexyl-méthoxy, cyclopropyl-méthylthio, cyclobutyl-méthylthio, cyclopentyl-méthylthio, cyclohexyl-méthylthio, cyclopropyl-méthylamino, cyclobutyl-méthylamino, cyclopentyl-méthylamino ou cyclohexyl-méthylamino éventuellement substitué par un groupe fluoro et/ou chloro, ou un groupe phényle, phényloxy, phénylthio, phénylamino, benzyle, benzyloxy, benzylthio ou benzylamino éventuellement substitué par un groupe fluoro, chloro, méthyle, éthyle, n- ou i-propyle, n-, i-, s- ou t-butyle, méthoxy, éthoxy, n- ou i-propoxy, ou - si deux groupes $R^5$ et $R^5$ vicinaux se trouvent au niveau d'une double liaison - conjointement avec le groupe $R^5$ vicinal, représente également un groupe benzo, et

$R^6$ représente un atome d'hydrogène, un groupe hydroxy, amino, un groupe méthyle, éthyle, n- ou i-propyle, n-, i- ou s-butyle, méthoxy, éthoxy, n- ou i-propoxy, méthylamino, éthylamino ou diméthylamino éventuellement substitué par un groupe fluoro et/ou chloro, méthoxy ou éthoxy, un groupe éthényle, propényle, éthinyle, propinyle ou propényloxy éventuellement substitué par un groupe fluoro et/ou chloro, un groupe cyclopropyle, cyclobutyle, cyclopentyle, cyclohexyle, cyclopropyl-méthyle, cyclobutyl-méthyle, cyclopentyl-méthyle ou cyclohexyl-méthyle éventuellement substitué par un groupe fluoro et/ou chloro, ou un groupe phényle ou benzyle éventuellement substitué par un groupe fluoro, chloro, méthyle, éthyle, n- ou i-propyle, n-, i-, s- ou t-butyle, méthoxy, éthoxy, n- ou i-propoxy, ou, conjointement avec un groupe $R^5$ ou $R^6$ vicinal, un groupe propane-1,3-diyle (triméthylène), butane-1,4-diyle (tétraméthylène) ou pentane-1-5-diyle (pentaméthylène) éventuellement substitué par un groupe méthyle et/ou éthyle.

**4.** Composés selon l'une quelconque des revendications 1 à 3, **caractérisés en ce que**

A représente une liaison simple ou un groupe méthylène,

$R^1$ représente un atome d'hydrogène, un groupe phényle
ou un groupe méthyle, éthyle, n- ou i-propyle éventuellement substitué par un groupe fluoro ou chloro,

$R^2$ représente un atome d'hydrogène ou un groupe méthyle, éthyle, n- ou i-propyle éventuellement substitué par un groupe fluoro ou chloro, ou, conjointement avec $R^1$, un groupe méthylène ou diméthylène,

$R^3$ représente un atome d'hydrogène, un groupe nitro, cyano, fluoro, chloro, bromo, iodo, ou un groupe méthyle, éthyle, n- ou i-propyle, méthoxy, éthoxy, méthylthio, éthylthio, méthylsulfinyle, éthylsulfinyle, méthylsulfonyle, éthylsulfonyle, méthylamino, éthylamino, diméthylamino ou diméthylaminosulfonyle éventuellement substitué par un groupe fluoro, chloro, méthoxy ou éthoxy,

$R^4$ représente un atome d'hydrogène, un groupe nitro, cyano, fluoro, chloro, bromo, iodo, ou un groupe méthyle, éthyle, n- ou i-propyle, méthoxy, éthoxy, méthylthio, éthylthio, méthylsulfinyle, éthylsulfinyle, méthylsulfonyle, éthylsulfonyle, méthylamino, éthylamino, diméthylamino ou diméthylaminosulfonyle éventuellement substitué par un groupe fluoro, chloro, méthoxy ou éthoxy,

Y représente un atome d'hydrogène, un groupe méthyle, éthyle, n- ou i-propyle, n-, i-, s- ou t-butyle éventuellement substitué par un groupe cyano, fluoro, chloro, bromo, méthoxy, éthoxy, n- ou i-propoxy, un groupe propényle, butényle, pentényle, propinyle ou butinyle éventuellement substitué par un groupe fluoro, chloro ou bromo, ou un groupe phényle ou benzyle éventuellement substitué par un groupe nitro, amino, cyano, fluoro, chloro, bromo, méthyle, éthyle, n- ou i-propyle, n-, i-, s- ou t-butyle, trifluorométhyle, méthoxyméthyle, éthoxyméthyle, méthoxyéthyle, éthoxyéthyle, méthoxy, éthoxy, n- ou i-propoxy, difluorométhoxy, trifluorométhoxy, méthylthio, éthylthio, n- ou i-propylthio, difluorométhylthio, trifluorométhylthio, méthylsulfinyle, éthylsulfinyle, trifluorométhylsulfinyle, méthylsulfonyle, éthylsulfonyle, trifluorométhylsulfonyle, acétyle, propionyle, méthoxycarbonyle, éthoxycarbonyle, acétylamino, propionylamino, méthoxycarbonylamino, éthoxycarbonylamino, méthylsulfonylamino ou éthylsulfonylamino,

$R^5$ représente un atome d'hydrogène, un groupe fluoro, chloro, bromo, un groupe méthyle, éthyle, n- ou i-propyle, n-, i- ou s-butyle, méthoxy, éthoxy, n- ou i-propoxy, n-, i- ou s-butoxy, méthylthio, éthylthio, n- ou i-propylthio, n-, i- ou s-butylthio, méthylsulfinyle, éthylsulfinyle, n- ou i-propylsulfinyle, méthylsulfonyle, éthylsulfonyle, n- ou i-propylsulfonyle éventuellement substitué par un groupe fluoro, chloro, méthoxy, éthoxy, n- ou i-propoxy, méthylthio, éthylthio, méthylsulfinyle, éthylsulfinyle, méthylsulfonyle, éthylsulfonyle, un groupe méthylamino, éthylamino, n- ou i-propylamino, n-, i- ou s-butylamino, diméthylamino ou diéthylamino, un groupe éthényle, propényle, butényle, éthinyle, propinyle, butinyle, propényloxy, buténylоxy, propénylthio éventuellement substitué par un groupe fluoro et/ou chloro, ou un groupe cyclopropyle, cyclopropyloxy, cyclopropylthio, cyclopropylamino, cyclopropylméthyle, cyclopropyl-méthoxy, cyclopropyl-méthylthio ou cyclopropyl-méthylamino éventuellement substitué par un groupe fluoro et/ou chloro, et

$R^6$ représente un atome d'hydrogène, un groupe amino, un groupe méthyle, éthyle, méthoxy, éthoxy, méthylamino ou éthylamino éventuellement substitué par un groupe fluoro et/ou chloro, méthoxy ou éthoxy, un groupe diméthylamino, ou un groupe cyclopropyle, cyclopropyl-méthyle, phényle ou benzyle éventuellement substitué par un groupe fluoro et/ou chloro, ou, conjointement avec un groupe $R^5$ ou $R^6$ vicinal, un groupe propane-1,3-diyle (triméthylène), butane-1,4-diyle (tétraméthylène) ou pentane-1,5-diyle (pentaméthylène) éventuellement substitué par un groupe méthyle et/ou éthyle.

**5.** Composés selon l'une quelconque des revendications 1 à 4, **caractérisés en ce que**

$R^1$ représente un atome d'hydrogène,
$R^2$ représente un atome d'hydrogène,
$R^3$ représente un groupe chloro, trifluorométhyle ou méthoxy,
$R^4$ représente un groupe chloro, trifluorométhyle ou méthylsulfonyle,
$R^5$ représente un atome d'hydrogène, un groupe méthyle ou éthyle, et
$R^6$ représente un atome d'hydrogène, un groupe méthyle, éthyle, n- ou i-propyle, n-, i-, s- ou t-butyle, cyclopropyle, cyclopropyl-méthyle, cyclopentyle, cyclopentyl-méthyle, cyclohexyle, cyclohexyl-méthyle, phényle ou benzyle.

**6.** Composés selon l'une quelconque des revendications 1 à 5, **caractérisés en ce que**

$R^5$ représente un atome d'hydrogène, et
$R^6$ représente un groupe méthyle, éthyle, n- ou i-propyle, n-, i-, s- ou t-butyle, cyclopropyle, cyclopropyl-méthyle ou phényle.

**7.** Composés selon l'une quelconque des revendications 1 à 6, **caractérisés en ce que**

n représente le nombre 0.

**8.** Composés selon l'une quelconque des revendications 1 à 7, **caractérisés en ce que**

Z représente l'un des groupes ci-dessous

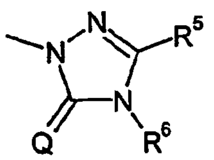

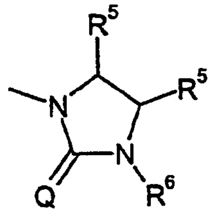

.

**9.** Composés selon l'une quelconque des revendications 1 à 8, **caractérisés en ce que**

Q représente un atome d'oxygène.

**10.** Composés selon l'une quelconque des revendications 1 à 9, **caractérisés en ce que**

Y représente un atome d'hydrogène, un groupe méthyle, éthyle, n- ou i-propyle, n-, i-, s- ou t-butyle éventuellement substitué par un groupe fluoro, chloro ou méthoxy, un groupe propényle, butényle, pentényle éventuellement substitué par un groupe fluoro ou chloro, ou un groupe phényle éventuellement substitué par un groupe amino, cyano, fluoro, chloro, méthyle, éthyle, trifluorométhyle, méthoxy ou éthoxy.

**11.** Composés de formule (IA)

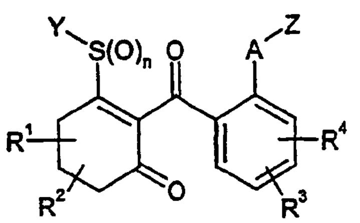

(IA)

dans laquelle n, A, $R^1$, $R^2$, $R^3$, $R^4$, Y et Z sont définis selon l'une quelconque des revendications 1 à 9.

**12.** Composés de formule (IB)

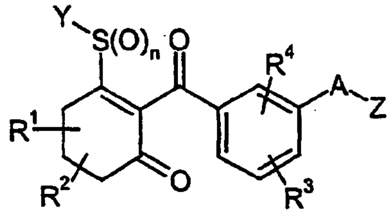

(IB)

dans laquelle n, A, $R^1$, $R^2$, $R^3$, $R^4$, Y et Z sont définis selon l'une quelconque des revendications 1 à 9.

**13.** Composés de formule (IC)

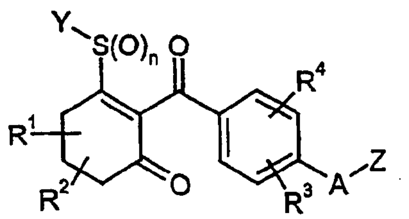

(IC)

dans laquelle n, A, $R^1$, $R^2$, $R^3$, $R^4$, Y et Z sont définis selon l'une quelconque des revendications 1 à 9.

**14.** Sels de sodium, de potassium, de magnésium, de calcium, d'ammonium, d'alkyle en $C_1$ à $C_4$-ammonium, de di-(alkyle en $C_1$ à $C_4$)-ammonium, de tri-(alkyle en $C_1$ à $C_4$)-ammonium, de tétra-(alkyle en $C_1$ à $C_4$)-ammonium, de tri-(alkyle en $C_1$ à $C_4$)-sulfonium, de cycloalkyle en $C_5$ ou $C_6$-ammonium et de di-(alkyle en $C_1$ à $C_2$)-benzyl-ammonium, de composés de formule (I) selon l'une quelconque des revendications 1 à 13, ainsi que leurs composés complexes avec des métaux tels que le cuivre, le fer, le cobalt et le nickel.

**15.** Procédé de préparation de composés selon l'une quelconque des revendications 1 à 12, **caractérisé en ce que** l'on fait réagir des composés de formule (II)

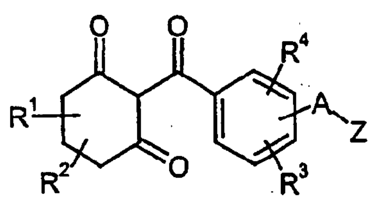

(II)

dans laquelle
A, $R^1$, $R^2$, $R^3$, $R^4$ et Z sont définis selon l'une quelconque des revendications 1 à 9,
avec un agent d'halogénation, éventuellement en présence d'un adjuvant de réaction et éventuellement en présence d'un diluant, et on fait réagir les halogéno-cyclohexénones de formule (III) ainsi obtenues (dans la première étape de réaction)

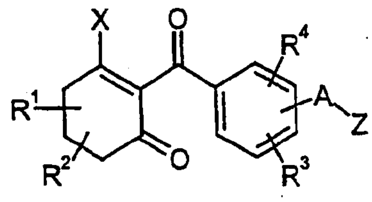

(III)

dans laquelle
A, R1, $R^2$, $R^3$, $R^4$ et Z sont définis selon l'une quelconque des revendications 1 à 9, et
X représente un atome d'halogène,

- après isolement intermédiaire ou sans isolement intermédiaire (*in situ*) -

dans une deuxième étape de réaction, avec des composés mercapto de formule (IV)

HS-Y (IV)

dans laquelle

Y a la signification susmentionnée,

éventuellement en présence d'un adjuvant de réaction et éventuellement en présence d'un diluant,
et éventuellement à la suite de ces réactions, on soumet les composés de formule (I) ainsi obtenus, dans le cadre de la définition des substituants, de manière classique à des réactions de substitution électrophile ou nucléophile, ou à des réactions d'oxydation ou de réduction, ou on convertit les composés de formule (I) de manière classique en sels.

**16.** Produits herbicides, **caractérisés par** leur teneur en au moins un composé selon l'une quelconque des revendications 1 à 12 et en additifs classiques.

**17.** Utilisation d'au moins un composé selon l'une quelconque des revendications 1 à 12 pour lutter contre le développement des mauvaises herbes.

**18.** Procédé permettant de lutter contre le développement des mauvaises herbes, **caractérisé en ce que** l'on fait agir au moins un composé selon l'une quelconque des revendications 1 à 13 ou un produit selon la revendication 16 sur les mauvaises herbes et/ou sur leur habitat.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 090262 A
- EP 135191 A
- EP 186118 A
- EP 186119 A
- EP 186120 A
- EP 319075 A
- WO 9626200 A
- WO 9746530 A
- WO 9907688 A
- WO 0005221 A